(19) Europäisches Patentamt
European Patent Office
Office européen des brevets

(11) **EP 2 367 008 A2**

(12) # EUROPEAN PATENT APPLICATION

(43) Date of publication:
**21.09.2011 Bulletin 2011/38**

(51) Int Cl.:
***G01N 33/74*** (2006.01)   ***G01N 33/68*** (2006.01)
***C07K 7/08*** (2006.01)   ***A61K 38/00*** (2006.01)

(21) Application number: **10009373.1**

(22) Date of filing: **04.06.2004**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **06.06.2003 US 476778 P**
**23.12.2003 US 532117 P**

(62) Document number(s) of the earlier application(s) in
accordance with Art. 76 EPC:
**09002616.2 / 2 093 570**
**04754499.4 / 1 636 593**

(27) Previously filed application:
**04.06.2004 EP 09002616**

(71) Applicant: **Genentech, Inc.**
**South San Francisco CA 94080-4990 (US)**

(72) Inventors:
• **Kirchhofer, Daniel K.**
**Los Altos, CA 94024 (US)**
• **Lazarus, Robert A.**
**Millbrae, CA 94030 (US)**
• **Yao, Xiaoyi**
**Lubbock, TX 79424 (US)**

(74) Representative: **Woolley, Lindsey Claire et al**
**Mewburn Ellis LLP**
**33 Gutter Lane**
**London**
**EC2V 8AS (GB)**

Remarks:
This application was filed on 09-09-2010 as a
divisional application to the application mentioned
under INID code 62.

(54) **Modulating the interaction betwenn HGF beta chain and C-Met**

(57)   The invention provides methods and compositions for modulating the HGF/c-met signalling pathway, in particular by regulating binding of HGF β chain to c-met.

**EP 2 367 008 A2**

**Description**

This application is a non-provisional application filed under 37 CFR 1.53(b)(1), claiming priority under 35 USC 119(e) to provisional application number 60/476,778 filed June 6, 2003, and provisional application number 60/532,117 filed December 23, 2003,the contents of which are incorporated in their entirety herein by reference.

TECHNICAL FIELD

The present invention relates generally to the fields of molecular biology and growth factor regulation. More specifically, the invention concerns modulators of the HGF/c-met signaling pathway, and uses of said modulators.

BACKGROUND

Hepatocyte growth factor (HGF), also known as scatter factor (SF), is the ligand for Met (Bottaro et al., 1991), a receptor tyrosine kinase encoded by the *c-met* protooncogene (Cooper et al., 1984a &b). HGF binding to Met induces phosphorylation of the intracellular kinase domain resulting in activation of a complex set of intracellular pathways that lead to cell growth, differentiation and migration in a variety of cell types; several recently published reviews provide a comprehensive overview (Birchmeier et al., 2003; Trusolino and Comoglio, 2002; Maulik et al., 2002). In addition to its fundamental importance in embryonic development and tissue regeneration, the HGF/Met signaling pathway has also been implicated in invasive tumor growth and metastasis and as such represents an interesting therapeutic target (Birchmeier et al., 2003; Trusolino and Comoglio, 2002; Danilkovitch-Miagkova and Zbar, 2002; Ma et al., 2003).

HGF belongs to the plasminogen-related growth factor family and comprises a 69 kDa α-chain containing the N-terminal finger domain (N) and four Kringle (K1-K4) domains, and a 34 kDa β-chain which has strong similarity to protease domains of chymotrypsin-like serine proteases from Clan PA(S)/FamilyS1 (Nakamura et al., 1989; Donate et al., 1994; Rawlings et al., 2002). Like plasminogen and other serine protease zymogens, HGF is secreted as a single chain precursor form (scHGF). scHGF binds to heparan sulfate proteoglycans, such as syndecan-1 (Derksen et al., 2002) on cell surfaces or in the extracellular matrix. Heparan sulfate proteoglycans bind to the N domain (Hartmann et al., 1998), which also contributes to the high affinity Met binding together with amino acids located in K1 (Lokker et al., 1994). Although scHGF is able to bind Met with high affinity, it cannot activate the receptor (Lokker et al., 1992; Hartmann et al., 1992). Acquisition of HGF signaling activity is contingent upon proteolytic cleavage (activation) of scHGF at Arg494-Val495 resulting in the formation of mature HGF, a disulfide-linked α/β heterodimer (Lokker et al., 1992; Hartmann et al., 1992; Naldini et al., 1992). The protease-like domain of HGF (HGF β-chain) is devoid of catalytic activity since it lacks the required Asp [c102]-His [c57]-Ser [c195] (standard chymotrypsinogen numbering in brackets throughout) catalytic triad found in all serine proteases (Perona and Craik, 1995; Hedstrom, 2002), having a Gln534 [c57] and Tyr673 [c195].

Because of its importance in regulating HGF activity, this process must be tightly controlled by HGF converting enzymes and their corresponding physiological inhibitors. scHGF activation is mediated *in vitro* by chymotrypsin-like serine proteases including hepatocyte growth factor activator (HGFA) (Miyazawa et al., 1993), matriptase/MT-SP1 (Takeuchi et al. 1999; Lin et al., 1999), urokinase-type plasminogen activator (Naldini et al., 1992), factor XIIa (Shimomura et al., 1995), factor XIa (Peek et al., 2002) and plasma kallikrein (Peek et al., 2002). Similar to scHGF, these proteases are produced as inactive precursors; their enzymatic activities are also tightly regulated by other activating proteases and both Kunitz- and serpin-type inhibitors.

Serine proteases and their activation process have been described (Donate et al., 1994). In serine proteases, activation cleavage of the zymogen effects a conformational rearrangement of the so-called 'activation domain' giving rise to a properly formed active site and the substrate/inhibitor interaction region. The activation domain constitutes three surface-exposed loops designated the [c140]-, [c180]- and [c220]-loops and insertion of the newly formed N-terminus into a hydrophobic pocket (Huber and Bode, 1978). In the homologous ligand/receptor pair macrophage stimulating protein (MSP)/Ron, the serine protease-like MSP β-chain provides the main energy for receptor binding (Wang et al., 1997; Miller and Leonard, 1998). This is reversed from the HGF/Met system where the high affinity receptor binding site for Met resides in the HGF α-chain (Lokker et al., 1994; Okigaki et al., 1992).

All references cited herein, including patent applications and publications, are incorporated by reference in their entirety.

DISCLOSURE OF THE INVENTION

Hepatocyte growth factor (HGF), a plasminogen-related growth factor, binds to its receptor tyrosine kinase Met (also referred to herein as c-Met or c-met), which is implicated in development, tissue regeneration and invasive tumor growth. The successful expression and purification of the HGF protease-like β chain, which is described herein, enabled a definitive determination of the nature of the interaction of HGF, specifically HGF β-chin, with c-Met, which led to a clearer understanding of the mechanism for c-Met activation. It is empirically demonstrated herein that the serine protease-like HGF β-chin itself binds to Met. In comparison, the zymogen-like form of HGF β has much weaker Met binding, suggesting optimal interactions result from conformational changes upon processing. A panel of β-chain mutants tested in cell migration, Met phosphorylation, HGF-dependent cell proliferation and Met binding assays showed that reduced biological activity of full length HGF mutants is due at least in part to reduced binding of the HGF β-chain to Met. The functional binding site comprises the 'activation domain' and 'active site region', similar to the substrate-processing site of serine proteases. The data indicate that activated (but not the zymogen-like form) β chain may comprise an interface required for optimal interaction with another molecule such as another HGF β chain so as to effect maximal/optimal c-met activation. Mutation analyses described herein provide a basis for design of HGF mutants capable of inhibiting wild type HGF/c-met interaction across a spectrum of potencies. Examples of such mutants are described herein. These mutants are capable of competing with wild type HGF for binding to c-met, yet exhibit reduced ability to effect c-met associated biological functions. This is particularly advantageous where complete or substantial inhibition of the HGF/c-met axis is undesirable; this is of particular concern because HGF and c-met are ubiquitously expressed in normal cells and tissues. These mutants can also be used as advantageous therapeutic agents for treating pathological conditions wherein reduced, but not complete absence of ,HGF/c-met biological activity is desirable. Method and compositions of the invention are based generally on these findings, which are described in greater detail below. It is shown that HGF β chain and its interaction with c-met can be a unique and advantageous target for greater fine-tuning in designing prophylatic and/or therapeutic approaches against pathological conditions associated with abnormal or unwanted signaling of the HGF/c-met pathway. Thus, the invention provides methods, compositions, kits and articles of manufacture for identifying and using substances that are capable of modulating the HGF/c-met pathway through modulation of HGF β chain binding to c-met, and for modulation of biological/physiological activities associated with HGF/c-met signaling.

Accordingly, in one aspect, the invention provides a method of screening for (or identifying) a substance that selectively binds activated hepatocyte growth factor β chain, said method comprising: (a) comparing (i) binding of a candidate substance to an activated HGF β chain (as described in greater detail below), with (ii) binding of the candidate substance to a reference HGF β chain, wherein said reference β chain is not capable of specific and/or substantial binding to c-met; whereby a candidate substance that exhibits greater binding affinity to the activated HGF β chain than to the reference HGF β chain is selected (or identified) as a substance that selectively binds activated HGF β chain. In some embodiments, the reference β chain is contained within a single chain HGF polypeptide. In some embodiments, the reference β chain is fused at its N-terminus to a portion of the C-terminal region of HGF α chain, wherein position 494 (corresponding to wild type human HGF) of the C-terminal region is an amino acid other than arginine (for e.g., glutamic acid). In some embodiments, the portion of the C-terminal region of HGF α chain comprises, consists or consists essentially of amino acid sequence from residue 479 to 494 of human HGF.

In another aspect, the invention provides a method of screening for a substance that blocks c-met activation, said method comprising screening for a substance that binds (preferably, but not necessarily, specifically) c-met and blocks specific binding of HGF β chain to c-met. In some embodiments, the substance competes with HGF β chain for binding to c-met. In one embodiment, the substance comprises, consists or consists essentially of an amino acid sequence having at least about 60%, 70%, 80%, 90%, 95%, 99% sequence similarity or identity with respect to wild type HGF (for e.g., human) β chain, for e.g., human β chain comprising amino acid residues 495(Val) to 728(Ser) (for e.g., the wild type HGF β chain as described herein). In some embodiments wherein the substance comprises, consists or consists essentially of such an amino acid sequence, position 604 and/or 561 is an amino acid other than cysteine. In some of these embodiments, the substance is not substantially capable of forming a link (covalent or non-covalent) with HGF α chain or portion thereof.

In some embodiments of methods of screening (identifying) of the invention, the methods comprise determining binding affinity of a candidate substance with respect to a target antigen which comprises, consists or consists essentially of a portion or all of HGF β chain in a naturally occurring form or variant form. Such target antigens can include any polypeptide that comprises, consists or consists essentially of an HGF β chain amino acid sequence comprising at least one mutation (in particular where said at least one mutation results in a change in the ability of HGF β chain to bind to c-Met). In some embodiments, the polypeptides comprise, consist or consist essentially of an HGF β chain amino acid sequence (either wild type or comprising at least one mutation) fused to a heterologous polypeptide sequence (such as a portion or all of the HGF α chain). Examples of such HGF β chains include those described herein, for e.g., zymogen-like HGF β chain (e.g., mutation at position 494), HGF β chain (Cys604Ser), and "active site region" mutants. The invention provides HGF mutants having a mutation in one or more positions in the β chain (including mutations in the active site region), such as positions 534, 578, 619, 673, 692, 693, 694, 695, 696, 699 and/or 702. Other mutants that are provided include those having mutations at positions within HGF that render it incapable of being activated (e.g., cleaved) *in vitro or in vivo;* an example of one such mutant comprises a mutation in positions 424 and/or 494.

In another aspect, the invention provides a method of screening for an HGF receptor antagonist which blocks binding of HGF to its receptor (for e.g., the binding of HGF to a first receptor molecule, and/or the binding of HGF, for e.g. through one or both α and β chains, to a second receptor molecule for receptor dimerization), said method comprising selecting for a substance that binds to at least one, two, three, four, or any number up to all of residues 534, 578, 619, 673, 692, 693, 694, 695, 696, 699 and/or 702 of HGF β chain. Combinations of two or more residues can include any of residues 534, 578, 619, 673, 692, 693, 694, 695, 696, 699 and/or 702 of HGF β chain. In one embodiment, the substance binds to at least one, or both, of residues 673 and 695. In another embodiment, the substance binds to (i) at least one, or both, of residues 673 and 695, and (ii) residue 692. In another embodiment, the substance binds to (i) at least one or both of residues 673 and 695, and (ii) residue 692, and (iii) at least one, or both, of residues 534 and 578. In another embodiment, the substance binds to (i) at least one or both of residues 673 and 695, and (ii) at least one, two, or all of residues 534, 578 and 692. In another embodiment, the substance binds to (i) at least one, both or all of residues 673, 695 and 696, and (ii) at least one, both or any number up to all of residues 534, 578, 692 and 694. In one embodiment, the substance binds to HGF β chain wherein if there is a mutation in position 534, 673 and/or 692, said β chain also comprises a mutation in at least one, both or any number up to all of positions 578, 694, 695 and 696. In some embodiments of these molecules, the activated β chain has a conformation of β chain obtained by cleavage of single chain HGF; and in some of these embodiments, said cleavage is at or adjacent to residues 494 and 495 of single chain HGF, for e.g., said cleavage may occur between residues 494 and 495 of single chain HGF. In one embodiment, the substance binds to at least one of residues 673, 693, 694, 695 and 696. In one embodiment, the substance binds to at least one of residues 692 and 702. In one embodiment, the substance binds to at least one of residues 534 and 578. In one embodiment, the substance binds to at least one of residues 513, 516, 619 and 699. In one embodiment, the substance binds to two or more residues selected from the group consisting of a first group consisting of residues 673, 693, 694, 695 and 696, a second group consisting of residues 692 and 702, a third group consisting of residues 534 and 578 and a fourth group consisting of residues 513, 516, 619 and 699. Said two or more residues can be selected from the same group or a combination of any of the 4 groups. In some embodiments, the substance further binds to residue 423, 424, 494 and/or 495.

As would be evident to one skilled in the art, screening assays consistent with those described above can also comprise a first step of screening based on a functional readout to obtain a first set of candidate modulatory substance, followed by a second step of screening based on ability of the first set of candidate modulatory substance to modulate binding of HGF β to c-met. A functional readout can be any that would be evident to one skilled in the art, based on a knowledge of biological activities associated with the HGF/c-met signaling pathway. These biological activities include but are not limited to C-met phosphorylation, phosphorylation of cellular molecules that are substrates of C-met kinase, cellular growth (proliferation, survival, etc.), angiogenesis, cell migration, cell morphogenesis, etc.

In one aspect, the invention provides HGF/c-met antagonists that disrupt the HGF/c-met signaling pathway. For example, the invention provides a molecule that binds to activated hepatocyte growth factor β chain and inhibits specific binding of said activated HGF β chain to c-met. In one embodiment, binding affinity of the molecule for the activated form of the β chain is greater than binding affinity of the molecule for the β chain in zymogen form. In some embodiments, the molecule binds to the active site/domain of the β chain. In some embodiments, said active site comprises at least one, two, three, four, five, six or all of residues 534, 578, 673, 692, 694, 695 and/or 696 of the β chain. Combinations of two or more residues can include any of residues 534, 578, 673, 692, 694, 695 and/or 696 of HGF β chain. In some embodiments, the molecule binds to at least one, two, three, four, or any number up to all of residues 534, 578, 619, 673, 692, 693, 694, 695, 696, 699 and/or 702 of HGF β chain. Combinations of two or more residues can include any of residues 534, 578, 619, 673, 692, 693, 694, 695, 696, 699 and/or 702 of HGF β chain. In one embodiment, the substance binds to at least one, or both, of residues 673 and 695. In another embodiment, the substance binds to (i) at least one, or both, of residues 673 and 695, and (ii) residue 692. In another embodiment, the substance binds to (i) at least one or both of residues 673 and 695, and (ii) residue 692, and (iii) at least one, or both, of residues 534 and 578. In another embodiment, the substance binds to (i) at least one or both of residues 673 and 695, and (ii) at least one, two, or all of residues 534, 578 and 692. In another embodiment, the substance binds to (i) at least one, both or all of residues 673, 695 and 696, and (ii) at least one, both or any number up to all of residues 534, 578, 692 and 694. In one embodiment, the substance binds to HGF β chain wherein if there is a mutation in position 534, 673 and/or 692, said β chain also comprises a mutation in at least one, both or any number up to all of positions 578, 694, 695 and 696. In one embodiment, the substance binds to at least one of residues 673, 693, 694, 695 and 696. In one embodiment, the substance binds to at least one of residues 692 and 702. In one embodiment, the substance binds to at least one of residues 534 and 578. In one embodiment, the substance binds to at least one of residues 513, 516, 619 and 699. In one embodiment, the substance binds to two or more residues selected from the group consisting of a first group consisting of residues 673, 693, 694, 695 and 696, a second group consisting of residues 692 and 702, a third group consisting of residues 534 and 578 and a fourth group consisting of residues 513, 516, 619 and 699. Said two or more residues can be selected from the same group or a combination of any of the 4 groups. In some embodiments, the substance further binds to residue 423, 424, 494 and/or 495. In some embodiments of these molecules, the activated β chain has a conformation of β chain obtained by cleavage of single chain HGF; and in some of these embodiments, said cleavage is at or adjacent to residues 494 and 495 of single chain HGF, for e.g., said cleavage may occur between residues 494 and 495 of single chain HGF.

In some embodiments, the substance or molecule is or comprises a small molecule, peptide, antibody, antibody fragment, aptamer, or a combination thereof.

In one aspect, the invention provides an HGF mutant that has HGF/c-met modulatory activity, for e.g. an antagonist of HGF/c-met activity or an HGF variant exhibiting a reduction, but not an absence, of HGF biological activity (e.g., cell growth stimulatory activity). In one embodiment, an antagonist of the invention is capable of inhibiting the biological activity of wild type (*in vivo*) HGF (such biological activity includes but is not limited to stimulation of cell proliferation, enhancement of cell survival, promotion of angiogenesis, induction/promotion of cell migration). In one embodiment, an HGF variant provides reduced cell growth (including but not limited to cell proliferation, cell survival, angiogenic, cell migration) promoting activity. In one embodiment, the HGF mutant is a zymogen-like HGF β chain (e.g., mutation at position 494). For example, these mutants include those having mutations at positions within HGF that render it incapable of being activated (e.g., cleaved) *in vitro* or *in vivo;* an example of one such mutant comprises a mutation in positions 424 and 494. In one embodiment, the HGF mutant is a single chain HGF, for instance HGF comprising a mutation in position 424 and/or 494, and/or a position adjacent to residue 424 and/or 494. In one embodiment, an HGF mutant of the invention further comprises a mutation in position 604 (e,g. HGF β chain Cys$^{604}$Ser). In one embodiment, an HGF mutant of the invention is an "active site region" mutant as described above. In one embodiment, the invention provides HGF mutants having a mutation in one or more positions in the β chain (including mutations in the active site region), such as positions 534, 578, 619, 673, 692, 693, 694, 695, 696, 699 and/or 702.

In some embodiments, binding of a substance or molecule of the invention to activated β chain inhibits c-met activation by HGF. In some embodiments, binding of said substance or molecule to activated β chain inhibits cell growth (such as cell proliferation, survival, angiogenesis, morphogenesis, migration) induced by HGF. In some embodiments, binding of said substance or molecule to activated β chain inhibits c-met receptor dimerization.

In some embodiments, a substance or molecule of the invention is obtained by a screening or identification method of the invention as described herein.

In some embodiments, a substance or molecule of the invention comprises a peptide. In some embodiments, said peptide comprises the sequence VDWVCFRDLGCDWEL. In some embodiments, the peptide comprises an amino acid sequence having at least 50%, 60%, 70%, 80%, 90%, 95%, 98% sequence identity or similarity with the sequence VDWVCFRDLGCDWEL. Variants of this sequence can be obtained by methods known in the art, for example by combinatorial mutagenesis (e.g., by phage display). Specific examples of such variants include but are not limited to those depicted in Table 1 below. In some embodiments, these peptides comprise modifications that enhance their inhibitory and/or therapeutic effect (including, for e.g., enhanced affinity, improved pharmacokinetics properties (such as half life, stability, clearance rate), reduced toxicity to the subject). Such modifications include, for e.g., modifications involving glycosylation, pegylation, substitution with non-naturally occurring but functionally equivalent amino acid, linking groups, etc. Suitable modifications are well known in the art, and furthermore can be determined empirically as necessary.

Table 1

| | | | | | | | | | | | | | | |
|---|---|---|---|---|---|---|---|---|---|---|---|---|---|---|
| V | D | W | I | C | F | R | D | I | G | C | D | W | V | V |
| V | D | W | I | C | L | R | D | V | G | C | D | W | V | Q |
| V | D | W | V | C | F | R | D | F | G | C | D | W | V | V |
| V | D | W | V | C | F | R | D | F | G | C | D | W | V | L |
| V | D | W | V | C | F | R | D | F | G | C | D | W | V | H |
| V | D | W | V | C | F | R | D | F | G | C | Y | W | E | Q |
| V | D | W | V | C | F | R | D | F | G | C | w | F | E | S |
| V | D | W | V | C | F | R | D | H | G | C | E | Y | V | E |
| V | D | W | V | C | F | R | D | I | G | C | D | W | V | L |
| V | D | W | V | C | F | R | E | F | G | C | D | W | V | L |
| V | D | W | V | C | F | R | E | I | G | C | D | W | V | L |
| V | D | W | V | C | F | R | G | I | G | C | D | W | V | L |
| V | D | W | V | C | L | R | D | I | G | C | D | W | V | P |
| V | D | W | V | C | F | R | D | L | G | C | D | Y | E | H |
| V | D | W | V | C | F | R | D | L | G | C | D | Y | V | L |
| V | D | W | V | C | F | R | E | L | G | C | D | W | V | V |
| V | D | W | V | C | F | R | E | L | G | C | D | W | V | F |
| V | D | W | V | C | F | R | D | M | G | C | Y | Y | E | L |
| V | D | W | V | C | F | R | D | M | G | C | D | W | V | L |

(continued)

| V | D | W | V | C | F | R | D | S | G | C | Y | Y | A | T |
|---|---|---|---|---|---|---|---|---|---|---|---|---|---|---|
| V | D | W | V | C | F | R | D | T | G | C | D | W | V | L |
| V | D | W | V | C | F | R | D | V | G | C | D | W | V | Q |
| V | D | W | V | C | F | R | D | V | G | C | D | W | V | L |
| V | D | W | V | C | F | R | E | V | G | C | D | W | V | L |
| V | D | W | V | C | F | R | D | V | G | C | D | W | V | M |
| V | D | W | V | C | F | R | D | Y | G | C | D | M | V | P |
| V | D | W | V | C | F | R | D | V | G | C | D | W | V | Q |
| V | D | W | V | C | F | R | D | Y | G | C | E | W | V | A |
| V | D | W | V | C | F | R | D | V | G | C | E | W | V | V |
| V | N | W | V | C | F | R | D | I | G | C | D | W | V | L |
| V | N | W | V | C | F | R | D | L | G | C | D | W | V | A |
| V | N | W | V | C | F | R | D | L | G | C | D | W | V | L |
| V | N | W | V | C | F | R | D | L | G | C | D | W | V | P |
| V | N | W | V | C | F | R | D | L | G | C | D | W | V | V |
| V | N | W | V | C | F | R | D | Q | G | C | D | W | V | L |
| V | N | W | V | C | F | R | D | V | G | C | D | W | V | L |
| V | N | W | V | C | F | R | E | L | G | C | D | W | V | L |
| V | N | W | V | C | L | R | D | V | G | C | D | W | V | L |

In one embodiment, the invention provides an HGF/c-met antagonist which is a substance or molecule that competes with hepatocyte growth factor β chain for binding to c-met. In some of the embodiments, said molecule inhibits c-met receptor multimerization (for e.g., dimerization). In some embodiments, said molecule comprises a variant (mutant) β chain having reduced ability to interact (for e.g., multimerize/dimerize) with another β chain molecule. In some embodiments, said molecule inhibits HGF β chain multimerization (for e.g., dimerization). In some embodiments, said molecule binds to c-met but exhibits reduced ability to effect c-met activation (for e.g., as indicated by reduced c-met phosphorylation, mitogen activated protein kinase (MAPK) phosphorylation, and/or reduced HGF/c-met dependent cell migration, cell proliferation, cell survival, cell morphogenesis, etc.). In one embodiment, the molecule comprises, consists or consists essentially of a polypeptide comprising at least a portion of an HGF β chain, wherein said β chain comprises a mutation in one or more of positions 695, 696 and 673. In one embodiment, the molecule comprises, consists or consists essentially of a polypeptide comprising a mutation in one or more of positions 695, 696 and 673, and a mutation in one or more of positions 534, 578, 692 and 694. Combinations of two or more residues can include any of residues 534, 578, 673, 692, 694, 695 and 696 of HGF β chain. In one embodiment, the molecule comprises, consists or consists essentially of a polypeptide comprising at least a portion of an HGF β chain, wherein said β chain comprises a mutation in at least one, or both, of residues 673 and 695. In another embodiment, the molecule comprises, consists or consists essentially of a polypeptide comprising at least a portion of an HGF β chain, wherein said β chain comprises a mutation in at least one, or both, of residues 673 and 695, and (ii) residue 692. In another embodiment, the molecule comprises, consists or consists essentially of a polypeptide comprising at least a portion of an HGF β chain, wherein said β chain comprises a mutation in at least one or both of residues 673 and 695, and (ii) residue 692, and (iii) at least one, or both, of residues 534 and 578. In another embodiment, the molecule comprises, consists or consists essentially of a polypeptide comprising at least a portion of an HGF β chain, wherein said β chain comprises a mutation in at least one or both of residues 673 and 695, and (ii) at least one, two, or all of residues 534, 578 and 692. In another embodiment, the molecule comprises, consists or consists essentially of a polypeptide comprising at least a portion of an HGF β chain, wherein said β chain comprises a mutation in at least one, both or all of residues 673, 695 and 696, and (ii) at least one, both or any number up to all of residues 534, 578, 692 and 694. In one embodiment, the molecule comprises, consists or consists essentially of a polypeptide comprising at least a portion of an HGF β chain, wherein said β chain comprises a mutation, and wherein if there is a mutation in position 534, 673 and/or 692, said β chain also comprises a mutation in at least one, both or any number up to all of positions 578, 694, 695 and 696. In one embodiment, the molecule comprises, consists or consists essentially of a polypeptide comprising at least a portion of an HGF β chain, wherein said β chain comprises a mutation in one or more positions in the β chain (including mutations in the active site region), such as positions 534, 578, 619, 673, 692, 693, 694, 695, 696, 699 and/or 702. In one embodiment, the molecule comprises, consists, or consists essentially of at least a portion of HGF, wherein said portion comprises a mutation at one or more positions within HGF that renders it incapable of being activated (e.g., cleaved) *in vitro* or *in vivo;* an example of one such mutant comprises a mutation in positions 424 and/or 494. In some of these embodiments, the β chain is linked (for e.g., by a disulfide bond) to at least a portion of the HGF alpha chain (or functional equivalents thereof). In some embodiments, the β chain is linked (for e.g., by a disulfide bond) to substantially all of the HGF alpha chain (or functional equivalents thereof). In some embodiments, the β chain is not linked to an HGF alpha chain sequence (or functional equivalents thereof). Other substances or molecules can be obtained by screening or identification methods of the invention. In some instances, the substance or molecule can be the product of modifying iterations of a starting substance or molecule designed based on the information provided herein, for e.g., based on small molecule scaffolds or peptide sequence predicted to interact with a functionally-significant residue, including but not limited to residues in the activation domain, active region, and/or specific residues (such as one or more of residues 534, 578, 619, 673, 692, 693, 694, 695, 696, 699 and/or 702) of HGF β chain (e.g., residues in the protease-like domain).

In any molecule of the invention wherein one or more positions is mutated relative to the wild type counterpart sequence, the mutation can be of any form that reduces or eliminates (or in some instances increases) the functional effect of the corresponding wild type residue. A mutation can be obtained in any suitable form known in the art (and/or determined empirically), e.g. by substitution, insertion, addition and/or deletion. In some embodiment, a mutation comprises a non-conservative substitution. Suitable substitutions include but are not limited to those described herein (in particular in the Examples), e.g. with amino acids such as alanine and serine.

In one aspect, a molecule/substance (e.g., HGF/c-met modulators as described herein) is linked to a toxin such as a cytotoxic agent. These molecules/substances can be formulated or administered in combination with an additive/enhancing agent, such as a radiation and/or chemotherapeutic agent.

The invention also provides methods and compositions useful for modulating disease states associated with dysregulation of the HGF/c-met signaling axis. Thus, in one aspect, the invention provides a method of modulating c-met activation in a subject, said method comprising administering to the subject an HGF/c-met modulator molecule of the invention (for e.g., a substance that inhibits specific binding of wild type (native) HGF β chain to c-met), whereby c-met activation is modulated. In one embodiment, said molecule is an HGF/c-met antagonist that inhibits HGF/c-met activity. In one embodiment, said antagonist inhibits specific binding of HGF β to c-met. In one embodiment, said molecule is an agonist that increases HGF/c-met activity. In one embodiment, said agonist has increased or effects increased specific binding of HGF β to c-met. In one aspect, the invention provides a method of treating a pathological condition associated with activation of c-met in a subject, said method comprising administering to the subject a c-met antagonist of the invention (for e.g., a substance that inhibits specific binding of wild type (native) HGF β chain to c-met), whereby c-met activation is inhibited.

The HGF/c-met signaling pathway is involved in multiple biological and physiological functions, including, for e.g., cell growth stimulation (e.g. cell proliferation, cell survival, cell migration, cell morphogenesis) and angiogenesis. Thus, in another aspect, the invention provides a method of inhibiting c-met activated cell growth (e.g. proliferation and/or survival), said method comprising contacting a cell or tissue with a c-met antagonist of the invention (for e.g., a substance that inhibits specific binding of wild type (native) HGF β chain to c-met), whereby cell proliferation associated with c-met activation is inhibited. In yet another aspect, the invention provides a method of modulating angiogenesis, said method comprising administering to a cell, tissue, and/or subject with a condition associated with abnormal angiogenesis a c-met antagonist of the invention (for e.g., a substance that inhibits specific binding of wild type (native) HGF β chain to c-met), whereby angiogenesis is modulated.

In one aspect, the invention provides use of a c-met antagonist of the invention in the preparation of a medicament for the therapeutic and/or prophylactic treatment of a disease, such as a cancer, a tumor, a cell proliferative disorder, an immune (such as autoimmune) disorder and/or an angiogenesis-related disorder. The c-met antagonist can be of any form described herein, including antibody, antibody fragment, small molecule (for e.g., an organic molecule), polypeptide (for e.g., an oligopeptide), nucleic acid (for e.g., an oligonucleotide, such as an antisense oligonucleotide), an aptamer, or combination thereof.

In one aspect, the invention provides use of a nucleic acid of the invention in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides use of an expression vector of the invention in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides use of a host cell of the invention in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides use of an article of manufacture of the invention in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides use of a kit of the invention in the preparation of a medicament for the therapeutic and/or prophylactic treatment of a disease, such as a cancer, a tumor, a cell proliferative disorder, an immune (such as autoimmune) disorder and/or an angiogenesis-related disorder.

In one aspect, the invention provides a method of inhibiting c-met activated cell proliferation, said method comprising contacting a cell or tissue with an effective amount of a c-met antagonist of the invention, whereby cell proliferation associated with c-met activation is inhibited.

In one aspect, the invention provides a method of treating a pathological condition associated with dysregulation of c-met activation in a subject, said method comprising administering to the subject an effective amount of a c-met antagonist of the invention, whereby said condition is treated.

In one aspect, the invention provides a method of inhibiting the growth of a cell that expresses c-met or hepatocyte growth factor, or both, said method comprising contacting said cell with a c-met antagonist of the invention thereby causing an inhibition of growth of said cell. In one embodiment, the cell is contacted by HGF expressed by a different cell (for e.g., through a paracrine effect).

In one aspect, the invention provides a method of therapeutically treating a mammal having a cancerous tumor comprising a cell that expresses c-met or hepatocyte growth factor, or both, said method comprising administering to said mammal an effective amount of a c-met antagonist of the invention, thereby effectively treating said mammal. In one embodiment, the cell is contacted by HGF expressed by a different cell (for e.g., through a paracrine effect).

In one aspect, the invention provides a method for treating or preventing a cell proliferative disorder associated with increased expression or activity of c-met or hepatocyte growth factor, or both, said method comprising administering to a subject in need of such treatment an effective amount of a c-met antagonist of the invention, thereby effectively treating or preventing said cell proliferative disorder. In one embodiment, said proliferative disorder is cancer.

In one aspect, the invention provides a method for inhibiting the growth of a cell, wherein growth of said cell is at least in part dependent upon a growth potentiating effect of c-met or hepatocyte growth factor, or both, said method comprising contacting said cell with an effective amount of a c-met antagonist of the invention, thereby inhibiting the growth of said cell. In one embodiment, the cell is contacted by HGF expressed by a different cell (for e.g., through a paracrine effect).

In one aspect, the invention provides a method of therapeutically treating a tumor in a mammal, wherein the growth of said tumor is at least in part dependent upon a growth potentiating effect of c-met or hepatocyte growth factor, or both, said method comprising contacting said cell with an effective amount of a c-met antagonist of the invention, thereby effectively treating said tumor. In one embodiment, the cell is contacted by HGF expressed by a different cell (for e.g., through a paracrine effect).

Methods of the invention can be used to affect any suitable pathological state, for example, cells and/or tissues associated with dysregulation of the HGF/c-met signaling pathway. In one embodiment, a cell that is targeted in a method of the invention is a cancer cell. For example, a cancer cell can be one selected from the group consisting of a breast cancer cell, a colorectal cancer cell, a lung cancer cell, a papillary carcinoma cell (for e.g., of the thyroid gland), a colon cancer cell, a pancreatic cancer cell, an ovarian cancer cell, a cervical cancer cell, a central nervous system cancer cell, an osteogenic sarcoma cell, a renal carcinoma cell, a hepatocellular carcinoma cell, a bladder cancer cell, a gastric carcinoma cell, a head and neck squamous carcinoma cell, a melanoma cell and a leukemia cell. In one embodiment, a cell that is targeted in a method of the invention is a hyperproliferative and/or hyperplastic cell. In one embodiment, a cell that is targeted in a method of the invention is a dysplastic cell. In yet another embodiment, a cell that is targeted in a method of the invention is a metastatic cell.

Methods of the invention can further comprise additional treatment steps. For example, in one embodiment, a method further comprises a step wherein a targeted cell and/or tissue (for e.g., a cancer cell) is exposed to radiation treatment or a chemotherapeutic agent.

As described herein, c-met activation is an important biological process the dysregulation of which leads to numerous pathological conditions. Accordingly, in one embodiment of methods of the invention, a cell that is targeted (for e.g., a cancer cell) is one in which activation of c-met is enhanced as compared to a normal cell of the same tissue origin. In one embodiment, a method of the invention causes the death of a targeted cell. For example, contact with an antagonist of the invention may result in a cell's inability to signal through the c-met pathway, which results in cell death.

Dysregulation of c-met activation (and thus signaling) can result from a number of cellular changes, including, for example, overexpression of HGF (c-met's cognate ligand) and/or c-met itself. Accordingly, in some embodiments, a method of the invention comprises targeting a cell wherein c-met or hepatoctye growth factor, or both, is more abundantly expressed by said cell (for e.g., a cancer cell) as compared to a normal cell of the same tissue origin. A c-met-expressing cell can be regulated by HGF from a variety of sources, i.e. in an autocrine or paracrine manner. For example, in one embodiment of methods of the invention, a targeted cell is contacted/bound by hepatocyte growth factor expressed in a different cell (for e.g., via a paracrine effect). Said different cell can be of the same or of a different tissue origin. In one embodiment, a targeted cell is contacted/bound by HGF expressed by the targeted cell itself (for e.g., via an autocrine effect/loop).

In one aspect, the invention provides a method comprising administering to a subject an HGF variant capable of effecting HGF biological activity at a supra-normal level (e.g., less than the level of activity obtained with a similar amount of wild type HGF under similar therapeutic conditions), wherein HGF activity is desired at a sub-optimal (i.e., less than wild type) levels, whereby the desired amount of HGF biological activity is achieved. In one embodiment, said HGF variant comprises a mutation at one or more of positions 534, 578, 619, 673, 692, 693, 694, 695, 696, 699 and/or 702. In one embodiment, said HGF variant comprises a mutation within HGF that renders it incapable of being activated (e.g., cleaved) *in vitro* or *in vivo;* an example of one such mutant comprises a mutation in positions 424 and/or 494. Suitable conditions to be treated by this method include any pathological conditions that are associated with an abnormally/undesirably low physiological level of HGF/c-met activity in a subject, and wherein there is a need to tightly regulate the amount of HGF/c-met activity induced by a therapeutic agent. Examples of such conditions include but are not limited to wound healing, cardiac hypertrophy, cardiac infarction, limb ischemia, peripheral arterial disease, etc.

Any of the c-met antagonists of the invention can be used in methods of the invention. For example, in some embodiments of methods of the invention, a c-met antagonist is a substance or molecule comprising, consisting or consisting essentially of an activated HGF β chain (or functional equivalent thereof), which in some embodiments is not disulfide linked to an HGF alpha chain (or functional equivalent thereof). In some embodiments, the substance or molecule comprises, consists or consists essentially of an activated HGF β chain (or functional equivalent thereof) comprising a mutation in one or more of positions 534, 578, 619, 673, 692, 693, 694, 695, 696, 699 and/or 702 (including any of the combinations described herein). In some embodiments, the activated chain is linked (for e.g., by a disulfide bond) to at least a portion of an HGF alpha chain (or functional equivalents thereof). In some embodiments, the activated β chain is linked (for e.g., by a disulfide bond) to substantially all of an HGF alpha chain (or functional equivalents thereof). In some embodiments, the activated β chain is not linked to an HGF alpha chain sequence (or functional equivalents thereof).

In some embodiments of methods of the invention, the substance or molecule is or comprises a small molecule, peptide, antibody, antibody fragment, aptamer, or a combination thereof.

In some embodiments of methods of the invention, a c-met antagonist is a substance or molecule comprising, consisting or consisting essentially of a peptide, which in some embodiments comprises, consists or consists essentially of the sequence VDWVCFRDLGCDWEL. In some embodiments, the peptide comprises, consists or consists essentially of an amino acid sequence having at least 50%, 60%, 70%, 80%, 90%, 95%, 98% sequence identity or similarity with the sequence VDWVCFRDLGCDWEL. In one embodiment, a variant of this sequence is any of those depicted in Table 1 above. In some embodiments, these peptides comprise modifications that enhance their inhibitory and/or therapeutic effect (including, for e.g., enhanced affinity, improved pharmacokinetics properties (such as half life, stability, clearance rate), reduced toxicity to the subject). Such modifications include, for e.g., modifications involving glycosylation, pegylation, substitution with non-naturally occurring but functionally equivalent amino acid, linking groups, etc. Suitable modifications are well known in the art, and furthermore can be determined empirically as necessary.

In some embodiments, methods of the invention utilize a substance or molecule obtained by any of the screening and/or identification methods of the invention.

In some embodiments of methods and compositions of the invention, a substance/molecule that inhibits HGF/c-met signaling does not substantially interfere with binding interaction between cellular components other than activated HGF β chain and c-met. For example, in one embodiment, the substance/molecule does not substantially interfere with binding of HGF α chain to c-met.

In one aspect, the invention provides compositions comprising one or more substances/molecules (for e.g., HGF/c-met antagonists) of the invention and a carrier. In one embodiment, the carrier is pharmaceutically acceptable.

In one aspect, the invention provides nucleic acids encoding a substance/molecule (for e.g., a HGF/c-met antagonist) of the invention. In one embodiment, a nucleic acid of the invention encodes a substance/molecule (for e.g., a HGF/c-met antagonist) which is or comprises a polypeptide (for e.g., an oligopeptide). In one embodiment, a nucleic acid of the invention encodes a substance/molecule (for e.g., a HGF/c-met antagonist) which is or comprises an antibody or fragment thereof.

In one aspect, the invention provides vectors comprising a nucleic acid of the invention.

In one aspect, the invention provides host cells comprising a nucleic acid or a vector of the invention. A vector can be of any type, for example a recombinant vector such as an expression vector. Any of a variety of host cells can be used. In one embodiment, a host cell is a prokaryotic cell, for example, *E. coli.* In one embodiment, a host cell is a eukaryotic cell, for example a mammalian cell such as Chinese Hamster Ovary (CHO) cell.

In one aspect, the invention provides methods for making a substance/molecule (for e.g., a HGF/c-met antagonist) of the invention. For example, the invention provides a method of making a c-met antagonist which is or comprises an antibody (or fragment thereof), said method comprising expressing in a suitable host cell a recombinant vector of the invention encoding said antibody (or fragment thereof), and recovering said antibody. In another example, the invention provides a method of making a substance/molecule (for e.g., a HGF/c-met antagonist) which is or comprises a polypeptide (such as an oligopeptide), said method comprising expressing in a suitable host cell a recombinant vector of the invention encoding said polypeptide (such as an oligopeptide), and recovering said polypeptide (such as an oligopeptide).

In one aspect, the invention provides an article of manufacture comprising a container; and a composition contained within the container, wherein the composition comprises one or more substances/molecules (for e.g., HGF/c-met antagonists) of the invention. In one embodiment, the composition comprises a nucleic acid of the invention. In one embodiment, a composition comprising a substance/molecule (for e.g., a HGF/c-met antagonist) further comprises a carrier, which in some embodiments is pharmaceutically acceptable. In one embodiment, an article of manufacture of the invention further comprises instructions for administering the composition (for e.g., the antagonist) to a subject.

In one aspect, the invention provides a kit comprising a first container comprising a composition comprising one or more substances/molecules (for e.g., HGF/c-met antagonists) of the invention; and a second container comprising a buffer. In one embodiment, the buffer is pharmaceutically acceptable. In one embodiment, a composition comprising a substance/molecule (for e.g., a HGF/c-met antagonist) further comprises a carrier, which in some embodiments is pharmaceutically acceptable. In one embodiment, a kit further comprises instructions for administering the composition (for e.g., the antagonist) to a subject.

BRIEF DESCRIPTION OF THE DRAWINGS

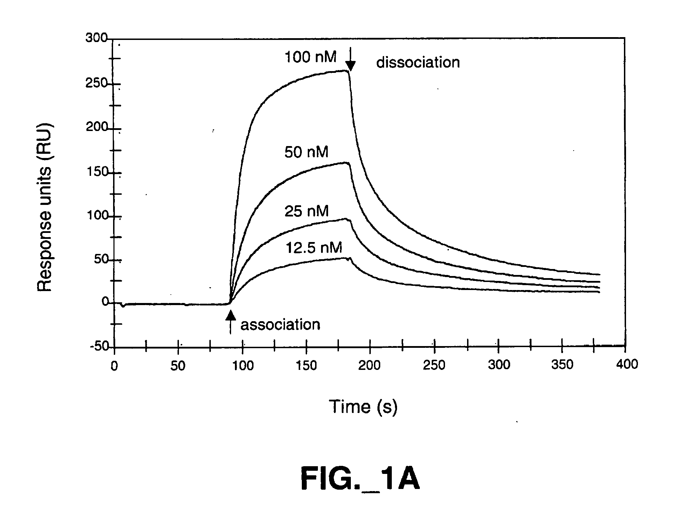

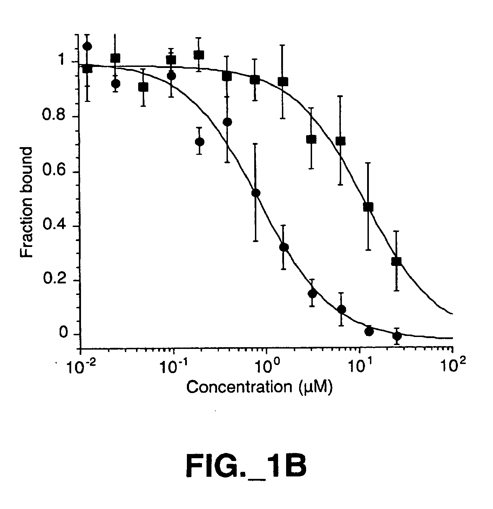

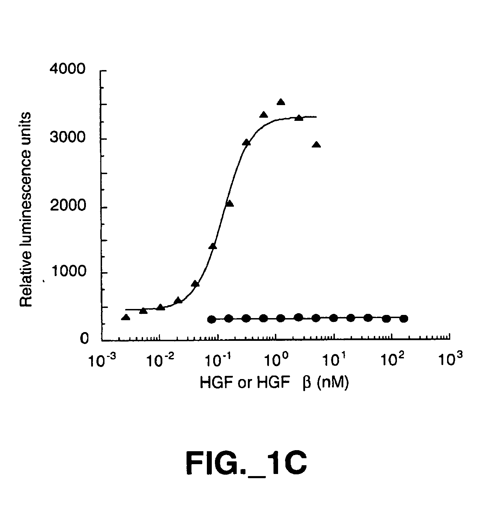

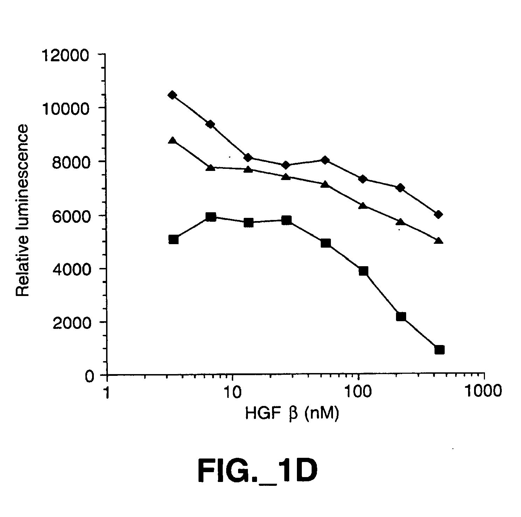

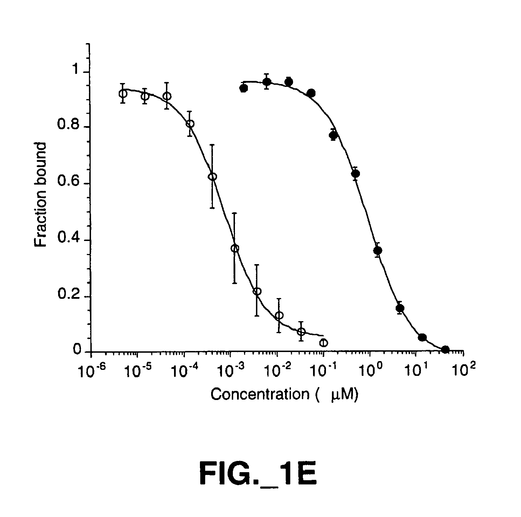

FIG. 1 HGF β Direct and Competition Binding and Activity in Met Phosphorylation Assays.

(A) Binding of HGF β to the extracellular domain of Met (Met ECD) by surface plasmon resonance. Met ECD was captured on a CM5 chip at -2000 resonance units. HGF β was injected in a series of concentrations from 12.5 nM to 100 nM. Arrows indicate the onset of the association and dissociation phases. Data were analyzed by Global Fit using a 1:1 binding model from which $k_{on}$, $k_{off}$ and $K_d$ values were determined.

(B) HGF β/Met-IgG competition ELISA. Met-IgG was captured on a plate coated with rabbit anti-human IgG Fc and incubated with a mixture containing 250 nM maleimide-coupled biotinylated wildtype HGF β and a series of concentrations of unlabeled HGF β (●) and proHGF β (■). The amount of biotinylated wildtype HGF β bound on the plate was detected by neutravidin-HRP. Data from at least 3 independent determinations each were normalized, averaged and fitted by a four parameter fit using Kaleidagraph from which $IC_{50}$ values were determined; error bars represent standard deviations.

(C) HGF-dependent phosphorylation of Met in A549 cells was carried out as described in the Examples using HGF (A) and HGF β (●).

(D) Inhibition of HGF-dependent phosphorylation of Met was carried out in duplicate as described in Examples using HGF at 0.5 nM (♦), 0.25 nM (▲) and 0.125 nM (■) to stimulate A549 cells in the presence of increasing concentrations of HGF β. (E) Full length HGF/Met-IgG competition ELISA. This was carried out similarly to (A) using 1 nM NHS-coupled biotinylated HGF and a series of concentrations of unlabeled HGF (○), and HGF β (●). Data from 3 independent determinations each were normalized, averaged and fitted as above.

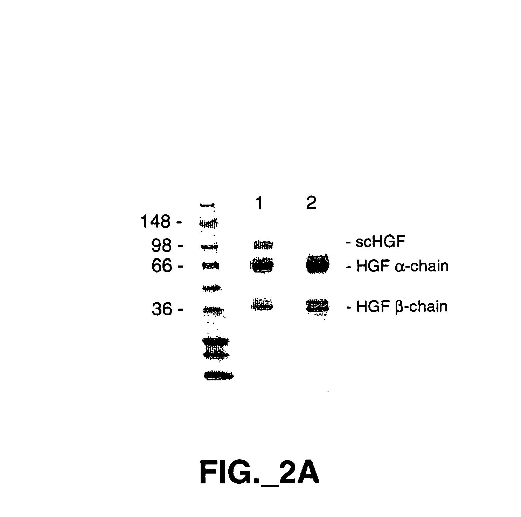

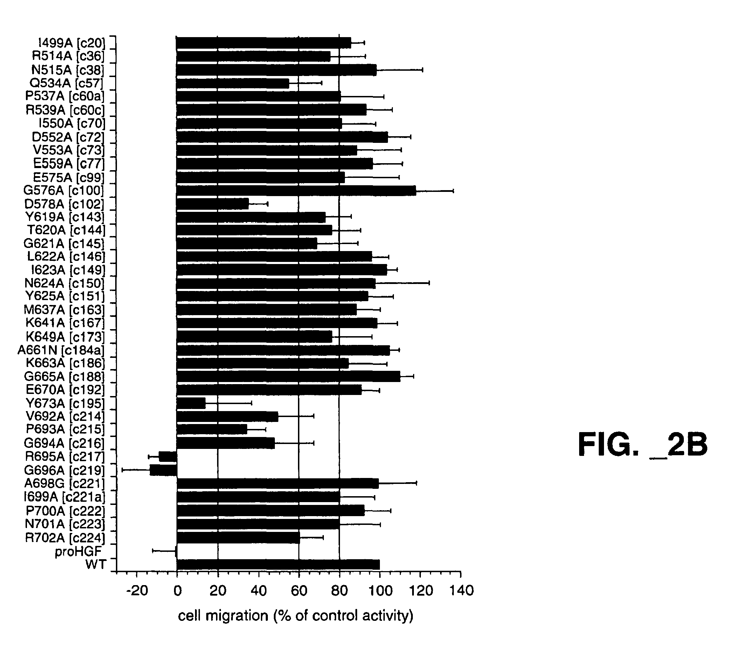

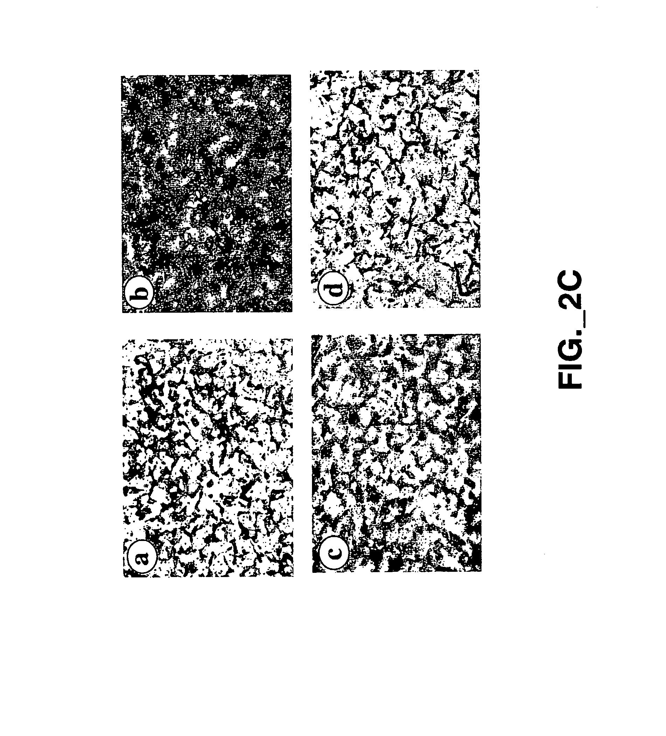

FIG. 2 HGF-dependent cell migration by HGF mutants. (A) Representative purity of HGF mutants. The purity of all HGF mutants analyzed by SDS-PAGE under reducing conditions is illustrated for cation exchange purified HGF 1623A. Incomplete conversion of the secreted single-chain form by CHO expression in 1 % FBS (v/v) is shown in lane 1. Additional exposure to 5% FBS completed the activation process yielding pure two-chain HGF I623A (lane 2). Molecular weight markers are shown as $M_r$ x $10^3$. B) Migration of MDA-MB435 cells in a transwell assay in the presence of I nM HGF mutants. Activities are expressed as percent migration of control cells exposed to 1 nM wildtype HGF; full length HGF sequence numbering [chymotrypsinogen numbering] are shown. Values represent the averages of 4-8 independent experiments ± SD. (C) Photographs of MDA-MB435 cell migration in the absence of wildtype HGF (a), with 1 nM wildtype HGF (b), 1 nM HGF R695A (c) and 1 nM HGF G696A (d).

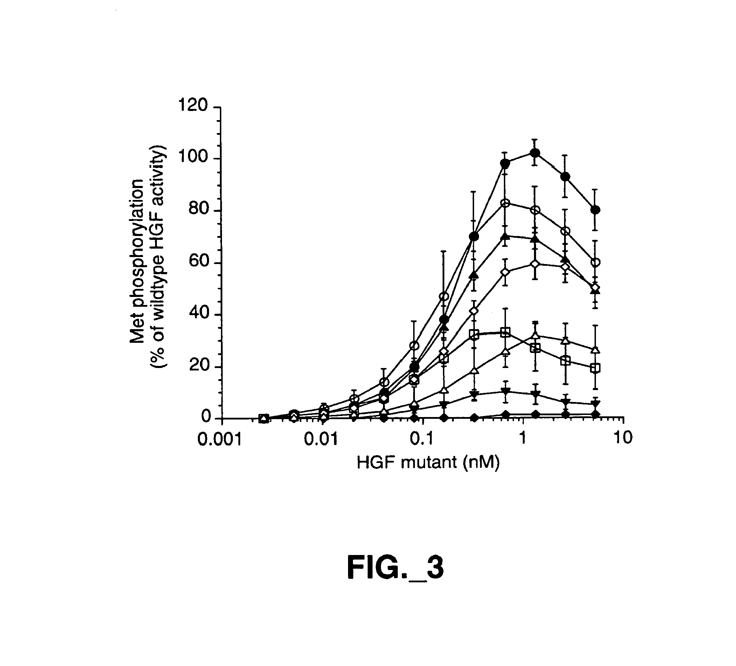

FIG. 3 HGF-dependent phosphorylation of Met by HGF mutants. Phosphorylation of Met of A549 cells was carried out as described in Examples using various concentrations of HGF (●), proHGF (♦), HGF Q534A (○), HGF D578A (▲), HGF Y673A (Δ), HGF V692A (◊), HGF R695A (□) and HGF G696A (▼).

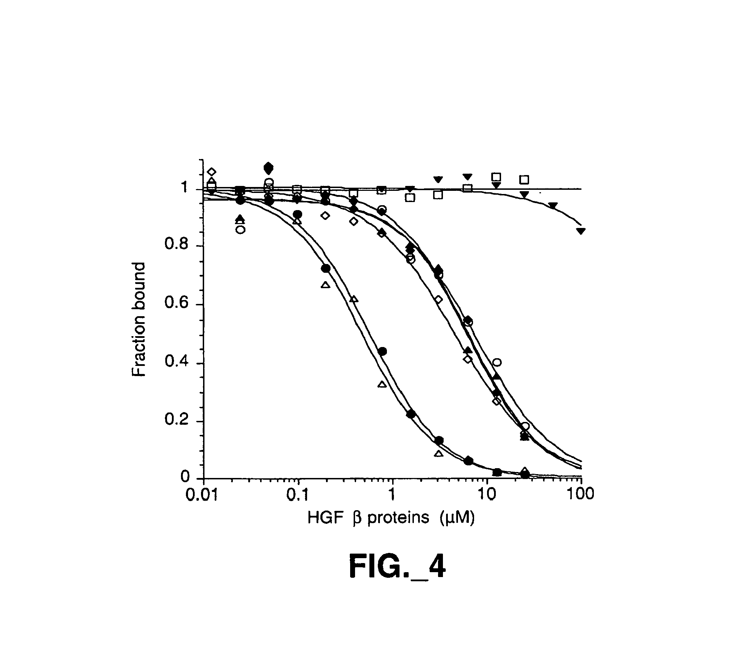

FIG. 4 Met competition binding of HGF β mutants. HGF β/Met-IgG competition ELISA was used to assess Met binding of wildtype HGF β (Δ), HGF β (●) and HGF β mutants Q534A [c57] (○), D578A [c102] (▲), Y619A [c143] (◊), R695A [c217] (□), G696A [c219] (▼) and 1699A [c221a] (♦). Data were fit by a four parameter fit using Kaleidagraph; representative individual competition assays are shown for multiple independent determinations where n ≥ 3.

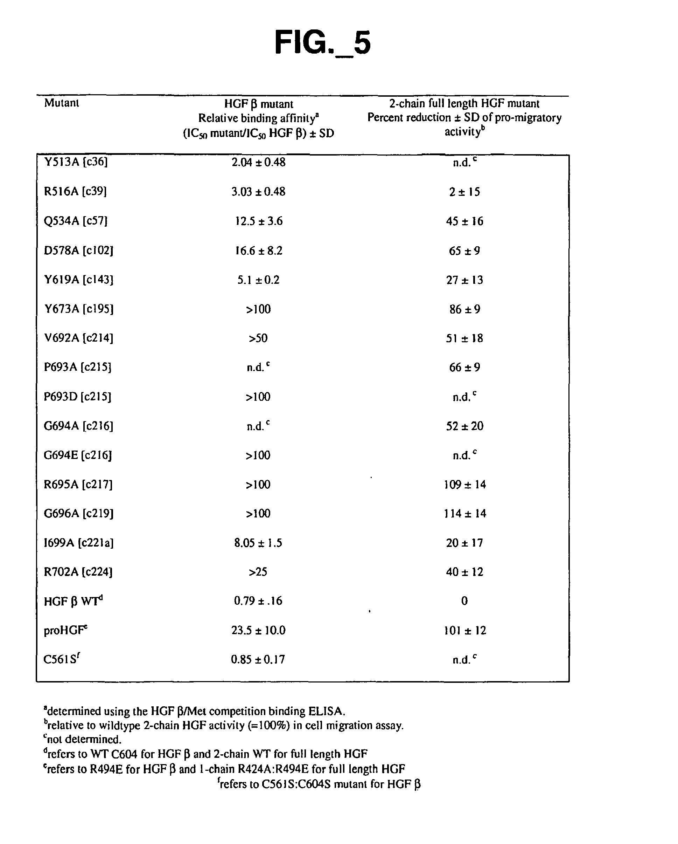

FIG. 5 Effects of mutations of the β-chain in HGF β and 2-chain HGF. Met competition binding data of HGF β mutants in the HGF β/Met competition binding ELISA and cell migration activity of 2-chain HGF mutants in the MDA-MB-435 cell migration assay are shown. HGF β-chain mutants were made in C604S background unless noted otherwise.

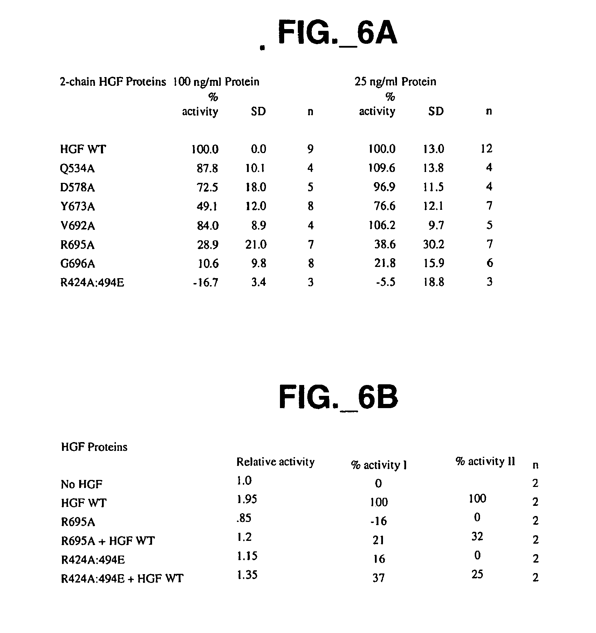

FIG. 6 Effect of HGF mutations on stimulation and inhibition of cell proliferation. (A) HGF-dependent cell proliferation by 2-chain HGF and 2-chain HGF mutants (having the indicated mutations) in BxPC3 cells. (B) Inhibition of HGF-dependent cell proliferation by 2-chain HGF mutant in HGF β chain (having the indicated mutations) and by 1-chain HGF in BxPC3 cells.

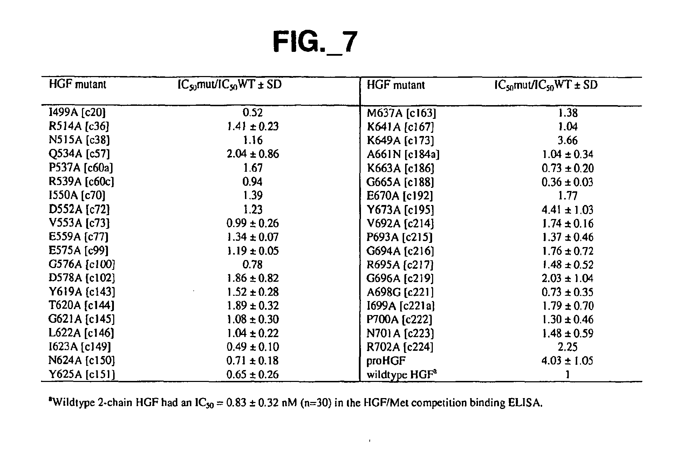

FIG. 7 Relative binding affinities of HGF mutants to Met determined by the HGF/Met competition binding ELISA.

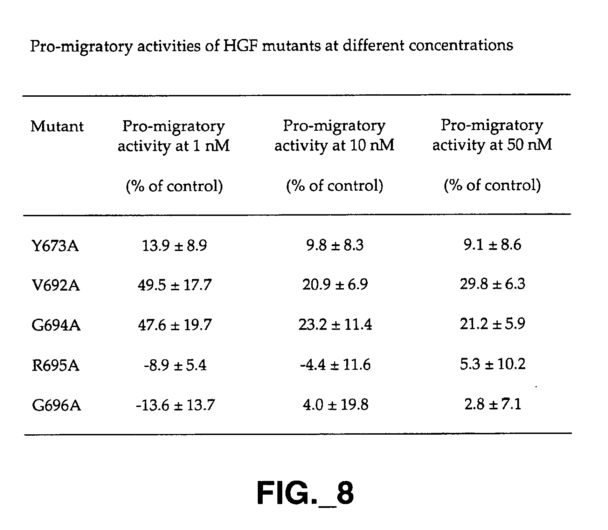

FIG. 8 Pro-migratory activities of HGF mutants at different concentrations.

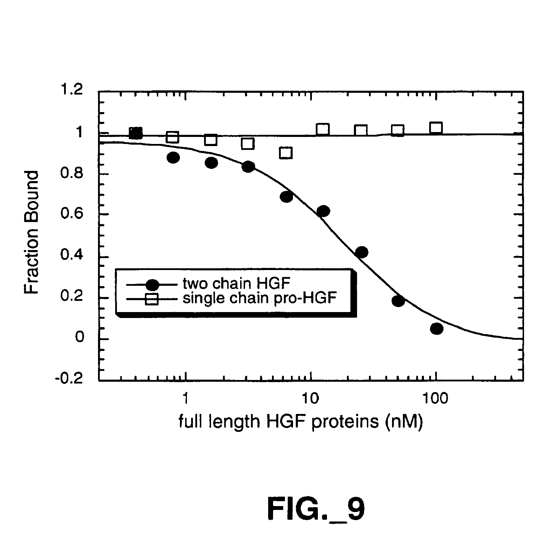

FIG. 9 Analysis of single chain pro-HGF and two chain HGF binding to c-Met-IgG determined by HGF β chain competition ELISA. The data were fitted by a four parameter fit using Kaleidagraph and $IC_{50}$ for two chain HGF was determined. Single chain pro-HGF at concentrations up to 100 nM did not compete with HGF β chain binding to c-Met-IgG.

## MODES FOR CARRYING OUT THE INVENTION

The invention provides methods, compositions, kits and articles of manufacture for identifying inhibitors of the HGF/c-met signaling pathway (in particular, inhibitors of HGF β chain binding to c-met), and methods of using such inhibitors..

Details of these methods, compositions, kits and articles of manufacture are provided herein.

*General Techniques*

The practice of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al., eds., 1987, and periodic updates); "PCR: The Polymerase Chain Reaction", (Mullis et al., ed., 1994); "A Practical Guide to Molecular Cloning" (Perbal Bernard V., 1988).

*Definitions*

"Percent (%) amino acid sequence identity" with respect to a peptide (for e.g., VDWVCFRDLGCDWEL) or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table A below. The ALIGN-2 sequence comparison computer program was authored by Genentech, Inc. and the source code shown in Table A below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Figure 8 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

Unless specifically stated otherwise, all % amino acid sequence identity values used herein are obtained as described in the immediately preceding paragraph using the ALIGN-2 computer program.

## Table A

```
/*
*
* C-C increased from 12 to 15
* Z is average of EQ
* B is average of ND
* match with stop is _M; stop-stop = 0; J (joker) match = 0
*/
#define _M      -8        /* value of a match with a stop */


int     _day[26][26] = {
/*      A B C D E F G H I J K L M N O P Q R S T U V W X Y Z */
/* A */  { 2, 0,-2, 0, 0,-4, 1,-1,-1, 0,-1,-2,-1, 0,_M, 1, 0,-2, 1, 1, 0, 0,-6, 0,-3, 0},
/* B */  { 0, 3,-4, 3, 2,-5, 0, 1,-2, 0, 0,-3,-2, 2,_M,-1, 1, 0, 0, 0, 0,-2,-5, 0,-3, 1},
/* C */  {-2,-4,15,-5,-5,-4,-3,-3,-2, 0,-5,-6,-5,-4,_M,-3,-5,-4, 0,-2, 0,-2,-8, 0, 0,-5},
/* D */  { 0, 3,-5, 4, 3,-6, 1, 1,-2, 0, 0,-4,-3, 2,_M,-1, 2,-1, 0, 0, 0,-2,-7, 0,-4, 2},
/* E */  { 0, 2,-5, 3, 4,-5, 0, 1,-2, 0, 0,-3,-2, 1,_M,-1, 2,-1, 0, 0, 0,-2,-7, 0,-4, 3},
/* F */  {-4,-5,-4,-6,-5, 9,-5,-2, 1, 0,-5, 2, 0,-4,_M,-5,-5,-4,-3,-3, 0,-1, 0, 0, 7,-5},
/* G */  { 1, 0,-3, 1, 0,-5, 5,-2,-3, 0,-2,-4,-3, 0,_M,-1,-1,-3, 1, 0, 0,-1,-7, 0,-5, 0},
/* H */  {-1, 1,-3, 1, 1,-2,-2, 6,-2, 0, 0,-2,-2, 2,_M, 0, 3, 2,-1,-1, 0,-2,-3, 0, 0, 2},
/* I */  {-1,-2,-2,-2,-2, 1,-3,-2, 5, 0,-2, 2, 2,-2,_M,-2,-2,-2,-1, 0, 0, 4,-5, 0,-1,-2},
/* J */  { 0, 0, 0, 0, 0, 0, 0, 0, 0, 0, 0, 0, 0, 0,_M, 0, 0, 0, 0, 0, 0, 0, 0, 0, 0, 0},
/* K */  {-1, 0,-5, 0, 0,-5,-2, 0,-2, 0, 5,-3, 0, 1,_M,-1, 1, 3, 0, 0, 0,-2,-3, 0,-4, 0},
/* L */  {-2,-3,-6,-4,-3, 2,-4,-2, 2, 0,-3, 6, 4,-3,_M,-3,-2,-3,-3,-1, 0, 2,-2, 0,-1,-2},
```

```
/* M */   {-1,-2,-5,-3,-2, 0,-3,-2, 2, 0, 0, 4, 6,-2,_M,-2,-1, 0,-2,-1, 0, 2,-4, 0,-2,-1},
/* N */   { 0, 2,-4, 2, 1,-4, 0, 2,-2, 0, 1,-3,-2, 2,_M,-1, 1, 0, 1, 0, 0,-2,-4, 0,-2, 1},
/* O */   {_M,_M,_M,_M,_M,_M,_M,_M,_M,_M,_M,_M,_M,_M,
           0,_M,_M,_M,_M,_M,_M,_M,_M,_M,_M,_M},
/* P */   { 1,-1,-3,-1,-1,-5,-1, 0,-2, 0,-1,-3,-2,-1,_M, 6, 0, 0, 1, 0, 0,-1,-6, 0,-5, 0},
/* Q */   { 0, 1,-5, 2, 2,-5,-1, 3,-2, 0, 1,-2,-1, 1,_M, 0, 4, 1,-1,-1, 0,-2,-5, 0,-4, 3},
/* R */   {-2, 0,-4,-1,-1,-4,-3, 2,-2, 0, 3,-3, 0, 0,_M, 0, 1, 6, 0,-1, 0,-2, 2, 0,-4, 0},
/* S */   { 1, 0, 0, 0, 0,-3, 1,-1,-1, 0, 0,-3,-2, 1,_M, 1,-1, 0, 2, 1, 0,-1,-2, 0,-3, 0},
/* T */   { 1, 0,-2, 0, 0,-3, 0,-1, 0, 0, 0,-1,-1, 0,_M, 0,-1,-1, 1, 3, 0, 0,-5, 0,-3, 0},
/* U */   { 0, 0, 0, 0, 0, 0, 0, 0, 0, 0, 0, 0, 0, 0, 0,_M, 0, 0, 0, 0, 0, 0, 0, 0, 0, 0, 0},
/* V */   { 0,-2,-2,-2,-2,-1,-1,-2, 4, 0,-2, 2, 2,-2,_M,-1,-2,-2,-1, 0, 0, 4,-6, 0,-2,-2},
/* W */   {-6,-5,-8,-7,-7, 0,-7,-3,-5, 0,-3,-2,-4,-4,_M,-6,-5, 2,-2,-5, 0,-6,17, 0, 0,-6},
/* X */   { 0, 0, 0, 0, 0, 0, 0, 0, 0, 0, 0, 0, 0, 0, 0,_M, 0, 0, 0, 0, 0, 0, 0, 0, 0, 0, 0},
/* Y */   {-3,-3, 0,-4,-4, 7,-5, 0,-1, 0,-4,-1,-2,-2,_M,-5,-4,-4,-3,-3, 0,-2, 0, 0,10,-4},
/* Z */   { 0, 1,-5, 2, 3,-5, 0, 2,-2, 0, 0,-2,-1, 1,_M, 0, 3, 0, 0, 0, 0,-2,-6, 0,-4, 4}
};


/*
 */
#include <stdio.h>
#include <ctype.h>


#define MAXJMP    16      /* max jumps in a diag */
#define MAXGAP    24      /* don't continue to penalize gaps larger than this */
#define JMPS      1024    /* max jmps in an path */
#define MX        4       /* save if there's at least MX-1 bases since last jmp */


#define DMAT      3       /* value of matching bases */
#define DMIS      0       /* penalty for mismatched bases */
#define DINS0     8       /* penalty for a gap */
#define DINS1     1       /* penalty per base */
#define PINS0     8       /* penalty for a gap */
#define PINS1     4       /* penalty per residue */


struct jmp {
        short         n[MAXJMP];   /* size of jmp (neg for dely) */
```

13

```
        unsigned short x[MAXJMP];   /* base no. of jmp in seq x */
};                                  /* limits seq to 2^16 -1 */


struct diag {
        int             score;      /* score at last jmp */
        long            offset;     /* offset of prev block */
        short           ijmp;       /* current jmp index */
        struct jmp      jp;         /* list of jmps */
};


struct path {
        int     spc;            /* number of leading spaces */
        short   n[JMPS];        /* size of jmp (gap) */
        int     x[JMPS];        /* loc of jmp (last elem before gap) */
};


char            *ofile;             /* output file name */
char            *namex[2];          /* seq names: getseqs() */
char            *prog;              /* prog name for err msgs */
char            *seqx[2];               /* seqs: getseqs() */
int             dmax;               /* best diag: nw() */
int             dmax0;              /* final diag */
int             dna;                /* set if dna: main() */
int             endgaps;                /* set if penalizing end gaps */
int             gapx, gapy;         /* total gaps in seqs */
int             len0, len1;         /* seq lens */
int             ngapx, ngapy;       /* total size of gaps */
int             smax;               /* max score: nw() */
int             *xbm;               /* bitmap for matching */
long            offset;             /* current offset in jmp file */
struct  diag    *dx;                /* holds diagonals */
struct  path    pp[2];              /* holds path for seqs */


char            *calloc(), *malloc(), *index(), *strcpy();
char            *getseq(), *g_calloc();
```

```
/* Needleman-Wunsch alignment program
 *
 * usage: progs file1 file2
 *   where file1 and file2 are two dna or two protein sequences.
 *   The sequences can be in upper- or lower-case an may contain ambiguity
 *   Any lines beginning with ';', '>' or '<' are ignored
 *   Max file length is 65535 (limited by unsigned short x in the jmp struct)
 *   A sequence with 1/3 or more of its elements ACGTU is assumed to be DNA
 *   Output is in the file "align.out"
 *
 * The program may create a tmp file in /tmp to hold info about traceback.
 * Original version developed under BSD 4.3 on a vax 8650
 */
#include "nw.h"
#include "day.h"


static   _dbval[26] = {
         1,14,2,13,0,0,4,11,0,0,12,0,3,15,0,0,0,5,6,8,8,7,9,0,10,0
};


static   _pbval[26] = {
         1, 2|(1<<('D'-'A'))|(1<<('N'-'A')), 4, 8, 16, 32, 64,
         128, 256, 0xFFFFFFF, 1<<10, 1<<11, 1<<12, 1<<13, 1<<14,
         1<<15, 1<<16, 1<<17, 1<<18, 1<<19, 1<<20, 1<<21, 1<<22,
         1<<23, 1<<24, 1<<25|(1<<('E'-'A'))|(1<<('Q'-'A'))
};


main(ac, av)                                                           main
         int      ac;
         char     *av[];
{
         prog = av[0];
         if (ac != 3) {
                 fprintf(stderr,"usage: %s file1 file2\n", prog);
```

```
                fprintf(stderr,"where file1 and file2 are two dna or two protein sequences.\n");
                fprintf(stderr,"The sequences can be in upper- or lower-case\n");
                fprintf(stderr,"Any lines beginning with ';' or '<' are ignored\n");
                fprintf(stderr,"Output is in the file \"align.out\"\n");
                exit(1);
        }
        namex[0] = av[1];
        namex[1] = av[2];
        seqx[0] = getseq(namex[0], &len0);
        seqx[1] = getseq(namex[1], &len1);
        xbm = (dna)? _dbval : _pbval;


        endgaps = 0;                    /* 1 to penalize endgaps */
        ofile = "align.out";            /* output file */


        nw();           /* fill in the matrix, get the possible jmps */
        readjmps();     /* get the actual jmps */
        print();        /* print stats, alignment */


        cleanup(0);     /* unlink any tmp files */
}




/* do the alignment, return best score: main()
 * dna: values in Fitch and Smith, PNAS, 80, 1382-1386, 1983
 * pro: PAM 250 values
 * When scores are equal, we prefer mismatches to any gap, prefer
 * a new gap to extending an ongoing gap, and prefer a gap in seqx
 * to a gap in seq y.
 */
nw()                                                                  nw
{
        char            *px, *py;               /* seqs and ptrs */
        int             *ndely, *dely;  /* keep track of dely */
        int             ndelx, delx;    /* keep track of delx */
```

```
int            *tmp;          /* for swapping row0, row1 */
int            mis;           /* score for each type */
int            ins0, ins1;    /* insertion penalties */
register       id;            /* diagonal index */
register       ij;            /* jmp index */
register       *col0, *col1;  /* score for curr, last row */
register       xx, yy;        /* index into seqs */


dx = (struct diag *)g_calloc("to get diags", len0+len1+1, sizeof(struct diag));


ndely = (int *)g_calloc("to get ndely", len1+1, sizeof(int));
dely = (int *)g_calloc("to get dely", len1+1, sizeof(int));
col0 = (int *)g_calloc("to get col0", len1+1, sizeof(int));
col1 = (int *)g_calloc("to get col1", len1+1, sizeof(int));
ins0 = (dna)? DINS0 : PINS0;
ins1 = (dna)? DINS1 : PINS1;


smax = -10000;
if (endgaps) {
        for (col0[0] = dely[0] = -ins0, yy = 1; yy <= len1; yy++) {
                col0[yy] = dely[yy] = col0[yy-1] - ins1;
                ndely[yy] = yy;
        }
        col0[0] = 0;      /* Waterman Bull Math Biol 84 */
}
else
        for (yy = 1; yy <= len1; yy++)
                dely[yy] = -ins0;


/* fill in match matrix
*/
for (px = seqx[0], xx = 1; xx <= len0; px++, xx++) {
        /* initialize first entry in col
        */
        if (endgaps) {
                if (xx == 1)
```

```
                col1[0] = delx = -(ins0+ins1);
        else
                col1[0] = delx = col0[0] - ins1;
        ndelx = xx;
}
else {
        col1[0] = 0;
        delx = -ins0;
        ndelx = 0;
}




                                                        ...nw
for (py = seqx[1], yy = 1; yy <= len1; py++, yy++) {
        mis = col0[yy-1];
        if (dna)
                mis += (xbm[*px-'A']&xbm[*py-'A'])? DMAT : DMIS;
        else
                mis += _day[*px-'A'][*py-'A'];


        /* update penalty for del in x seq;
         * favor new del over ongong del
         * ignore MAXGAP if weighting endgaps
         */
        if (endgaps || ndely[yy] < MAXGAP) {
                if (col0[yy] - ins0 >= dely[yy]) {
                        dely[yy] = col0[yy] - (ins0+ins1);
                        ndely[yy] = 1;
                } else {
                        dely[yy] -= ins1;
                        ndely[yy]++;
                }
        } else {
                if (col0[yy] - (ins0+ins1) >= dely[yy]) {
                        dely[yy] = col0[yy] - (ins0+ins1);
                        ndely[yy] = 1;
```

```
        } else
                ndely[yy]++;
}


/* update penalty for del in y seq;
 * favor new del over ongong del
 */
if (endgaps || ndelx < MAXGAP) {
        if (col1[yy-1] - ins0 >= delx) {
                delx = col1[yy-1] - (ins0+ins1);
                ndelx = 1;
        } else {
                delx -= ins1;
                ndelx++;
        }
} else {
        if (col1[yy-1] - (ins0+ins1) >= delx) {
                delx = col1[yy-1] - (ins0+ins1);
                ndelx = 1;
        } else
                ndelx++;
}


/* pick the maximum score; we're favoring
 * mis over any del and delx over dely
 */
```

...nw

```
id = xx - yy + len1 - 1;
if (mis >= delx && mis >= dely[yy])
```

```
                        col1[yy] = mis;
            else if (delx >= dely[yy]) {
                        col1[yy] = delx;
                        ij = dx[id].ijmp;
                        if (dx[id].jp.n[0] && (!dna || (ndelx >= MAXJMP
                        && xx > dx[id].jp.x[ij]+MX) || mis > dx[id].score+DINS0)) {
                                    dx[id].ijmp++;
                                    if (++ij >= MAXJMP) {
                                                writejmps(id);
                                                ij = dx[id].ijmp = 0;
                                                dx[id].offset = offset;
                                                offset += sizeof(struct jmp) + sizeof(offset);
                                    }
                        }
                        dx[id].jp.n[ij] = ndelx;
                        dx[id].jp.x[ij] = xx;
                        dx[id].score = delx;
            }
            else {
                        col1[yy] = dely[yy];
                        ij = dx[id].ijmp;
            if (dx[id].jp.n[0] && (!dna || (ndely[yy] >= MAXJMP
                        && xx > dx[id].jp.x[ij]+MX) || mis > dx[id].score+DINS0)) {
                                    dx[id].ijmp++;
                                    if (++ij >= MAXJMP) {
                                                writejmps(id);
                                                ij = dx[id].ijmp = 0;
                                                dx[id].offset = offset;
                                                offset += sizeof(struct jmp) + sizeof(offset);
                                    }
                        }
                        dx[id].jp.n[ij] = -ndely[yy];
                        dx[id].jp.x[ij] = xx;
                        dx[id].score = dely[yy];
            }
            if (xx == len0 && yy < len1) {
```

```
                              /* last col
                               */
                              if (endgaps)
                                      col1[yy] -= ins0+ins1*(len1-yy);
                              if (col1[yy] > smax) {
                                      smax = col1[yy];
                                      dmax = id;

                                    }

                            }

                    }
                    if (endgaps && xx < len0)
                            col1[yy-1] -= ins0+ins1*(len0-xx);
                    if (col1[yy-1] > smax) {
                            smax = col1[yy-1];
                            dmax = id;

                          }
                    tmp = col0; col0 = col1; col1 = tmp;

              }
      (void) free((char *)ndely);
      (void) free((char *)dely);
      (void) free((char *)col0);
      (void) free((char *)col1);                        }


/*
*
* print() -- only routine visible outside this module
*
* static:
* getmat() -- trace back best path, count matches: print()
* pr_align() -- print alignment of described in array p[]: print()
* dumpblock() -- dump a block of lines with numbers, stars: pr_align()
* nums() -- put out a number line: dumpblock()
* putline() -- put out a line (name, [num], seq, [num]): dumpblock()
* stars() - -put a line of stars: dumpblock()
* stripname() -- strip any path and prefix from a seqname
*/
```

```
#include "nw.h"


#define SPC    3
#define P_LINE        256     /* maximum output line */
#define P_SPC 3        /* space between name or num and seq */


extern  _day[26][26];
int     olen;          /* set output line length */
FILE    *fx;           /* output file */


print()                                                          print
{
        int     lx, ly, firstgap, lastgap;  /* overlap */

        if ((fx = fopen(ofile, "w")) == 0) {
                fprintf(stderr,"%s: can't write %s\n", prog, ofile);
                cleanup(1);
        }
        fprintf(fx, "<first sequence: %s (length = %d)\n", namex[0], len0);
        fprintf(fx, "<second sequence: %s (length = %d)\n", namex[1], len1);
        olen = 60;
        lx = len0;
        ly = len1;
        firstgap = lastgap = 0;
        if (dmax < len1 - 1) {    /* leading gap in x */
                pp[0].spc = firstgap = len1 - dmax - 1;
                ly -= pp[0].spc;
        }
        else if (dmax > len1 - 1) {        /* leading gap in y */
                pp[1].spc = firstgap = dmax - (len1 - 1);
                lx -= pp[1].spc;
        }
        if (dmax0 < len0 - 1) {   /* trailing gap in x */
                lastgap = len0 - dmax0 -1;
                lx -= lastgap;
```

```
        }
        else if (dmax0 > len0 - 1) {        /* trailing gap in y */
                lastgap = dmax0 - (len0 - 1);
                ly -= lastgap;
        }
        getmat(lx, ly, firstgap, lastgap);
        pr_align();
}
```

```
/*
 * trace back the best path, count matches
 */
static
getmat(lx, ly, firstgap, lastgap)                                        getmat
        int     lx, ly;                 /* "core" (minus endgaps) */
        int     firstgap, lastgap;              /* leading trailing overlap */
{
        int             nm, i0, i1, siz0, siz1;
        char            outx[32];
        double          pct;
        register                n0, n1;
        register char   *p0, *p1;

        /* get total matches, score
         */
        i0 = i1 = siz0 = siz1 = 0;
        p0 = seqx[0] + pp[1].spc;
        p1 = seqx[1] + pp[0].spc;
        n0 = pp[1].spc + 1;
        n1 = pp[0].spc + 1;

        nm = 0;
        while ( *p0 && *p1 ) {
```

```
        if (siz0) {
                p1++;
                n1++;
                siz0--;
        }
        else if (siz1) {
                p0++;
                n0++;
                siz1--;
        }
        else {
                if (xbm[*p0-'A']&xbm[*p1-'A'])
                        nm++;
                if (n0++ == pp[0].x[i0])
                        siz0 = pp[0].n[i0++];
                if (n1++ == pp[1].x[i1])
                        siz1 = pp[1].n[i1++];
                p0++;
                p1++;
        }
}


/* pct homology:
 * if penalizing endgaps, base is the shorter seq
 * else, knock off overhangs and take shorter core
 */
if (endgaps)
        lx = (len0 < len1)? len0 : len1;
else
        lx = (lx < ly)? lx : ly;
pct = 100.*(double)nm/(double)lx;
fprintf(fx, "\n");
fprintf(fx, "<%d match%s in an overlap of %d: %.2f percent similarity\n",
        nm, (nm == 1)? "" : "es", lx, pct);
```

24

```
        fprintf(fx, "<gaps in first sequence: %d", gapx);                    ...getmat
        if (gapx) {
                (void) sprintf(outx, " (%d %s%s)",
                        ngapx, (dna)? "base":"residue", (ngapx == 1)? "":"s");
                fprintf(fx,"%s", outx);


        fprintf(fx, ", gaps in second sequence: %d", gapy);
        if (gapy) {
                (void) sprintf(outx, " (%d %s%s)",
                        ngapy, (dna)? "base":"residue", (ngapy == 1)? "":"s");
                fprintf(fx,"%s", outx);
        }
        if (dna)
                fprintf(fx,
                "\n<score: %d (match = %d, mismatch = %d, gap penalty = %d + %d per
base)\n",
                smax, DMAT, DMIS, DINS0, DINS1);
        else
                fprintf(fx,
                "\n<score: %d (Dayhoff PAM 250 matrix, gap penalty = %d + %d per
residue)\n",
                smax, PINS0, PINS1);
        if (endgaps)
                fprintf(fx,
                "<endgaps penalized. left endgap: %d %s%s, right endgap: %d %s%s\n",
                firstgap, (dna)? "base" : "residue", (firstgap == 1)? "" : "s",
                lastgap, (dna)? "base" : "residue", (lastgap == 1)? "" : "s");
        else
                fprintf(fx, "<endgaps not penalized\n");
}


static          nm;             /* matches in core -- for checking */
static          lmax;           /* lengths of stripped file names */
static          ij[2];          /* jmp index for a path */
```

```
static          nc[2];          /* number at start of current line */
static          ni[2];          /* current elem number -- for gapping */
static          siz[2];
static char     *ps[2];         /* ptr to current element */
static char     *po[2];         /* ptr to next output char slot */
static char     out[2][P_LINE];         /* output line */
static char     star[P_LINE];   /* set by stars() */


/*
 * print alignment of described in struct path pp[]
 */
static
pr_align()                                                              pr_align
{
        int             nn;     /* char count */
        int             more;
        register        i;


        for (i = 0, lmax = 0; i < 2; i++) {
                nn = stripname(namex[i]);
                if (nn > lmax)
                        lmax = nn;

                nc[i] = 1;
                ni[i] = 1;
                siz[i] = ij[i] = 0;
                ps[i] = seqx[i];
                po[i] = out[i];                 }


        for (nn = nm = 0, more = 1; more; ) {                            ...pr_align
                for (i = more = 0; i < 2; i++) {
                        /*
                         * do we have more of this sequence?
                         */
                        if (!*ps[i])
```

```
                continue;

                more++;

                if (pp[i].spc) {   /* leading space */
                        *po[i]++ = ' ';
                        pp[i].spc--;
                }
                else if (siz[i]) { /* in a gap */
                        *po[i]++ = '-';
                        siz[i]--;
                }
                else {              /* we're putting a seq element
                                    */
                        *po[i] = *ps[i];
                        if (islower(*ps[i]))
                                *ps[i] = toupper(*ps[i]);
                        po[i]++;
                        ps[i]++;

                        /*
                         * are we at next gap for this seq?
                         */
                        if (ni[i] == pp[i].x[ij[i]]) {
                                /*
                                 * we need to merge all gaps
                                 * at this location
                                 */
                                siz[i] = pp[i].n[ij[i]++];
                                while (ni[i] == pp[i].x[ij[i]])
                                        siz[i] += pp[i].n[ij[i]++];
                        }
                        ni[i]++;
                }
        }
        if (++nn == olen || !more && nn) {
```

```
                dumpblock();
                for (i = 0; i < 2; i++)
                        po[i] = out[i];
                nn = 0;
            }
        }
    }


/*
 * dump a block of lines, including numbers, stars: pr_align()
 */
static
dumpblock()                                                        dumpblock
{
        register        i;


        for (i = 0; i < 2; i++)
                *po[i]-- = '\0';



                                                                ...dumpblock
        (void) putc('\n', fx);
        for (i = 0; i < 2; i++) {
                if (*out[i] && (*out[i] != ' ' || *(po[i]) != ' ')) {
                        if (i == 0)
                                nums(i);
                        if (i == 0 && *out[1])
                                stars();
                        putline(i);
                        if (i == 0 && *out[1])
                                fprintf(fx, star);
                        if (i == 1)
                                nums(i);
                }
        }
}
```

28

```
/*
 * put out a number line: dumpblock()
 */
static
nums(ix)                                                              nums
        int     ix;       /* index in out[] holding seq line */
{
        char            nline[P_LINE];
        register                i, j;
        register char   *pn, *px, *py;

        for (pn = nline, i = 0; i < lmax+P_SPC; i++, pn++)
                *pn = ' ';
        for (i = nc[ix], py = out[ix]; *py; py++, pn++) {
                if (*py == ' ' || *py == '-')
                        *pn = ' ';
                else {
                        if (i%10 == 0 || (i == 1 && nc[ix] != 1)) {
                                j = (i < 0)? -i : i;
                                for (px = pn; j; j /= 10, px--)
                                        *px = j%10 + '0';
                                if (i < 0)
                                        *px = '-';
                        }
                        else
                                *pn = ' ';
                        i++;
                }
        }
        *pn = '\0';
        nc[ix] = i;
        for (pn = nline; *pn; pn++)
                (void) putc(*pn, fx);
        (void) putc('\n', fx);
}
```

29

```
/*
 * put out a line (name, [num], seq, [num]): dumpblock()
 */
static
putline(ix)                                                         putline
        int     ix;                     {
```

...putline

```
        int             i;
        register char   *px;

        for (px = namex[ix], i = 0; *px && *px != ':'; px++, i++)
                (void) putc(*px, fx);
        for (; i < lmax+P_SPC; i++)
                (void) putc(' ', fx);

        /* these count from 1:
         * ni[] is current element (from 1)
         * nc[] is number at start of current line
         */
        for (px = out[ix]; *px; px++)
                (void) putc(*px&0x7F, fx);
        (void) putc('\n', fx);
}


/*
 * put a line of stars (seqs always in out[0], out[1]): dumpblock()
 */
static
stars()                                                             stars
{
        int             i;
        register char   *p0, *p1, cx, *px;
```

```
if (!*out[0] || (*out[0] == ' ' && *(po[0]) == ' ') ||
    !*out[1] || (*out[1] == ' ' && *(po[1]) == ' '))
        return;
px = star;
for (i = lmax+P_SPC; i; i--)
        *px++ = ' ';


for (p0 = out[0], p1 = out[1]; *p0 && *p1; p0++, p1++) {
        if (isalpha(*p0) && isalpha(*p1)) {

                if (xbm[*p0-'A']&xbm[*p1-'A']) {
                        cx = '*';
                        nm++;
                }
                else if (!dna && _day[*p0-'A'][*p1-'A'] > 0)
                        cx = '.';
                else
                        cx = ' ';
        }
        else
                cx = ' ';
        *px++ = cx;
}
*px++ = '\n';
*px = '\0';
}




/*
 * strip path or prefix from pn, return len: pr_align()
 */
```

```
static
stripname(pn)                                                           stripname
        char    *pn;    /* file name (may be path) */
{
        register char    *px, *py;


        py = 0;
        for (px = pn; *px; px++)
                if (*px == '/')
                        py = px + 1;
        if (py)
                (void) strcpy(pn, py);
        return(strlen(pn));


}



/*
 * cleanup() -- cleanup any tmp file
 * getseq() -- read in seq, set dna, len, maxlen
 * g_calloc() -- calloc() with error checkin
 * readjmps() -- get the good jmps, from tmp file if necessary
 * writejmps() -- write a filled array of jmps to a tmp file: nw()
 */
#include "nw.h"
#include <sys/file.h>


char    *jname = "/tmp/homgXXXXXX";                /* tmp file for jmps */
FILE    *fj;


int     cleanup();                                 /* cleanup tmp file */
long    lseek();


/*
 * remove any tmp file if we blow
 */
```

```
cleanup(i)                                                      cleanup
        int     i;
{
        if (fj)
                (void) unlink(jname);
        exit(i);
}


/*
 * read, return ptr to seq, set dna, len, maxlen
 * skip lines starting with ';', '<', or '>'
 * seq in upper or lower case
 */
char    *
getseq(file, len)                                               getseq
        char    *file;   /* file name */
        int     *len;    /* seq len */
{
        char            line[1024], *pseq;
        register char   *px, *py;
        int             natgc, tlen;
        FILE            *fp;

        if ((fp = fopen(file,"r")) == 0) {
                fprintf(stderr,"%s: can't read %s\n", prog, file);
                exit(1);
        }
        tlen = natgc = 0;
        while (fgets(line, 1024, fp)) {
                if (*line == ';' || *line == '<' || *line == '>')
                        continue;
                for (px = line; *px != '\n'; px++)
                        if (isupper(*px) || islower(*px))
                                tlen++;
        }
        if ((pseq = malloc((unsigned)(tlen+6))) == 0 {
```

EP 2 367 008 A2

```
            fprintf(stderr,"%s: malloc() failed to get %d bytes for %s\n", prog, tlen+6, file);
            exit(1);
    }
    pseq[0] = pseq[1] = pseq[2] = pseq[3] = '\0';




                                                                          ...getseq

    py = pseq + 4;
    *len = tlen;
    rewind(fp);


    while (fgets(line, 1024, fp)) {
        if (*line == ';' || *line == '<' || *line == '>')
            continue;
        for (px = line; *px != '\n'; px++) {
            if (isupper(*px))
                *py++ = *px;
            else if (islower(*px))
                *py++ = toupper(*px);
            if (index("ATGCU",*(py-1)))
                natgc++;
        }
    }
    *py++ = '\0';
    *py = '\0';
    (void) fclose(fp);
    dna = natgc > (tlen/3);
    return(pseq+4);
}


char    *
g_calloc(msg, nx, sz)                                                    g_calloc
        char    *msg;           /* program, calling routine */
        int     nx, sz;         /* number and size of elements */

        {
```

```
        char            *px, *calloc();

        if ((px = calloc((unsigned)nx, (unsigned)sz)) == 0) {
                if (*msg) {
                        fprintf(stderr, "%s: g_calloc() failed %s (n=%d, sz=%d)\n", prog, msg,
nx, sz);
                        exit(1);
                }
        }
        return(px);
}


/*
 * get final jmps from dx[] or tmp file, set pp[], reset dmax: main()
 */
readjmps()                                                                  readjmps
{
        int             fd = -1;
        int             siz, i0, i1;
        register        i, j, xx;

        if (fj) {
                (void) fclose(fj);
                if ((fd = open(jname, O_RDONLY, 0)) < 0) {
                        fprintf(stderr, "%s: can't open() %s\n", prog, jname);
                        cleanup(1);
                }
        }
        for (i = i0 = i1 = 0, dmax0 = dmax, xx = len0; ; i++) {
                while (1) {
                        for (j = dx[dmax].ijmp; j >= 0 && dx[dmax].jp.x[j] >= xx; j--)
                                ;

                                                                         ...readjmps
                        if (j < 0 && dx[dmax].offset && fj) {
                                (void) lseek(fd, dx[dmax].offset, 0);
```

```
                                (void) read(fd, (char *)&dx[dmax].jp, sizeof(struct jmp));
                                (void) read(fd, (char *)&dx[dmax].offset,
       sizeof(dx[dmax].offset));
                                dx[dmax].ijmp = MAXJMP-1;
                        }
                        else
                                break;
                }
                if (i >= JMPS) {
                        fprintf(stderr, "%s: too many gaps in alignment\n", prog);
                        cleanup(1);
                }
                if (j >= 0) {
                        siz = dx[dmax].jp.n[j];
                        xx = dx[dmax].jp.x[j];
                        dmax += siz;
                        if (siz < 0) {              /* gap in second seq */
                                pp[1].n[i1] = -siz;
                                xx += siz;
                                /* id = xx - yy + len1 - 1
                                 */
                                pp[1].x[i1] = xx - dmax + len1 - 1;
                                gapy++;
                                ngapy -= siz;
/* ignore MAXGAP when doing endgaps */
                                siz = (-siz < MAXGAP || endgaps)? -siz : MAXGAP;
                                i1++;
                        }
                        else if (siz > 0) {        /* gap in first seq */
                                pp[0].n[i0] = siz;
                                pp[0].x[i0] = xx;
                                gapx++;
                                ngapx += siz;
/* ignore MAXGAP when doing endgaps */
                                siz = (siz < MAXGAP || endgaps)? siz : MAXGAP;
                                i0++;
```

```
                    }
            }
        else
            break;
    }


    /* reverse the order of jmps
    */
    for (j = 0, i0--; j < i0; j++, i0--) {
            i = pp[0].n[j]; pp[0].n[j] = pp[0].n[i0]; pp[0].n[i0] = i;
            i = pp[0].x[j]; pp[0].x[j] = pp[0].x[i0]; pp[0].x[i0] = i;
    }
    for (j = 0, i1--; j < i1; j++, i1--) {
            i = pp[1].n[j]; pp[1].n[j] = pp[1].n[i1]; pp[1].n[i1] = i;
            i = pp[1].x[j]; pp[1].x[j] = pp[1].x[i1]; pp[1].x[i1] = i;
    }
    if (fd >= 0)
            (void) close(fd);
    if (fj) {
            (void) unlink(jname);
            fj = 0;
            offset = 0;
    }                                    }



/*
 * write a filled jmp struct offset of the prev one (if any): nw()
 */
writejmps(ix)                                                                        writejmps
        int     ix;
{
        char    *mktemp();

        if (!fj) {
                if (mktemp(jname) < 0) {
                        fprintf(stderr, "%s: can't mktemp() %s\n", prog, jname);
```

```
                cleanup(1);
        }
        if ((fj = fopen(jname, "w")) == 0) {
                fprintf(stderr, "%s: can't write %s\n", prog, jname);
                exit(1);
        }
}
(void) fwrite((char *)&dx[ix].jp, sizeof(struct jmp), 1, fj);
(void) fwrite((char *)&dx[ix].offset, sizeof(dx[ix].offset), 1, fj);
```

The term "vector," as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "recombinant vectors"). In general, expression vectors of utility in recombinant DNA techniques are often in the form of plasmids. In the present specification, "plasmid" and "vector" may be used interchangeably as the plasmid is the most commonly used form of vector.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, .alpha.-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), "(O)NR.sub.2 ("amidate"), P(O)R, P(O)OR', CO or CH.sub.2 ("formacetal"), in which each R or R' is independently H or substituted or unsubstituted alkyl (1-20 C.) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

The term "hepatocyte growth factor" or "HGF", as used herein, refers, unless specifically or contextually indicated otherwise, to any native or variant (whether native or synthetic) HGF polypeptide that is capable of activating the HGF/c-met signaling pathway under conditions that permit such process to occur. The term "wild type HGF" generally refers to a polypeptide comprising the amino acid sequence of a naturally occurring HGF protein. Thet term "wild type HGF sequence" generally refers to an amino acid sequence found in a naturally occurring HGF.

"Activated HGF β chain", or variations thereof, refers to any HGF β chain having the conformation that is adopted by wild type HGF β chain upon conversion of wild type HGF protein from a single chain form to a 2 chain form (i.e., α and β chain), said conversion resulting at least in part from cleavage between residue 494 and residue 495 of the wild type HGF protein. In some embodiments, said conformation refers specifically to the conformation of the activation domain of the protease-like domain in the chain. In some embodiments, said conformation refers even more specifically to the conformation of the active site region of the protease-like domain in the β chain. Generally, adoption of said conformation reveals a c-met binding site, as described herein.

The terms "antibody" and "immunoglobulin" are used interchangeably in the broadest sense and include monoclonal antibodies (for *e.g.*, full length or intact monoclonal antibodies), polyclonal antibodies, multivalent antibodies, multispecific antibodies (*e.g.*, bispecific antibodies so long as they exhibit the desired biological activity) and may also include certain antibody fragments (as described in greater detail herein). An antibody can be human, humanized and/or affinity matured.

"Antibody fragments" comprise only a portion of an intact antibody, wherein the portion preferably retains at least one, preferably most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an in vivo half life substantially similar to an intact antibody. For e.g., such an antibody fragment may comprise on antigen binding arm linked to an Fc sequence capable of conferring in vivo stability to the fragment.

The term "monoclonal antibody" as used herein refers to an antibody obtained from a population of substantially homogeneous antibodies, *i.e.*, the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (*e.g.,* murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues.

An "affinity matured" antibody is one with one or more alterations in one or more CDRs thereof which result in an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

A "blocking" antibody or an "antagonist" antibody is one which inhibits or reduces biological activity of the antigen it binds (for e.g., activated HGF β chain or site/epitope on c-met to which activated HGF β binds). Preferred blocking antibodies or antagonist antibodies substantially or completely inhibit the biological activity of the antigen.

An "agonist antibody", as used herein, is an antibody which mimics at least one of the functional activities of a polypeptide of interest (for e.g., an antibody could provide at least one of the c-met activating functions of activated HGF β chain).

A "disorder" is any condition that would benefit from treatment with a substance/molecule or method of the invention. This includes chronic and acute disorders or diseases including those pathological conditions which predispose the mammal to the disorder in question. Non-limiting examples of disorders to be treated herein include malignant and benign tumors; non-leukemias and lymphoid malignancies; neuronal, glial, astrocytal, hypothalamic and other glandular, macrophagal, epithelial, stromal and blastocoelic disorders; and inflammatory, immunologic and other angiogenesis-related disorders.

The terms "cell proliferative disorder" and "proliferative disorder" refer to disorders that are associated with some degree of abnormal cell proliferation. In one embodiment, the cell proliferative disorder is cancer.

"Tumor", as used herein, refers to all neoplastic cell growth and proliferation, whether malignant or benign, and all pre-cancerous and cancerous cells and tissues. The terms "cancer", "cancerous", "cell proliferative disorder", "proliferative disorder" and "tumor" are not mutually exclusive as referred to herein.

The terms "cancer" and "cancerous" refer to or describe the physiological condition in mammals that is typically characterized by unregulated cell growth/proliferation. Examples of cancer include but are not limited to, carcinoma, lymphoma, blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma and various types of head and neck cancer.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or disorder.

An "effective amount" refers to an amount effective, at dosages and for periods of time necessary, to achieve the desired therapeutic or prophylactic result.

A "therapeutically effective amount" of a substance/molecule of the invention, agonist or antagonist may vary according to factors such as the disease state, age, sex, and weight of the individual, and the ability of the substance/molecule, agonist or antagonist to elicit a desired response in the individual. A therapeutically effective amount is also one in which any toxic or detrimental effects of the substance/molecule, agonist or antagonist are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The term "cytotoxic agent" as used herein refers to a substance that inhibits or prevents the function of cells and/or causes destruction of cells. The term is intended to include radioactive isotopes (e.g., $At^{211}$, $I^{131}$, $I^{125}$, $Y^{90}$, $R^{186}$, $R^{188}$, $Sm^{153}$, $Bi^{212}$, $P^{32}$ and radioactive isotopes of Lu), chemotherapeutic agents e.g. methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma11 and calicheamicin omegal1 (see, e.g., Agnew, Chem Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® paclitaxel (Bristol- Myers Squibb Oncology, Princeton, N.J.), ABRAXANE™ Cremophor-free, albumin-engineered nanoparticle formulation of paclitaxel (American Pharmaceutical Partners, Schaumberg, Illinois), and TAXOTERE® doxetaxel (Rhône- Poulenc Rorer, Antony, France); chloranbucil; GEMZAR® gemcitabine; 6- thioguanine; mercaptopurine; methotrexate; platinum analogs such as cisplatin and carboplatin; vinblastine; platinum; etoposide (VP-16); ifosfamide; mitoxantrone; vincristine; NAVELBINE® vinorelbine; novantrone; teniposide; edatrexate; daunomycin; aminopterin; xeloda; ibandronate; CPT-11; topoisomerase inhibitor RFS 2000; difluorometlhylornithine (DMFO); retinoids such as retinoic acid; capecitabine; and pharmaceutically acceptable salts, acids or derivatives of any of the above.

Also included in the definition of "chemotherapeutic agent" above are anti-hormonal agents that act to regulate or inhibit hormone action on tumors such as anti-estrogens and selective estrogen receptor modulators (SERMs), including, for example, tamoxifen (including NOLVADEX® tamoxifen), raloxifene, droloxifene, 4-hydroxytamoxifen, trioxifene, keoxifene, LY117018, onapristone, and FARESTON·toremifene; aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole; and anti-androgens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in abherant cell proliferation, such as, for example, PKC-alpha, Ralf and H-Ras; ribozymes such as a VEGF expression inhibitor (e.g., ANGIOZYME® ribozyme) and a HER2 expression inhibitor; vaccines such as gene therapy vaccines, for example, ALLOVECTIN® vaccine, LEUVECTIN® vaccine, and VAXID® vaccine; PROLEUKIN® rIL-2; LURTOTECAN® topoisomerase 1 inhibitor; ABARELIX® rmRH; and pharmaceutically acceptable salts, acids or derivatives of any of the above.

A "growth inhibitory agent" when used herein refers to a compound or composition which inhibits growth of a cell whose growth is dependent upon HGF/c-met activation either *in vitro* or *in vivo*. Thus, the growth inhibitory agent may be one which significantly reduces the percentage of HGF/c-met-dependent cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

"Doxorubicin" is an anthracycline antibiotic. The full chemical name of doxorubicin is (8S-cis)-10-[(3-amino-2,3,6-trideoxy-$\alpha$-L-lyxo-hexapyranosyl)oxy]-7,8,9,10-tetrahydro-6,8,11-trihydroxy-8-(hydroxyacetyl)-1-methoxy-5,12-naphthacenedione.

<u>Vector Construction</u>

Polynucleotide sequences encoding the polypeptides described herein can be obtained using standard recombinant techniques. Desired polynucleotide sequences may be isolated and sequenced from appropriate source cells. Source cells for antibodies would include antibody producing cells such as hybridoma cells. Alternatively, polynucleotides can be synthesized using nucleotide synthesizer or PCR techniques. Once obtained, sequences encoding the immunoglobulins are inserted into a recombinant vector capable of replicating and expressing heterologous polynucleotides in a host cell. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication (in particular when the vector is inserted into a prokaryotic cell), a selection marker gene, a promoter, a ribosome binding site (RBS), a signal sequence, the heterologous nucleic acid insert and a transcription termination sequence.

In general, plasmid vectors containing replicon and control sequences which are derived from a species compatible with the host cell are used in connection with these hosts. The vector ordinarily carries a replication site, as well as marking sequences which are capable of providing phenotypic selection in transformed cells. For example, *E. coli* is typically transformed using pBR322, a plasmid derived from an *E. coli* species. pBR322 contains genes encoding ampicillin (Amp) and tetracycline (Tet) resistance and thus provides easy means for identifying transformed cells. pBR322, its derivatives, or other microbial plasmids or bacteriophage may also contain, or be modified to contain, promoters which can be used by the microbial organism for expression of endogenous proteins.

In addition, phage vectors containing replicon and control sequences that are compatible with the host microorganism can be used as transforming vectors in connection with these hosts. For example, bacteriophage such as $\lambda$GEM.TM.-11 may be utilized in making a recombinant vector which can be used to transform susceptible host cells such as *E. coli* LE392.

Either constitutive or inducible promoters can be used in the present invention, in accordance with the needs of a particular situation, which can be ascertained by one skilled in the art. A large number of promoters recognized by a variety of potential host cells are well known. The selected promoter can be operably linked to cistron DNA encoding a polypeptide described herein by removing the promoter from the source DNA via restriction enzyme digestion and inserting the isolated promoter sequence into the vector of choice. Both the native promoter sequence and many heterologous promoters may be used to direct amplification and/or expression of the target genes. However, heterologous promoters are preferred, as they generally permit greater transcription and higher yields of expressed target gene as compared to the native target polypeptide promoter.

Promoters suitable for use with prokaryotic hosts include the PhoA promoter, the $\beta$-galactamase and lactose promoter systems, a tryptophan (trp) promoter system and hybrid promoters such as the *tac* or the *trc* promoter. However, other promoters that are functional in bacteria (such as other known bacterial or phage promoters) are suitable as well. Their nucleotide sequences have been published, thereby enabling a skilled worker operably to ligate them to cistrons encoding the target light and heavy chains (Siebenlist et al. (1980) Cell 20: 269) using linkers or adaptors to supply any required restriction sites.

In some embodiments, each cistron within a recombinant vector comprises a secretion signal sequence component that directs translocation of the expressed polypeptides across a membrane. In general, the signal sequence may be a component of the vector, or it may be a part of the target polypeptide DNA that is inserted into the vector. The signal sequence selected for the purpose of this invention should be one that is recognized and processed (i.e. cleaved by a signal peptidase) by the host cell. For prokaryotic host cells that do not recognize and process the signal sequences native to the heterologous polypeptides, the signal sequence is substituted by a prokaryotic signal sequence selected, for example, from the group consisting of the alkaline phosphatase, penicillinase, lpp, or heat-stable enterotoxin II (STII) leaders, LamB, PhoE, PelB, OmpA and MBP.

Prokaryotic host cells suitable for expressing polypeptides include Archaebacteria and Eubacteria, such as Gram-negative or Gram-positive organisms. Examples of useful bacteria include Escherichia (e.g., *E. coli*), Bacilli (e.g., B. subtilis), Enterobacteria, Pseudomonas species (e.g., P. aeruginosa), Salmonella typhimurium, Serratia marcescans, Klebsiella, Proteus, Shigella, Rhizobia, Vitreoscilla, or Paracoccus. Preferably, gram-negative cells are used. Preferably the host cell should secrete minimal amounts of proteolytic enzymes, and additional protease inhibitors may desirably be incorporated in the cell culture.

Polypeptide Production

Host cells are transformed or transfected with the above-described expression vectors and cultured in conventional nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, $CaPO_4$ precipitation and electroporation. Successful transfection is generally recognized when any indication of the operation of this vector occurs within the host cell.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cell-wall barriers. Another method for transformation employs polyethylene glycol/ DMSO. Yet another technique used is electroporation.

Prokaryotic cells used to produce the polypeptides of the invention are grown in media known in the art and suitable for culture of the selected host cells. Examples of suitable media include luria broth (LB) plus necessary nutrient supplements. In preferred embodiments, the media also contains a selection agent, chosen based on the construction of the expression vector, to selectively permit growth of prokaryotic cells containing the expression vector. For example, ampicillin is added to media for growth of cells expressing ampicillin resistant gene.

Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithioerythritol and dithiothreitol.

The prokaryotic host cells are cultured at suitable temperatures. For *E. coli* growth, for example, the preferred temperature ranges from about 20°C to about 39°C, more preferably from about 25°C to about 37°C, even more preferably at about 30°C. The pH of the medium may be any pH ranging from about 5 to about 9, depending mainly on the host organism. For *E. coli,* the pH is preferably from about 6.8 to about 7.4, and more preferably about 7.0.

If an inducible promoter is used in the expression vector, protein expression is induced under conditions suitable for the activation of the promoter. For example, if a PhoA promoter is used for controlling transcription, the transformed host cells may be cultured in a phosphate-limiting medium for induction. A variety of other inducers may be used, according to the vector construct employed, as is known in the art.

Polypeptides described herein expressed in a microorganism may be secreted into and recovered from the periplasm of the host cells. Protein recovery typically involves disrupting the microorganism, generally by such means as osmotic shock, sonication or lysis. Once cells are disrupted, cell debris or whole cells may be removed by centrifugation or filtration. The proteins may be further purified, for example, by affinity resin chromatography. Alternatively, proteins can be transported into the culture media and isolated therefrom. Cells may be removed from the culture and the culture supernatant being filtered and concentrated for further purification of the proteins produced. The expressed polypeptides can be further isolated and identified using commonly known methods such as fractionation on immunoaffinity or ion-exchange columns; ethanol precipitation; reverse phase HPLC; chromatography on silica or on a cation exchange resin such as DEAE; chromatofocusing; SDS-PAGE; ammonium sulfate precipitation; gel filtration using, for example, Sephadex G-75; hydrophobic affinity resins, ligand affinity using a suitable antigen immobilized on a matrix and Western blot assay.

Besides prokaryotic host cells, eukaryotic host cell systems are also well established in the art. Suitable hosts include mammalian cell lines such as CHO, and insect cells such as those described below.

Polypeptide Purification

Polypeptides that are produced may be purified to obtain preparations that are substantially homogeneous for further assays and uses. Standard protein purification methods known in the art can be employed. The following procedures are exemplary of suitable purification procedures: fractionation on immunoaffinity or ion-exchange columns, ethanol precipitation, reverse phase HPLC, chromatography on silica or on a cation-exchange resin such as DEAE,

chromatofocusing, SDS-PAGE, ammonium sulfate precipitation, and gel filtration using, for example, Sephadex G-75.

Methods Of The Invention

The invention provides various methods based on the finding that activated HGF β is capable of directly binding to c-met, and that such binding can be inhibited with the appropriate substance or molecule.

Various substances or molecules (including peptides, etc.) may be employed as therapeutic agents. These substances or molecules can be formulated according to known methods to prepare pharmaceutically useful compositions, whereby the product hereof is combined in admixture with a pharmaceutically acceptable carrier vehicle. Therapeutic formulations are prepared for storage by mixing the active ingredient having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients or stabilizers are nontoxic to recipients at the dosages and concentrations employed, and include buffers such as phosphate, citrate and other organic acids; antioxidants including ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagine, arginine or lysine; monosaccharides, disaccharides and other carbohydrates including glucose, mannose, or dextrins; chelating agents such as EDTA; sugar alcohols such as mannitol or sorbitol; salt-forming counterions such as sodium; and/or nonionic surfactants such as TWEEN™, PLURONICS™ or PEG.

The formulations to be used for *in vivo* administration must be sterile. This is readily accomplished by filtration through sterile filtration membranes, prior to or following lyophilization and reconstitution.

Therapeutic compositions herein generally are placed into a container having a sterile access port, for example, an intravenous solution bag or vial having a stopper pierceable by a hypodermic injection needle.

The route of administration is in accord with known methods, e.g. injection or infusion by intravenous, intraperitoneal, intracerebral, intramuscular, intraocular, intraarterial or intralesional routes, topical administration, or by sustained release systems.

Dosages and desired drug concentrations of pharmaceutical compositions of the present invention may vary depending on the particular use envisioned. The determination of the appropriate dosage or route of administration is well within the skill of an ordinary physician. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" In Toxicokinetics and New Drug Development, Yacobi et al., Eds., Pergamon Press, New York 1989, pp. 42-96.

When *in vivo* administration of a substance or molecule of the invention is employed, normal dosage amounts may vary from about 10 ng/kg to up to 100 mg/kg of mammal body weight or more per day, preferably about 1 μg/kg/day to 10 mg/kg/day, depending upon the route of administration. Guidance as to particular dosages and methods of delivery is provided in the literature; see, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

Where sustained-release administration of a substance or molecule is desired in a formulation with release characteristics suitable for the treatment of any disease or disorder requiring administration of the substance or molecule, microencapsulation of the substance or molecule is contemplated. Microencapsulation of recombinant proteins for sustained release has been successfully performed with human growth hormone (rhGH), interferon- (rhIFN-), interleukin-2, and MN rgp120. Johnson et al., Nat. Med., 2:795-799 (1996); Yasuda, Biomed. Ther., 27:1221-1223 (1993); Hora et al., Bio/Technology, 8:755-758 (1990); Cleland, "Design and Production of Single Immunization Vaccines Using Polylactide Polyglycolide Microsphere Systems," in Vaccine Design: The Subunit and Adjuvant Approach, Powell and Newman, eds, (Plenum Press: New York, 1995), pp. 439-462; WO 97/03692, WO 96/40072, WO 96/07399; and U.S. Pat. No. 5,654,010.

The sustained-release formulations of these proteins were developed using poly-lactic-coglycolic acid (PLGA) polymer due to its biocompatibility and wide range of biodegradable properties. The degradation products of PLGA, lactic and glycolic acids, can be cleared quickly within the human body. Moreover, the degradability of this polymer can be adjusted from months to years depending on its molecular weight and composition. Lewis, "Controlled release of bioactive agents from lactide/glycolide polymer," in: M. Chasin and R. Langer (Eds.), Biodegradable Polymers as Drug Delivery Systems (Marcel Dekker: New York, 1990), pp. 1-41.

This invention encompasses methods of screening compounds to identify those that inhibit HGF/c-met signaling through interfering with HGF β chain and c-met interaction. Screening assays are designed to identify compounds that bind or complex with activated (and preferably not zymogen-like) HGF β chain and/or c-met (at a site on c-met that inhibits binding of activated HGF β chain to c-met), or otherwise interfere with the interaction of activated HGF β chain with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

All assays for antagonists are common in that they call for contacting the drug candidate with a site on HGF β chain (or equivalent thereof) and/or c-met that is involved in the binding interaction of activated HGF β chain and c-met, under conditions and for a time sufficient to allow these two components to interact.

In binding assays, the interaction is binding and the complex formed can be isolated or detected in the reaction mixture. In a particular embodiment, a candidate substance or molecule is immobilized on a solid phase, e.g., on a microtiter plate, by covalent or non-covalent attachments. Non-covalent attachment generally is accomplished by coating the solid surface with a solution of the substance/molecule and drying. Alternatively, an immobilized affinity molecule, such as an antibody, e.g., a monoclonal antibody, specific for the substance/molecule to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to an activated HGF β chain or c-met, its interaction with the polypeptide can be assayed by methods well known for detecting protein-protein interactions. Such assays include traditional approaches, such as, e.g., crosslinking, co-immunoprecipitation, and co-purification through gradients or chromatographic columns. In addition, protein-protein interactions can be monitored by using a yeast-based genetic system described by Fields and co-workers (Fields and Song, Nature (London), 340:245-246 (1989); Chien et al., Proc. Natl. Acad. Sci. USA, 88:9578-9582 (1991)) as disclosed by Chevray and Nathans, Proc. Natl. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of activated HGF β chain and c-met can be tested as follows: usually a reaction mixture is prepared containing the activated HGF β chain and c-met (or equivalent thereof that comprises the cognate activated HGF β chain binding site on c-met) under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and activated HGF β chain and/or c-met (or equivalent thereof as described above) present in the mixture is monitored as described hereinabove. The formation of a complex in the control reaction(s) but not in the reaction mixture containing the test compound indicates that the test compound interferes with the interaction of activated HGF β chain and c-met.

To assay for inhibitors (such as antagonists), 2-chain HGF comprising activated HGF β chain may be added to a cell along with the compound to be screened for a particular activity and the ability of the compound to inhibit the activity of interest in the presence of the 2-chain HGF suggests that the compound could be an antagonist to the activated HGF β chain, a property that could be further confirmed by determining its ability to bind or interact specifically with activated HGF β chain and not with HGF α chain (for e.g., as found in 2-chain HGF or single chain HGF).

More specific examples of potential antagonists include an oligonucleotide (which may be an aptamer) that binds to the activated HGF β chain and/or its binding site on c-met, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of HGF β chain that recognizes a HGF β chain binding partner but imparts no effect, thereby competitively inhibiting the action of wild type HGF β chain.

Potential antagonists include small molecules that bind to the active site of HGF β chain, the binding site of activated HGF β chain on c-met, or other relevant binding site of activated HGF β chain, thereby blocking the normal biological activity of the activated HGF β chain. Examples of small molecules include, but are not limited to, small peptides or peptide-like molecules, preferably soluble peptides, and synthetic non-peptidyl organic or inorganic compounds.

These small molecules can be identified by any one or more of the screening assays discussed hereinabove and/or by any other screening techniques well known for those skilled in the art.

As described herein, a substance/molecule of the invention can be a peptide. Methods of obtaining such peptides are well known in the art, and include screening peptide libraries for binders to a suitable target antigen. In one embodiment, suitable target antigens would comprise activated HGF β chain (or portion thereof that comprises binding site for c-met), which is described in detail herein. For e.g., a suitable target antigen is an activated HGF β chain polypeptide as described herein, or a 2-chain HGF polypeptide (which, as described herein, comprises an activated HGF β chain component). In some instances, in particular where a desired substance/molecule is one that binds to any significant degree activated HGF β chain but not HGF α chain and/or zymogen-form HGF β chain, a candidate binder can also be screened for lack of substantial binding capability with respect to a polypeptide comprising HGF β chain in zymogen form (i.e., unactivated HGF β chain) (for e.g., single chain HGF). Libraries of peptides are well known in the art, and can also be prepared according to art methods. See, for e.g., Clark et al., U.S. Pat. No. 6,121,416. Libraries of peptides fused to a heterologous protein component, such as a phage coat protein, are well known in the art, for e.g., as described in Clark et al., *supra.* In one embodiment, a peptide having ability to block binding of activated HGF β chain to c-met comprises the amino acid sequence VDWVCFRDLGCDWEL, or variants thereof. Variants of a first peptide binder can be generated by screening mutants of the peptide to obtain the characteristics of interest (e.g., enhancing target binding affinity, enhanced pharmacokinetics, reduced toxicity, improved therapeutic index, etc.). Mutagenesis techniques are well known in the art. Furthermore, scanning mutagenesis techniques (such as those based on alanine scanning) can be especially helpful to assess structural and/or functional importance of individual amino acid residues within a peptide.

Determination of the ability of a candidate substance/molecule of the invention, such as a peptide comprising the amino acid sequence VDWVCFRDLGCDWEL or variant thereof, to modulate HGF/c-met signaling and/or biological activities associated with said signaling, can be performed by testing the modulatory capability of the substance/molecule in *in vitro* or *in vivo* assays, which are well established in the art, for e.g., as described in Okigaki et al., *supra;* Matsumoto et al., *supra*; Date et al., FEBS Let. (1997), 420:1-6; Lokker et al., *supra*; Hartmann et al., *supra.*

Anti-activated HGF β chain Antibodies

The present invention further provides methods comprising use of anti-activated HGF β chain antibodies. Exemplary antibodies include polyclonal, monoclonal, humanized, bispecific, and heteroconjugate antibodies.

1. Polyclonal Antibodies

The anti-activated HGF chain antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include an activated HGF β chain (or portion thereof) or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

2. Monoclonal Antibodies

The anti-activated HGF β chain antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the activated HGF β chain (or portion thereof) or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against activated HGF β chain. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

Antibodies can also be generated by screening phage display libraries for antibodies or antibody fragments that bind with suitable/desired affinity to activated HGF β chain (or equivalent). Such techniques are well known in the art, for e.g., as disclosed in U.S Pat. Nos. 5,750,373; 5,780,279; 5,821,047;6,040,136; 5,427,908; 5,580,717, and references therein.

3. Human and Humanized Antibodies

The anti-activated HGF β chain antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')$_2$ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321: 522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Biotechnology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

## 4. Bispecific Antibodies

Bispecific antibodies are monoclonal, preferably human or humanized, antibodies that have binding specificities for at least two different antigens. In the present case, one of the binding specificities is for activated HGF β chain and/or HGF β chain binding site of c-met, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. $F(ab')_2$ bispecific antibodies). Techniques for generating bispecific antibodies from antibody fragments have been described in the literature. For example, bispecific antibodies can be prepared can be prepared using chemical linkage. Brennan et al., Science 229:81 (1985) describe a procedure wherein intact antibodies are proteolytically cleaved to generate $F(ab')_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby et al., J. Exp. Med. 175:217-225 (1992) describe the production of a fully humanized bispecific antibody $F(ab')_2$ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90: 6444-6448 (1993) has provided an alternative mechanism for making bispecific antibody fragments. The fragments comprise a heavy-chain variable domain ($V_H$) connected to a light-chain variable domain ($V_L$) by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994).

Antibodies with more than two valencies are contemplated. For example, trispecific antibodies can be prepared. Tutt et al., J. Immunol. 147:60 (1991).

Exemplary bispecific antibodies may bind to two different epitopes on activated HGF β chain or to an epitope on activated HGF β chain and an epitope on another polypeptide (for e.g., c-met or HGF α chain).

5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

6. Effector Function Engineering

It may be desirable to modify the antibody of the invention with respect to effector function, so as to enhance, *e.g.*, the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced antitumor activity may also be prepared using heterobifunctional cross-linkers as described in Wolff et al. Cancer Research, 53: 2560-2565 (1993). Alternatively, an antibody can be engineered that has dual Fc regions and may thereby have enhanced complement lysis and ADCC capabilities. See Stevenson et al., Anti-Cancer Drug Design, 3: 219-230 (1989).

7. Immunoconjugates

The invention also pertains to immunoconjugates comprising an antibody conjugated to a cytotoxic agent such as a chemotherapeutic agent, toxin (*e.g.*, an enzymatically active toxin of bacterial, fungal, plant, or animal origin, or fragments thereof), or a radioactive isotope (*i.e.*, a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include $^{212}Bi$, $^{131}I$, $^{131}In$, $^{90}Y$, and $^{186}Re$. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987), Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.*, avidin) that is conjugated to a cytotoxic agent (*e.g.*, a radionucleotide).

8. Immunoliposomes

The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al ., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

9. Pharmaceutical Compositions of Antibodies

Antibodies as well as other molecules identified by the screening assays disclosed hereinbefore, can be administered for the treatment of various disorders in the form of pharmaceutical compositions.

If whole antibodies are used as inhibitors, internalizing antibodies are preferred. However, lipofections or liposomes can also be used to deliver a substance/molecule of the invention into cells where that is desired. Where antibody fragments are used, the smallest inhibitory fragment is preferred. For example, based upon the variable-region sequences of an antibody, peptide molecules can be designed that retain the ability to bind activated HGF β chain and/or HGF β chain binding site on c-met and/or interfere with interaction between activated HGF β chain and c-met. Such peptides can be synthesized chemically and/or produced by recombinant DNA technology. See, *e.g.*, Marasco et al., Proc. Natl. Acad. Sci. USA, 90: 7889-7893 (1993). The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles, and nanocapsules) or in macroemulsions. Such techniques are disclosed in Remington's Pharmaceutical Sciences, *supra.*

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e.g.*, films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and $\gamma$ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

The following are examples of the methods and compositions of the invention. It is understood that various other embodiments may be practiced, given the general description provided above.

EXAMPLES

MATERIALS & METHODS

*Materials*

The mature forms of the Met ECD (Glu25 to Gln929) domain containing a C-terminal His$_6$ tag was expressed in insect cells and purified by Ni-NTA metal chelate and gel filtration chromatography using standard protocols described below. Met-IgG fusion protein was obtained as previously described (Mark et al., 1992).

*Expression and purification of HGF $\beta$ proteins*

HGF $\beta$ proteins were expressed in insect cells using baculovirus secretion vector pAcGP67 (BD Biosciences, Pharmingen, San Diego, CA), which contains a signal sequence for secretion of the product into the media. All constructs contained a His$_6$ tag at the carboxy terminus and were purified to homogeneity (>95% purity) by Ni NTA metal chelate and gel filtration chromatography. For wildtype HGF $\beta$, a cDNA fragment encoding the HGF $\beta$-chain from residues Val495 [c16] to Ser728 [c250] was cloned by PCR such that Val495 [c16] was inserted immediately after the secretion signal sequence. Site-directed mutagenesis was carried out using QuikChange™ (Stratagene, La Jolla, CA) with oligonucleotide 5'CCTAATTATGGATCCACAATTCCTG3' to make HGF $\beta$ containing a Cys604 [c128] to Ser mutation (HGF $\beta$) to avoid potential complications of an exposed unpaired Cys in the protease-like domain. HGF $\beta$ mutants Y513A [c36], R516A [c39], Q534A [c57], D578A [c102], Y619A [c143], Y673A [c195], V692A [c214], P693D [c215], G694E [c216], R695A [c217], G696A [c219], I699A [c221a] and R702A [c224] were made as above in the HGF $\beta$ construct (having the C604S mutation). HGF $\beta$ mutant C561S [c78] (i.e., C561S:C604S) was also made as above in the HGFβ construct in order to eliminate both free cysteines. proHGF $\beta$ encodes HGF from residues Asn479 to Ser728 and has a R494E mutation made using the oligonucleotide 5'CAAAACGAAACAATTGGAAGTTGTAAATGGGATTC3'. The cysteine was not altered in this construct to allow putative disulfide formation between Cys487 and Cys604. Numbering of amino acid position is as follows: full length HGF sequence [chymotrypsinogen numbering].

Baculovirus vectors containing the desired inserts were transfected into *Spodoptera frugiperda* (Sf 9) cells on plates in TNM-FH media via the Baculogold™ Expression System according to manufacturer's instructions (BD Biosciences Pharmingen, San Diego, CA). After 2-4 rounds of virus amplification, 10 ml of viral stock was used to infect 11 of High Five™ cells (Invitrogen, San Diego, CA) in suspension at $5\times10^5$ cells/ml in TNM-FH media. Cultures were incubated at 27 °C for 72 h before harvesting the culture media by centrifugation at 8,000 x g for 15 min. Cell culture media was applied to a 4 ml Ni-NTA agarose column (Qiagen, Valencia, CA). After washing with 4 column volumes of 50 mM Tris•HCl, pH 8.0, 500 mM NaCl, 5 mM imidazole, HGF $\beta$ proteins were eluted with 50 mM Tris•HCl pH 8.0, 500 mM NaCl, 500 mM imidazole. The eluate was pooled and applied to a Superdex™ -200 column (Amersham Biosciences, Piscataway, NJ) equilibrated in 10 mM HEPES pH 7.2, 150 mM NaCl, 5 mM CaCl$_2$. Protein peaks were collected and concentrated using a Centriprep™ YM-10 (Millipore, Bedford, MA). Fractions were analyzed by 12% SDS-PAGE stained with Coomassie blue. All mutations were verified by DNA sequencing and mass spectrometry. Protein concentration was determined by quantitative amino acid analysis. N-terminal sequencing revealed a single correct N-terminus present for proHGF β and HGF β. Purified proteins showed the correct molecular mass on SDS-PAGE; multiple bands observed were likely due to heterogeneous glycosylation, consistent with the mass spectrometry data having molecular masses - 2 kDa higher than predicted from the sequence.

*Construction, expression and purification of full length HGF proteins*

Recombinant proteins were produced in 11 cultures of Chinese hamster ovary (CHO) cells by transient transfection (Peek et al., 2002). Amino acid changes were introduced by site-directed mutagenesis (Kunkel, 1985) and verified by DNA sequencing. The expression medium (F-12/Dulbecco's modified Eagle's medium) contained 1% (v/v) ultra low IgG fetal bovine serum (FBS) (Gibco, Grand Island, NY). After 8 days the medium was harvested and supplemented with FBS to give a final content of 5-10% (v/v). Additional incubation for 2-3 days at 37 °C resulted in complete single-chain HGF conversion. This step was omitted for expression of proHGF, an uncleavable single chain form, which has amino acid changes at the activation cleavage site (R494E) and at a protease-susceptible site in the α-chain (R424A) (Peek et al., 2002). Mutant proteins were purified from the medium by HiTrap-Sepharose SP cation exchange chromatography (Amersham Biosciences, Piscataway, NJ) as described (Peek et al., 2002). Examination by SDS-PAGE (4-20% gradient gel) under reducing conditions and staining with Simply Blue Safestain showed that all mutant HGF proteins were >95% pure and were fully converted into α/β-heterodimers except for proHGF, which remained as a single-chain form. Protein concentration for each mutant was determined by quantitative amino acid analysis.

*HGF β and Met binding affinity by surface plasmon resonance*

The binding affinity of HGF β for Met was determined by surface plasmon resonance using a Biacore 3000 instrument (Biacore, Inc., Piscataway, NJ). The Met ECD domain was immobilized on a CM5 chip using amine coupling at -2000 resonance units according to the manufacturer's instructions. A series of concentrations of HGF β (i.e., C604S mutant) in 10 mM HEPES pH 7.2, 150 mM NaCl, 5 mM $CaCl_2$ ranging from 12.5 nM to 100 nM were injected at a flow rate of 20 μl/min for 40 s. Bound HGF β was allowed to dissociate for 10 min. Appropriate background subtraction was carried out. The association ($k_{on}$) and dissociation ($k_{off}$) rate constants were obtained by a global fitting program provided with the instrument; the ratio of $k_{off}/k_{on}$ was used to calculate the dissociation constant ($K_d$).

*Binding of HGF β to Met and competition binding ELISA*

Microtiter plates (Nunc, Roskilde, Denmark) were coated overnight at 4°C with 2 μg/ml of rabbit anti-human IgG Fc specific antibody (Jackson ImmunoResearch Laboratory, West Grove, PA) in 50 mM sodium carbonate buffer, pH 9.6. After blocking with 1% BSA in HBS buffer (50 mM HEPES pH 7.2, 150 mM NaCl, 5 mM $CaCl_2$ and 0.1% Tween-20), 1 μ/ml Met-IgG fusion protein (Mark et al., 1992) was added and plates were incubated for 1 h with gentle shaking at room temperature. After washing with HBS buffer, HGF β proteins were added for 1 h. Bound HGF β was detected using anti-His-HRP (Qiagen, Valencia, CA) followed by addition of $TMB/H_2O_2$ substrate (KPL, Gaithersburg, MD). The reaction was stopped with 1M $H_3PO_4$ and the $A_{450}$ was measured on a Molecular Devices SpectraMax Plus[384] microplate reader. The effective concentration to give half-maximal binding ($EC_{50}$) was determined by a four parameter fit using Kaleidagraph (Synergy Software, Reading, PA).
In order to develop a competition ELISA, wildtype HGF β was biotinylated using a 20-fold molar excess of biotin-maleimide (Pierce, Rockford, IL) at room temperature for 2 h. Plates were treated as above except biotinylated wildtype HGF β was used and detected using HRP-neutravidin (Pierce, Rockford, IL). Competition assays contained a mixture of 250 nM biotinylated wildtype HGF β and various concentrations of proteins as indicated (e.g., unlabeled HGF β variants, HGF (i.e., 2-chain) or proHGF (i.e., HGF in single chain form)). After incubation for 1 h at room temperature, the amount of biotinylated wildtype HGF β bound on the plate was measured as described above. $IC_{50}$ values were determined by fitting the data to a four-parameter equation (Kaleidagraph, Synergy Software, Reading, PA).

*Binding of HGF mutants to Met*

Biotinylated HGF was prepared using the Sigma immunoprobe biotinylation kit (Sigma, St. Louis, MO). Microtiter plates were coated with rabbit anti-human IgG Fc specific antibody as above. Plates were washed in PBS 0.05% (v/v) Tween-20 followed by a 1 h incubation with 0.5% (w/v) of BSA, 0.05% Tween-20 in PBS, pH 7.4 at room temperature. After washing, 1 nM biotinylated HGF and 0.2 nM Met-IgG fusion protein (Mark et al., 1992) together with various concentrations of HGF mutants, wildtype HGF β was added to the wells and incubated for 2 h. After washing, bound biotinylated HGF was detected by addition of diluted (1:3000) streptavidin horseradish peroxidase conjugate (Zymed, South San Francisco, CA) followed by SureBlue TMB peroxidase substrate and stop solution TMB STOP (KPL, Gaithersburg, MD). The $A_{450}$ was measured and $IC_{50}$ values were determined as described above. Relative binding affinities are expressed as the $IC_{50}$(mutant)/$IC_{50}$(wildtype HGF).

*HGF-dependent phosphorylation of Met*

The kinase receptor activation assay (KIRA) was run as follows. Confluent cultures of lung carcinoma A549 cells (CCL-185, ATCC, Manassas, VA), previously maintained in growth medium (Ham's F12/DMEM 50:50 (Gibco, Grand Island, NY) containing 10% FBS, (Sigma, St. Louis, MO), were detached using Accutase (ICN, Aurora, OH) and seeded in 96 well plates at a density of 50,000 cells per well. After overnight incubation at 37˚C, growth media was removed and cells were serum starved for 30 to 60 min in medium containing 0.1 % FBS. Met phosphorylation activity by HGF or HGF mutants was determined from addition of serial dilutions from 500 to 0.2 ng/ml in medium containing 0.1 % FBS followed by a 10 min incubation at 37˚C, removal of media and cell lysis with 1X cell lysis buffer (Cat. #9803, Cell Signaling Technologies, Beverly, MA) supplemented with 1X protease inhibitor cocktail set I (Cat #539131, Calbiochem, San Diego, CA). HGF β-chain was carried out similarly starting at 5 μg/ml. Inhibition of HGF dependent Met phophorylation activity by HGF β-chain was determined from addition of serial dilutions from 156 to 0.06 nM to assay plates followed by a 15 min incubation at 37˚C, addition of HGF at 12.5, 25 or 50 nM, an additional 10 min incubation at 37˚C, removal of media and cell lysis as above. Cell lysates were analyzed for phosphorylated Met via an electrochemiluminescence assay using a Bio Veris M-Series instrument (Bio Veris Corporation, Gaithersburg, MD). Antiphosphotyrosine mAb 4G10 (Upstate, Lake Placid, MY) was labeled with BV-TAG via NHS-ester chemistry according to manufacturer's directions (Bio Veris). Anti-Met ECD mAb 1928 (Genentech, South San Francisco, CA) was biotinylated using biotin-X-NHS (Research Organics, Cleveland, OH). The BV-TAG-labeled 4G10 and biotinylated anti-Met mAb were diluted in assay buffer (PBS, 0.5% Tween-10, 0.5% BSA) and the cocktail was added to the cell lysates. After incubation at room temperature with vigorous shaking for 1.5 to 2 h, streptavidin magnetic beads (Dynabeads, Bio Veris) were added and incubated for 45 min. The beads with bound material (anti-Met antibody/Met/anti-phosphotyrosine antibody) were captured by an externally applied magnet. After a wash step the chemiluminescent signal generated by the light source was measured as relative luminescent units on a Bio Veris instrument. For each experiment, the Met phosphorylation induced by HGF mutants was expressed in percent of the maximal signal obtained with 2-chain HGF.

*Proliferation Assay*

BxPC3 (human pancreatic adeocarcinoma; ECACC No. 93120816) were obtained from the European Collection of Cell Cultures (CAMR Centre for Applied Microbiology and Research, Porton Down, Salisbury, Wiltshire (UK) and were used in an HGF-dependent proliferation assay. Cells were grown in RPMI medium containing 10% FCS (Sigma F-6178, St. Louis, MO), 10 mM HEPES, 2 mM glutamine, 1X Penicillin-Streptomycin (Invitrogen 15140-122, Carlsbad, CA), 100 X penicillin 10000 u/ml- streptomycin 10000 μg/ml), and G418 (Invitrogen 10131-035) at 250 μg/mL The cells were sequentially washed with PBS, PBS containing 10 mM EDTA, then removed using trypsin. The cells were harvested into serum containing media and the cell density was determined using a hemacytometer. Cells at 50000-75000/ml were seeded at 200 μl per well into a white bottom MT plate (Cultur Plate™ 6005680 Packard/PerkinElmer, Boston, MA) using the inside 60 wells only and allowed to grow for 24 h. The media was removed, the cells were washed with PBS and 200 μl of serum-free media containing 0.1 % BSA (SF-BSA) was added back to the cells. The cells were grown for an additional 24 h. The media was removed and the various test HGF proteins (n=4) in 150 μl of SF-BSA were added to the wells. 2-chain HGF was used at 1 nM final for inhibition assays. Controls were run in the absence of HGF and/or in the absence of test HGF proteins.
The cells were allowed to grow for 72 h and then assayed using the CellTiter-Glo Luminescent Kit (Promega G7571, Madison, WI). The procedure followed is described in Promega Technical Bulletin TB288. The microtiter plate was read on a Tropix TR717 microplate luminometer (Berthold 75323 Bad Wildbad, Germany). The percent of stimulation or inhibition of cell proliferation was normalized to the appropriate controls.

*Cell migration assay*

Breast cancer cells MDA-MB-435 (HTB-129, ATCC, Manassas, VA) were cultured in recommended serum-supplemented medium. Confluent cells were detached in PBS containing 10 mM EDTA and diluted with serum-free medium to a final concentration of 0.6-0.8 x $10^5$ cells/ml. 0.2 ml of this suspension (1.2-1.6 x $10^5$ total cells) was added in triplicate to the upper chambers of 24-well transwell plates (8 μm pore size) (HTS Multiwell™ Insert System, Falcon, Franklin Lakes, NJ) pre-coated with 10 μg/ml of rat tail collagen Type I (Upstate, Lake Placid, NY). Wildtype HGF or HGF mutants were added to the lower chamber at 100 ng/ml in serum-free medium, unless specified otherwise. HGF β-chain was also tested at 30 μg/ml. After incubation for 13-14 h, cells on the apical side of the membrane were removed and those that migrated to the basal side were fixed in 4% paraformaldehyde followed by staining with a 0.5% crystal violet solution. After washing and airdrying, cells were solubilized in 10% acetic acid and the $A_{560}$ was measured on a Molecular Devices microplate reader. Pro-migratory activities of HGF mutants were expressed as percent of HGF controls after subtracting basal migration in the absence of HGF. Photographs of stained cells were taken with a Spot digital camera (Diagnostics Instruments, Inc., Sterling Heights, MI) connected to a Leitz microscope (Leica Mikroskope & Systeme GmbH, Wetzlar, Germany). Pictures were acquired by Adobe Photoshop 4.0.1 (Adobe Systems Inc., San Jose, CA).

RESULTS

*Binding of HCF β to Met*

HGF β binding to Met was assessed from the change in resonance units measured by surface plasmon resonance on a CM5 chip derivatized with the extracellular domain of Met (Met ECD). The results show that HGF β binds to Met ECD with a $K_d$ of 87 nM calculated from relatively fast association ($k_{on}$ = 1.18 $\times$ 10$^5$ M$^{-1}$s$^{-1}$) and dissociation rate constants ($k_{off}$ = 0.0103 s$^-$) (Fig. 1A). Similar results were obtained for binding to the c-met Sema domain, where a Kd of 27 nM was calculated (data not shown). Binding of HGF β to Met was also confirmed by a second independent method using a plate ELISA. Following incubation of biotinylated HGF β with a properly oriented Met-IgG fusion bound to an immobilized anti-Fc antibody and detection with HRP-neutravidin, an EC$_{50}$ value of 320 $\pm$ 140 nM was determined (n = 6; data not shown).

Since single-chain HGF binds to Met with comparable affinity to two-chain HGF, but does not induce Met phosphorylation (Lokker et al., 1992; Hartmann et al., 1992), we hypothesized that this may be due to the lack of a Met binding site in the uncleaved form of the β-chain. To test this hypothesis, we expressed and purified proHGF β, a zymogen-like form of HGF β containing the C-terminal 16 residues from the HGF α-chain and a mutation at the cleavage site (R494E) to ensure that the single-chain form remained intact. Binding of HGF β and proHGF β to Met was determined with a competition binding ELISA, resulting in IC$_{50}$ values of 0.86 $\pm$ 0.17 and 11.6 $\pm$ 1.8 μM, respectively (Fig. 1B). The 13.5-fold reduced binding shows that while a Met binding site on the zymogen-like HGF β does in fact exist, it is not optimal. The loss of binding affinity of proHGF β is also exemplified in the data summarized in Fig. 5. Indeed, in other rounds of experimentations, zymogen-like β chain was found to be much less efficient in its ability to compete with the labeled β chain for binding to c-Met, having an IC$_{50}$ of ca. 41 μM, about 75-fold higher than the value found for HGF β chain of 0.56 μM in this assay, demonstrating that zymogen-like HGF β chain has significantly decreased binding to c-Met (data not shown). Therefore, a variety of experiments confirm that zymogen-like β chain (proHGF β) is a sub-optimal Met ligand.

*Inhibition of activity by HGF β*

Although HGF β binds to Met, it does not induce Met phosphorylation (Fig. 1C). Figure 1C shows that HGF β was completely inactive, even at concentrations that exceeded optimal phosphorylation activity by full length HGF by >1000-fold. Similarly, in MDA-MB-435 cell migration assays, HGF β at concentrations of up to 0.95 μM had no effect. However, HGF β does inhibit HGF-dependent phosphorylation of Met in a concentration dependent manner (Fig. 1D), although the inhibition was incomplete at the highest concentration used. Inhibition of Met phosphorylation is consistent with a direct competition with HGF for Met binding. In agreement with this, competition binding assays show that HGF β inhibits full length HGF binding to Met (Fig. 1E), albeit at rather high concentrations (IC$_{50}$ = 830 $\pm$ 26 nM; n = 3). By comparison, full length wildtype HGF had an IC$_{50}$ value of 0.86 $\pm$ 0.47 nM (n = 3) in this assay.

*Mutations in HGF and HGF β affect cell migration and Met phosphorylation*

To identify the Met binding site in the β-chain we systematically changed residues in regions corresponding to the activation domain and the active site of serine proteases, herein referred to as 'activation domain' and 'active site region' of HGF. Initial expression of HGF mutants in CHO cells yielded a mixture of single- and two-chain HGF forms, exemplified by mutant HGF I623A (Fig. 2A). Complete conversion of residual uncleaved HGF was accomplished by additional exposure of the harvested culture medium to 5-10% serum for several days (Fig. 2A). The purity of HGF I623A following purification by cation exchange chromatography is representative of all HGF mutants (Fig. 2A).

The functional consequence of mutating β-chain residues in HGF was assessed by determining the ability of the HGF mutants to stimulate migration of MDA-MB435 cells. The results showed that 3 HGF mutants, R695A [c217], G696A [c219] and Y673A [c195] were severely impaired, having less than 20% of wildtype activity, while 5 mutants Q534A [c57], D578A [c102], V692A [c214], P693A [c215] and G694A [c216] had 20%-60% of wildtype activity (Fig. 2B).

An additional set of 9 mutants (R514A, P537A, Y619A, T620A, G621A, K649A, I699A, N701A and R702A) had 60-80% of wildtype activity. The remaining 21 mutants had activities >80% that of wildtype and were considered essentially unchanged from HGF. As expected, proHGF did not stimulate cell migration (Fig. 2B). The decreased ability of 1 nM R695A [c217] or G696A [c219] to promote cell migration is illustrated in Figure 2C, showing that migration in the presence of either mutant is similar to basal migration in the absence of HGF.

To examine whether reduced activities in cell migration correlated with reduced Met phosphorylation, a subset of HGF mutants was examined in a kinase receptor assay (KIRA). For wildtype HGF and HGF mutants, maximal Met phosphorylation was observed at concentrations between 0.63 and 1.25 nM (Fig. 3). The maximal Met phosphorylation achieved by mutants Y673A [c195], R695A [c217] and G696A [c219] was less than 30% of wildtype, agreeing with their minimal or absent pro-migratory activities. Mutants Q534A [c57], D578A [c102] and V692A [c214] had intermediate activities (30-60%) in cell migration assays; they also had intermediate levels of Met phosphorylation, having 56%-83% that of wildtype HGF. In agreement with its lack of cell migration activity, proHGF had no Met phosphorylation activity (Fig. 3).

*Effect of β-chain mutations on binding of HGF and HGF β-chain to Met*

The affinity of each mutant to Met-IgG fusion protein was analyzed by HGF competition binding. Except for K649A [c173] and Y673A [c195] (both ca. 4-fold weaker binding), all (>30) HGF mutants had essentially the same binding affinity as two-chain HGF ($IC_{50}$ = 0.83 $\pm$ 0.32 nM; n = 30), indicated by their $IC_{50}$ ratios ($IC_{50}$mut/$IC_{50}$ WT), which ranged from 0.36 to 2.25 (Figure 7). HGF Y673A [c195] and proHGF showed ca. 4-fold weaker binding to Met-IgG compared to HGF (Figure 7). [It should be noted that while absolute values of binding affinity measurements can vary between experiments, the effect of specific mutations on binding ability compared to wild type is reproducible between multiple experiments.] We also examined the cell migration activities of selected mutants at 10- and 50-fold higher concentrations; no increase in pro-migratory activity was observed (Figure 8). Therefore, the impaired function of HGF mutants is not due to reduced binding to Met, since an increase in concentration of up to 50-fold had no compensatory effect.

The poor correlation between HGF mutant binding to Met and either HGF-dependent cell migration or Met phosphorylation is likely due to the relatively high affinity between Met and the HGF α-chain, which could mask any reduced affinity due to the β-chain. Therefore, we made selected mutations in HGF β itself to eliminate any α-chain effects. HGF β mutants Y513A [c36], R516A [c39], Q534A [c57], D578A [c102], Y619A [c143],Y673A [c195], V692A [c214], P693D [c215], G694E [c216], R695A [c217], G696A [c219], I699A [c221a] and R702A [c224] were tested in a competition ELISA with biotinylated HGF β binding to Met-IgG. HGF β mutant C561S [c78] (C604S:C561S) was tested to assess activity in mutants with no free cysteines. Mutants were made in the HGF β C604S [c128] background to avoid any potential dimerization during purification, although this mutation had no effect on binding to Met-IgG. The binding affinities of the mutants were then normalized to HGF β, which had an $IC_{50}$ - 0.55 $\pm$ 0.38 $\mu$M (n = 16). Results are shown in Fig. 5. A selected subset of these are graphically depicted in Fig. 4. Most mutants had reduced binding affinity to Met and some mutants - e.g R695A [c217] and G696A [c219] - did not compete for binding at all (see Fig. 5). We now see a strong correlation for reduced activity of full length two-chain HGF mutants with reduced binding of the corresponding mutant of HGF β. It was found that some mutants (e.g. R695A [c217], G696A [c219] and Y673A [c195]), that had the greatest loss in migration activity (as 2-chain full length HGF mutants) also had the greatest loss in Met binding (as HGF β mutants). Conversely, mutants with a small reduction of migration activity (e.g. Y619A [c143] and I699A [c221a]) also had a small (less than 10-fold) reduction in Met binding Fig. 5. Thus, the elimination of HGF α-chain binding contribution in this Met binding assay revealed that the reduced migration activity of full length HGF mutants was due to an impaired binding interaction of the HGF β-chain with the Met receptor.

*Mutations in HGF result in reduction in growth stimulatory activity and enhanced inhibition of HGF-dependent cell proliferation*

As shown in Fig. 6A, mutants in HGF β chain are less active as activators of proliferation in BxPC3 cells. The exemplary mutants in Fig. 6A reflect a wide spectrum of magnitudes of reduction in growth stimulatory activity. Note that HGF WT activity at 25 ng/ml was 83.6 $\pm$ 13.0 % (n = 12) of the activity at 100 ng/ml. % activity refers to the amount of proliferation in the presence of HGF or HGF mutant (100 ng/ml or 25 ng/ml) minus amount of proliferation in the absence of HGF). SD is the standard deviation and n is the number of independent determinations.

Mutants in HGF β chain are also capable of acting as inhibitors of cell proliferation in the presence of wild type HGF (Fig. 6B). In Fig. 6B, relative activity refers to relative activity in proliferation assay. % Inhibition can be calculated in several ways, two of which are shown in Fig. 6B. % activity I refers to the amount of proliferation normalized to no HGF (0%) and 25 ng/ml HGF WT (100%). Thus HGF R695A or HGF R424A:494E inhibit 79% or 63% of HGF-dependent cell proliferation activity, respectively. % activity 11 refers to the amount of proliferation normalized to 5 $\mu$g/ml HGF

R695A (0%) or HGF R424:R494E (0%) and 25 ng/ml HGF WT (100%). Thus HGF R695A or HGF R424A:494E HGF inhibit 68% or 75% of HGF-dependent cell proliferation activity, respectively. Note that HGF WT was at 25 ng/ml 2-chain HGF; 2-chain R695A and 1-chain R424A:494E were at 5 $\mu$g/ml.

*Binding of HGF β chain to c-Met cannot be competed by single chain pro-HGF*

Our data indicate that the HGF β chain binds to c-Met, and more specifically to the Sema domain of c-Met. The HGF α chain also binds to c-Met and may also bind to the Sema domain. We addressed whether the binding sites for these two chains might overlap on c-Met. The results showed that single chain pro-HGF, having an intact α chain and a zymogen-like β chain, does not compete with HGF β chain binding to c-Met-IgG at the concentrations indicated in Fig. 9. However, two chain HGF, having an intact α chain and an activated β chain, does compete with an $IC_{50}$ of 19 nM, supporting the conclusion that α and β chains bind at different sites on c-Met (Fig. 9). A control experiment in a competition ELISA showed that the single chain pro-HGF competed with biotinylated two chain HGF binding to c-Met-IgG with an $IC_{50}$ value of 12 nM, similar to the $IC_{50}$ value of 6 nM for two chain HGF (data not shown).

DISCUSSION

HGF acquires biological activity upon proteolytic conversion of the single chain precursor form into two-chain HGF (Naka et al., 1992; Hartmann et al., 1992; Lokker et al., 1992; Naldini et al. 1992). Based on the structural similarity of HGF with chymotrypsin-like serine proteases (Perona and Craik, 1995; Rawlings et al., 2002; Donate et al., 1994) and plasminogen in particular, we hypothesize that this activation process is associated with structural changes occurring in the HGF β-chain. Herein is provided evidence that the 'activated' HGF β-chain contains a distinct Met binding site located in a region that corresponds to the substrate/inhibitor binding site of chymotrypsin-like serine proteases.

*HGF binding interactions to Met*

Binding studies with purified HGF β-chains revealed that the 'activated' form of HGF β (Val495-Ser728) binds to Met with ca. 14-fold higher affinity than its precursor form, proHGF β (Asn479-Ser728), consistent with the view that optimization of the Met binding site is contingent upon processing of single-chain HGF. This suggested that the Met binding site includes the HGF region undergoing conformational rearrangements after scHGF cleavage, i.e. the 'activation domain'. Indeed, functional analysis of HGF variants with amino acid substitutions in the 'activation domain' led to the identification of the functional Met binding site. However, HGF mutants with the greatest losses in pro-migratory activities (Q534A, D578A, Y673A, V692A, P693A, G694A, R695A, G696A and R702A) displayed essentially unchanged binding affinities for Met, except for Y673A (4-fold loss), because HGF affinity is dominated by the HGF α-chain (Lokker et al., 1994; Okigaki et al., 1992). Consistent with this, the reduced activities remained unchanged upon increasing the concentration of HGF mutants by more than 50-fold (Fig. 8). Therefore, the reduced activities of HGF mutants were interpreted as resulting from perturbed molecular interactions of HGF β-chain with its specific, low affinity binding site on Met. In support of this, we found that the reduced biological activities of selected HGF mutants (2-chain full length) were well correlated with reduced Met binding of the corresponding HGF β mutants in an assay that eliminated the binding contribution of the HGF α-chain. For instance, the HGF β mutants R695A [c217], G696A [c219] and Y673A [c195] had no measurable Met binding, correlating with greatly impaired biological functions as full length mutants.

In agreement with the data for a relatively low affinity binding site for HGF β binding to Met, surface plasmon resonance experiments with immobilized Met extracellular domain showed that HGF β bound Met with a $K_d$ of ca. 90 nM. The apparent affinity differences observed between $K_d$ and $IC_{50}$ values are due to the different assays used, e.g. where the higher $IC_{50}$ values reflect the higher concentrations of HGF β necessary to compete with 250 nM biotinylated HGF β for binding to Met.

The functional Met binding site is centered on 'catalytic triad residues' Gln534 [c57], Asp578 [c102], Tyr673 [c195] and the [c220]-loop (residues Val692, Gly694, Arg695 and Gly696). All of our Ala substitutions would not require large changes of main chain conformation except at Gly696. Here, phi/psi angles of 50˚/146˚ and substitution of non-Gly residue would cause conformational changes in the [c220]-loop, leading to reduced activity of the G696A mutant. Together, these residues bear a remarkable resemblance to the substrate-processing region of true serine proteases. This finding agrees with an earlier study, which identified Y673 and V692 as important residues for Met activation (Lokker et al., 1992). The normal activity measured for the HGF variant Q534H in that study may reflect functional compensation of Gln by His, a relatively close isostere.

The functional importance of the [c220]-loop has precedent in the well-described family of chymotrypsin-like serine proteases (Perona and Craik, 1994; Hedstrom, 2002). The extended canonical conformation of substrates and inhibitors includes residues that can form main chain interactions from [c214-c218]. This region is also recognized as an allosteric regulator of thrombin catalytic activity (Di Cera et al., 1995) and as an interaction site with its inhibitor hirudin (Stubbs and Bode, 1993). In addition, residues in Factor VIIa and thrombin that correspond to HGF R695 [c217] are important for enzyme-catalyzed substrate processing (Tsiang et al., 1995; Dickinson et al., 1996). Moreover, the corresponding residue in MSP, R683 [c217], plays a pivotal role in the high affinity interaction of MSP β-chain with its receptor Ron (Danilkovitch et al., 1999). MSP R683 [c217] is part of a cluster of five surface exposed arginine residues proposed to be involved in high affinity binding to Ron (Miller and Leonard, 1998). Although only R695 [c217] and possibly K649 [c173] are conserved in HGF, these residues are all located within the Met binding region of the HGF β-chain, leading us to speculate that the Ron binding site on the MSP β-chain is highly homologous.

The results described herein with HGF Ala mutants agree with a previous study where Tyr673 [c195] and Val692 [c214] were each replaced by serine (Lokker et al., 1991). The normal biological activity measured for HGF variant Q534H [c57] in two previous reports (Lokker et al., 1991; Matsumoto et al., 1991) may reflect functional compensation of Gln by His, a relatively close isostere. However, our results contrast with previous studies demonstrating that HGF β-chain itself neither binds to nor inhibits HGF binding to Met (Hartmann et al., 1992; Matsumoto et al., 1998). In one instance, the HGF β-chain was different from ours, having extra α-chain residues derived from elastase cleavage of HGF, which could adversely affect Met binding. However, it is more likely that either the concentrations used, the sensitivity of the assays or the extent of pro-HGF processing may have been insufficient to observe binding to this low affinity site (Matsumoto et al., 1998). HGF β-chain has been reported to bind to Met, although only in the presence of NK4 fragment from the α-chain (Matsumoto et al., 1998).

*Signaling Mechanisms*

In principle, the existence of two Met binding sites - one high affinity and one low affinity - in one HGF molecule could support a 2:1 model of a Met:HGF signaling complex, analogous to the proposed 2:1 model of Ron:MSP (Miller and Leonard, 1998). In the related MSP/Ron ligand/receptor system, individual α- and β-chains of MSP, which are devoid of signaling activity, can bind to Ron and compete with full length MSP for receptor binding (Danilkovitch et al., 1999). The same is true in the HGF/Met system. However, biochemical studies have not identified any 2:1 complexes of Met:HGF (Gherardi et al., 2003). In addition, this model of receptor activation requires some as yet unknown molecular mechanism that would prevent one HGF molecule from simultaneously binding to one Met receptor through its α-and β-chains.

Alternatively, the HGF β-chain might have critical functions in receptor activation beyond those involved in direct interactions with Met that would favor a 2:2 complex of HGF:Met. We found that proHGF β,, the single chain 'unactivated' form of the HGF β-chain, bound more tightly to Met than several mutants in the 'activated' form of HGF β, i.e. Y673A, V692A and R695A (e.g., Fig. 5). Importantly, all three corresponding full length HGF mutants show measurable receptor phosphorylation and/or pro-migratory activities, however proHGF does not, even at concentrations 1000-fold more than that needed for activity by HGF. This significant distinction leads us to consider additional functions of the HGF β-chain in receptor activation.

*Conclusion*

In conclusion, the results presented herein show that the β-chain of HGF contains a hitherto unknown interaction site with Met, which is similar to the 'active site region' of serine proteases. Thus HGF is bivalent, having a high affinity Met binding site in the NK1 region of the α-chain. Other important interactions may occur between two HGF β-chains, two HGF α-chains (Donate et al., 1994) and, as found with MSP/Ron (Angeloni et al., 2004), between two Met Sema domains. Furthermore, heparin also plays a key role in HGF/Met receptor binding. The identification of a distinct Met binding site on the HGF β-chain provides a scientific and empirical rationale for the design of new classes of Met inhibitors with therapeutic potential for diseases such as cancer.

<u>PARTIAL LIST OF REFERENCES</u>

Angeloni, D., Danilkovitch-Miagkova, A., Miagkov, A., Leonard, E. J., and Lerman, M. I. (2004). The soluble sema domain of the RON receptor inhibits MSP-induced receptor activation. J. Biol. Chem., 279, 3726-3732.

Birchmeier, C., Birchmeier, W., Gherardi, E., and Vande Woude, G. F. (2003). Met, metastasis, motility and more. Nature Rev. Mol. Cell Biol. 4, 915-925.

Boose, J. A., Kuismanen, E., Gerard, R., Sambrook, J., and Gething, M. J. (1989). The single-chain form of tissue-type plasminogen activator has catalytic activity: Studies with a mutant enzyme that lacks the cleavage site. Bio-

# EP 2 367 008 A2

chemistry 28, 638-643.

Bottaro, D. P., Rubin, J. S., Faletto, D. L., Chan, A. M., Kmiecik, T. E., Vande Woude, G. F., and Aaronson, S. A. (1991). Identification of the hepatocyte growth factor receptor as the c-met protooncogene product. Science 251, 802-804.

Broze Jr., G. J. (2001). Protein Z-dependent regulation of coagulation. Thromb. Haemost. 86, 8-13.

CCP4 (1994). The CCP4 suite: Programs for protein crystallography. Acta Crystallogr D50, 760-763.

Chirgadze et al., FEBS Letters (1998), 430:126-129.

Cohen, G. H. (1997). Align - a program to superimpose protein coordinates, accounting for insertions and deletions. J Appl. Crystallog. 30, 1160-1161.

Cooper, C. S., Blair, D. G., Oskarsson, M. K., Tainsky, M. A., Eader, L. A., and Vande Woude, G. F. (1984a). Characterization of human transforming genes from chemically transformed, teratocarcinoma, and pancreatic carcinoma cell lines. Cancer Res. 44, 1-10.

Cooper, C. S., Park, M., Blair, D. G., Tainsky, M. A., Huebner, K., Croce, C. M., and Vande Woude, G. F. (1984b). Molecular cloning of a new transforming gene from a chemically transformed human cell line. Nature 311, 29-33.

Danilkovitch, A., Miller, M., and Leonard, E. J. (1999). Interaction of macrophage-stimulating protein with its receptor. J. Biol. Chem. 274, 29937-29943.

Danilkovitch-Miagkova, A., and Zbar, B. (2002). Dysregulation of Met receptor tyrosine kinase activity in invasive tumors. J. Clin. Invest. 109, 863-867.

Date et al., FEBS Lett. (1997), 420:1-6.

Dennis, M. S., Eigenbrot, C., Skelton, N. J., Ultsch, M. H., Santell, L., Dwyer, M. A., O'Connell, M. P., and Lazarus, R. A. (2000). Peptide exosite inhibitors of factor VIIa as anticoagulants. Nature 404, 465-470.

Derksen, P. W., Keehnen, R. M. J., Evers, L. M., van Oers, M. H. J., Spaargaren, M., and Pals, S. T. (2002). Cell surface proteoglycan syndecan-1 mediates hepatocyte growth factor binding and promotes Met signalling in multiple myeloma. Blood 99, 1405-1410.

Di Cera, E., Guinto, E. R., Vindigni, A., Dang, Q. D., Ayala, Y. M., Wuyi, M., and Tulinsky, A. (1995). The Na+ binding site of thrombin. J. Biol. Chem. 270, 22089-22092.

Dickinson, C. D., Kelly, C. R., and Ruf, W. (1996). Identification of surface residues mediating tissue factor binding and catalytic function of the serine protease factor VIIa. Proc. Natl. Acad. Sci. USA 93, 14379-14384.

Donate, L. E., Gherardi, E., Srinivasan, N., Sowdhamini, R., Aparicio, S., and Blundell, T. L. (1994). Molecular evolution and domain structure of plasminogen-related growth factors (HGF/SF and HGFI/MSP). Protein Sci. 3, 2378-2394.

Drain, J., Bishop, J. R., and Hajduk, S. L. (2001). Haptoglobin-related protein mediates trypanosome lytic factor binding to Trypanosomes. J. Biol. Chem. 276, 30254-30260.

Gherardi, E., Youles, M. E., Miguel, R. N., Blundell, T. L., Iamele, L., Gough, J., Bandyopadhyay, A., Hartmann, G., and Butler, P. J. (2003). Functional map and domain structure of MET, the product of the c-met protooncogene and receptor for hepatocyte growth factor/scatter factor. Proc. Natl. Acad. Sci. USA 100, 12039-12044.

Hartmann, G., Naldini, L., Weidner, K. M., Sachs, M., Vigna, E., Comoglio, P. M., and Birchmeier, W. (1992). A functional domain in the heavy chain of scatter factor/hepatocyte growth factor binds the c-Met receptor and induces cell dissociation. Proc. Natl. Acad. Sci. USA 89, 11574-11578.

Hartmann, G., Prospero, T., Brinkmann, V., Ozcelik, C., Winter, G., Hepple, J., Batley, S., Bladt, F., Sachs, M., Birchmeier, C., et al. (1998). Engineered mutants of HGF/SF with reduced binding to heparan sulphate proteoglycans, decreased clearance and enhanced activity in vivo. Curr. Biol. 8, 125-134.

Hedstrom, L. (2002). Serine protease mechanism and specificity. Chem. Rev. 102, 4501-4523.

Huber, R., and Bode, W. (1978). Structural basis of the activation and action of trypsin. Acc Chem. Res. 11, 114-122.

Kunkel, T. A. (1985) Proc. Natl. Acad. Sci USA 82, 488-492.

Kurosky, A., Barnett, D. R., Lee, T. H., Touchstone, B., Hay, R. E., Amott, M. S., Bowman, B. H., and Fitch, W. M. (1980). Covalent structure of human haptoglobin: a serine protease homolog. Proc. Natl. Acad. Sci. USA 77, 3388-3392.

Lijnen, H. R., Van Hoef, B., Nelles, L., and Collen, D. (1990). Plasminogen activation with single-chain urokinase-type plasminogen activator (scu-PA). Studies with active site mutagenized plasminogen (Ser740 $\to$ Ala) and plasmin-resistant scu-PA (Lys158 $\to$ Glu). J. Biol. Chem. 265, 5232-5236.

Lin, C.-Y., Anders, J., Johnson, M., Sang, Q. A., and Dickson, R. B. (1999). Molecular cloning of cDNA for matriptase, a matrix-degrading serine protease with trypsin-like activity. J. Biol. Chem. 274, 18231-18236.

Lokker, N. A., Mark, M. R., Luis, E. A., Bennett, G. L., Robbins, K. A., Baker, J. B., and Godowski, P. J. (1992). Structure-function analysis of hepatocyte growth factor: identification of variants that lack mitogenic activity yet retain high affinity receptor binding. EMBO J. 11, 2503-2510.

Lokker, N. A., Presta, L. G., and Godowski, P. J. (1994). Mutational analysis and molecular modeling of the N-terminal kringle-containing domain of hepatocyte growth factor identifies amino acid side chains important for interaction with the c-Met receptor. Protein Eng. 7, 895-903.

Ma, P. C., Maulik, G., Christensen, J., and Salgia, R. (2003). c-Met: structure, functions and potential for therapeutic inhibition. Cancer Metastasis Rev. 22, 309-325.

Malkowski, M. G., Martin, P. D., Guzik, J. C., and Edwards, B. F. P. (1997). The co-crystal structure of unliganded bovine $\alpha$-thrombin and prethrombin-2: movement of the Tyr-Pro-Pro-Trp segment and active site residues upon ligand binding. Protein Sci. 6, 1438-1448.

Mark, M. R., Lokker, N. A., Zioncheck, T. F., Luis, E. A., and Godowski, P. J. (1992). Expression and characterization of hepatocyte growth factor receptor-IgG fusion proteins. Effects of mutations in the potential proteolytic cleavage site on processing and ligand binding. J. Biol. Chem. 267, 26166-26171.

Matsumoto, K., Takehara, T., Inoue, H., Hagiya, M., Shimizu, S., and Nakamura, T. (1991). Biochem. Biophys. Res. Commun. 181, 691-699.

Matsumoto, K., Kataoka, H., Date, K., and Nakamura, T. (1998). Cooperative interaction between $\alpha$- and $\beta$-chains of hepatocyte growth factor on c-Met receptor confers ligand-induced receptor tyrosine phosphorylation and multiple biological responses. J. Biol. Chem. 273, 22913-22920.

Maulik, G., Shrikhande, A., Kijima, T., Ma, P. C., Morrison, P. T., and Salgia, R. (2002). Role of the hepatocyte growth factor receptor, c-Met, in oncogenesis and potential for therapeutic inhibition. Cytokine Growth Factor Rev. 13, 41-59.

Miller, M., and Leonard, E. J. (1998). Mode of receptor binding and activation by plasminogen-related growth factors. FEBS Lett. 429, 1-3.

Miyazawa, K., Shimomura, T., Kitamura, A., Kondo, J., Morimoto, Y., and Kitamura, N. (1993). Molecular cloning and sequence analysis of the cDNA for a human serine protease responsible for activation of hepatocyte growth factor. J. Biol. Chem. 268, 10024-10028.

Naka, D., Ishii, T., Yoshiyama, Y., Miyazawa, K., Hara, H., Hishida, T., and Kitamura, N. (1992). Activation of hepatocyte growth factor by proteolytic conversion of single chain form to a heterodimer. J. Biol. Chem. 267, 20114-20119.

Nakamura, T., Nishizawa, T., Hagiya, M., Seki, T., Shimonishi, M., Sugimura, A., Tashiro, K., and Shimizu, S. (1989). Molecular cloning and expression of human hepatocyte growth factor. Nature 342, 440-443.

Naldini, L., Tamagnone, L., Vigna, E., Sachs, M., Hartmann, G., Birchmeier, W., Daikuhara, Y., Tsubouchi, H., Blasi, F., and Comoglio, P. M. (1992). Extracellular proteolytic cleavage by urokinase is required for activation of hepatocyte growth factor/scatter factor. EMBO J. 11, 4825-4833.

Okigaki, M., Komada, M., Uehara, Y., Miyazawa, K., and Kitamura, N. (1992). Functional characterization of human hepatocyte growth factor mutants obtained by deletion of structural domains. Biochemistry 31, 9555-9561.

Parry, M. A., Fernandez-Catalan, C., Bergner, A., Huber, R., Hopfner, K. P., Schlott, B., Guhrs, K. H., and Bode, W. (1998). The ternary microplasmin-staphylokinase-microplasmin complex is a proteinase-cofactor-substrate complex in action. Nat. Struct. Biol. 5, 917-923.

Peek, M., Moran, P., Mendoza, N., Wickramasinghe, D., and Kirchhofer, D. (2002). Unusual proteolytic activation of pro-hepatocyte growth factor by plasma kallikrein and coagulation factor XIa. J. Biol. Chem. 277, 47804-47809.

Peisach, E., Wang, J., de los Santos, T., Reich, E., and Ringe, D. (1999). Crystal structure of the proenzyme domain of plasminogen. Biochemistry 38, 11180-11188.

Perona, J. J., and Craik, C. S. (1995). Structural basis of substrate specificity in the serine proteases. Protein Sci. 4, 337-360.

Rawlings, N. D., O'Brien, E., and Barrett, A. J. (2002). MEROPS: the protease database. Nucl. Acid Res. 30, 343-346.

Renatus, M., Engh, R. A., Stubbs, M. T., Huber, R., Fischer, S., Kohnert, U., and W., B. (1997). Lysine 156 promotes the anomalous proenzyme activity of tPA: X-ray crystal structure of single-chain human tPA. EMBO J. 16, 4797-4805.

Richardson, J. L., Kroger, B., Hoeffken, W., Sadler, J. E., Pereira, P., Huber, R., Bode, W., and Fuentes-Prior, P. (2000). Crystal structure of the human $\alpha$-thrombin-haemadin complex: an exosite II-binding inhibitor. EMBO J. 19, 5650-5660.

Shimomura, T., Miyazawa, K., Komiyama, Y., Hiraoka, H., Naka, D., Morimoto, Y., and Kitamura, N. (1995). Activation of hepatocyte growth factor by two homologous proteases, blood-coagulation factor XIIa and hepatocyte growth factor activator. Eur J Biochem 229, 257-261.

Stubbs, M., and Bode, W. (1993). A player of many parts: The spotlight falls on thrombin's structure. Thromb. Res. 69, 1-58.

Takeuchi, T., Shuman, M. A., and Craik, C. S. (1999). Reverse biochemistry: use of macromolecular protease inhibitors to dissect complex biological processes and identify a membrane-type serine protease in epithelial cancer and normal tissue. Proc Natl Acad Sci USA 96, 11054-11061.

Trusolino et al., FASEB J. (1998), 12:1267-1280.

Trusolino, L., and Comoglio, P. M. (2002). Scatter-factor and semaphorin receptors: cell signalling for invasive growth. Nature Rev. Cancer 2, 289-300.

Tsiang, M., Jain, A. K., Dunn, K. E., Rojas, M. E., Leung, L. L. K., and Gibbs, C. S. (1995). Functional mapping of the surface residues of human thombin. J. Biol. Chem. 270, 16854-16863.

Vijayalakshmi, J., Padmanabhan, K. P., Mann, K. G., and Tulinsky, A. (1994). The isomorphous structures of prethrombin2, hirugen-, and PPACK-thrombin: changes accompanying activation and exosite binding to thrombin. Protein Sci. 3, 2254-2271.

Wang, D., Bode, W., and Huber. R (1985). Bovine chymotrypsinogen A: x-ray crystal structure analysis and refinement of a new crystal form at 1.8Å resolution. J. Mol. Biol. 185, 595-624.

Wang, D., Julian, F. M., Breathnach, R., Godowski, P. J., Takehara, T., Yoshikawa, W., Hagiya, M., and Leonard, E. J. (1997). Macrophage stimulating protein (MSP) binds to its receptor via the MSP β chain. J. Biol. Chem. 272, 16999-17004.

The following numbered paragraphs (paras.) contain further statements of various aspects of the present invention:-

1. A method of screening for or identifying a substance that selectively binds activated hepatocyte growth factor (HGF) β chain, said method comprising:

comparing (i) binding of a candidate substance to an activated HGF β chain, with (ii) binding of the candidate substance to a reference HGF β chain, wherein said reference β chain does not substantially bind to c-met,

whereby a candidate substance that exhibits greater binding affinity to the activated HGF β chain than to the reference HGF β chain is selected as a substance that selectively binds activated HGF β chain.

2. The method of para. 1 wherein the reference β chain is contained within a single chain HGF polypeptide.

3. The method of para. 1 wherein the reference β chain is fused at its N-terminus to a portion of the C-terminal region of HGF α chain, wherein position 494 (corresponding to wild type human HGF) of the C-terminal region is an amino acid other than arginine.

4. The method of para. 3 wherein the amino acid at position 494 is glutamic acid.

5. The method of para. 3 or 4 wherein the portion of the C-terminal region of HGF comprises amino acid sequence from residue 479 to 494 of human HGF.

6. A method of screening for a substance that blocks c-met activation, said method comprising screening for a substance that binds c-met and blocks specific binding of HGF β chain to c-met.

7. The method of para. 6 wherein the substance competes with HGF β chain for binding to c-met.

8. A method of modulating c-met activation in a subject, said method comprising administering to the subject a substance that modulates specific binding of HGF β chain to c-met, whereby c-met activation is modulated.

9. The method of para. 8 wherein the substance inhibits specific binding of HGF β chain to c-met, whereby c-met activation is decreased.

10. The method of para. 8 wherein the substance increases specific binding of HGF β chain to c-met, whereby c-met activation is increased.

11. A method of inhibiting c-met activated cell proliferation, said method comprising contacting a cell or tissue with a substance that inhibits specific binding of HGF β chain to c-met, whereby cell proliferation associated with c-met activation is inhibited.

12. A method of treating a pathological condition associated with activation of c-met in a subject, said method comprising administering to the subject a substance that inhibits specific binding of HGF β chain to c-met, whereby c-met activation is inhibited.

13. The method of any of paras. 8-12 wherein the substance is an activated HGF β chain that is not disulfide linked to an HGF alpha chain.

14. The method of any of para. 8-12, where the substance is a peptide comprising the sequence VDWVCFRDLGCDWEL.

15. The method of any of paras. 8-12, wherein the substance is obtained by any of the methods of paras. 1-7.

16. The method of any of paras. 1-15, wherein the substance is a small molecule, peptide, antibody, antibody fragment, aptamer, or mixtures thereof.

17. A method of screening for an HGF receptor antagonist which blocks binding of HGF to its receptor, said method comprising selecting for a substance that binds to at least one of residues 534, 578, 619, 673, 692, 693, 694, 695, 696, 699 and/or 702 of HGF β chain.

18. The method of para. 17, wherein the substance binds to at least residues 673 and 695.

19. The method of para. 18, wherein the substance also binds at least one of residues 534, 578 and 692.

20. A molecule that binds to activated hepatocyte growth factor β chain and inhibits specific binding of said activated HGF β chain to c-met.

21. The molecule of para. 20, wherein binding affinity of the molecule for the activated form of the β chain is greater than binding affinity of the molecule for the β chain in zymogen form.

22. The molecule of para. 20 or 21 which binds to the active site of then β chain.

23. The molecule of para. 22, wherein said active site comprises at least one of residues 534, 578, 619, 673, 692, 693, 694, 695, 696, 699 and/or 702 of the β chain.

24. The molecule of para. 22, wherein the activated β chain has a conformation of β chain obtained by cleavage of single chain HGF.

25. The molecule of para. 24, wherein said cleavage is at or adjacent to residues 494 and 495 of single chain HGF.

26. The molecule of para. 25, wherein said cleavage occurs between residues 494 and 495 of single chain HGF.

27. The molecule of para. 20 or 21, wherein said molecule is a small molecule, an antibody or fragment thereof, a peptide, or a combination thereof.

28. The molecule of para. 20 or 21, wherein binding of said molecule to the activated β chain inhibits c-met activation by HGF.

29. The molecule of para. 20 or 21, wherein binding of said molecule to the activated β chain inhibits cell proliferation induced by HGF.

30. The molecule of para. 20 or 21, wherein binding of said molecule to the activated β chain inhibits c-met receptor dimerization.

31. The molecule of any of paras. 20-30 which is obtained by the method of any of paras. 1-5 and 17-19.

32. The molecule of para. 20 or 21 which is a peptide comprising the sequence VDWVCFRDLGCDWEL.

33. A molecule that competes with hepatocyte growth factor β chain for binding to c-met.

34. The molecule of para. 33, wherein said molecule is a substance obtained by the method of any of paras. 6-7.

35. The molecule of any of paras. 33-34, wherein said molecule inhibits c-met receptor dimerization.

## Claims

1. A method of screening for or identifying a substance that selectively binds activated hepatocyte growth factor (HGF) β chain, said method comprising:

comparing (i) binding of a candidate substance to an activated HGF β chain, with (ii) binding of the candidate substance to a reference HGF β chain, wherein said reference β chain does not substantially bind to c-met, whereby a candidate substance that exhibits greater binding affinity to the activated HGF β chain than to the reference HGF β chain is selected as a substance that selectively binds activated HGF β chain.

2. The method of claim 1 wherein the reference β chain (i) is contained within a single chain HGF polypeptide, or (ii) is fused at its N-terminus to a portion of the C-terminal region of HGF α chain, wherein position 494 (corresponding to wild type human HGF) of the C-terminal region is an amino acid other than arginine.

3. The method of claim 2 (ii) wherein the amino acid at position 494 is glutamic acid and/or wherein the portion of the C-terminal region of HGF comprises amino acid sequence from residue 479 to 494 of human HGF.

4. A method of screening for a substance that blocks c-met activation, said method comprising screening for a substance that binds c-met and blocks specific binding of HGF β chain to c-met.

5. The method of claim 6 wherein the substance competes with HGF β chain for binding to c-met.

6. A method of inhibiting c-met activated cell proliferation, said method comprising contacting a cell or tissue with a substance that inhibits specific binding of HGF β chain to c-met, whereby cell proliferation associated with c-met activation is inhibited.

7. A method of treating a pathological condition associated with activation of c-met in a subject, said method comprising administering to the subject a substance that inhibits specific binding of HGF β chain to c-met, whereby c-met activation is inhibited.

8. The method of claim 6 or 7 wherein the substance is (i) an activated HGF β chain that is not disulfide linked to an HGF alpha chain; (ii) a peptide comprising the sequence VDWVCFRDLGCDWEL; or (iii) obtained by any of the methods of claims 1-5.

9. The method of any of claims 1-8, wherein the substance is a small molecule, peptide, antibody, antibody fragment, aptamer, or mixtures thereof.

10. A method of screening for an HGF receptor antagonist which blocks binding of HGF to its receptor, said method comprising selecting for a substance that binds to at least one of residues 534, 578, 619, 673, 692, 693, 694, 695, 696, 699 and/or 702 of HGF β chain.

11. The method of claim 10, wherein the substance binds to at least residues 673 and 695 and, optionally, wherein the substance also binds at least one of residues 534, 578 and 692.

12. A molecule that binds to activated hepatocyte growth factor β chain and inhibits specific binding of said activated HGF β chain to c-met.

13. The molecule of claim 12, wherein binding affinity of the molecule for the activated form of the β chain is greater than binding affinity of the molecule for the β chain in zymogen form.

14. The molecule of claim 12 or 13: (i) which binds to the active site of the β chain; (ii) wherein said molecule is a small molecule, an antibody or fragment thereof, a peptide, or a combination thereof; (iii) wherein binding of said molecule to the activated β chain inhibits c-met activation by HGF; (iv) wherein binding of said molecule to the activated β chain inhibits cell proliferation induced by HGF; (v) wherein binding of said molecule to the activated β chain inhibits c-met receptor dimerization; or (vi) which is a peptide comprising the sequence VDWVCFRDLGCDWEL.

15. The molecule of claim 14 (i), wherein (i) said active site comprises at least one of residues 534, 578, 619, 673, 692, 693, 694, 695, 696, 699 and/or 702 of the β chain; or (ii) the activated β chain has a conformation of β chain obtained by cleavage of single chain HGF and, optionally, wherein said cleavage is at or adjacent to residues 494 and 495 of single chain HGF or occurs between residues 494 and 495 of single chain HGF.

16. The molecule of any of claims 12-15 which is obtained by the method of any of claims 1-3 and 10-11.

**17.** A molecule that competes with hepatocyte growth factor β chain for binding to c-met.

**18.** The molecule of claim 17, wherein said molecule is a substance obtained by the method of any of claims 4-5 and/or wherein said molecule inhibits c-met receptor dimerization.

**FIG._1A**

FIG._1B

FIG._1C

FIG._1D

FIG._1E

FIG._2A

FIG. _2B

FIG._2C

FIG._3

EP 2 367 008 A2

**FIG._4**

# FIG._5

| Mutant | HGF β mutant Relative binding affinity[a] ($IC_{50}$ mutant/$IC_{50}$ HGF β) ± SD | 2-chain full length HGF mutant Percent reduction ± SD of pro-migratory activity[b] |
|---|---|---|
| Y513A [c36] | 2.04 ± 0.48 | n.d.[c] |
| R516A [c39] | 3.03 ± 0.48 | 2 ± 15 |
| Q534A [c57] | 12.5 ± 3.6 | 45 ± 16 |
| D578A [c102] | 16.6 ± 8.2 | 65 ± 9 |
| Y619A [c143] | 5.1 ± 0.2 | 27 ± 13 |
| Y673A [c195] | >100 | 86 ± 9 |
| V692A [c214] | >50 | 51 ± 18 |
| P693A [c215] | n.d.[c] | 66 ± 9 |
| P693D [c215] | >100 | n.d.[c] |
| G694A [c216] | n.d.[c] | 52 ± 20 |
| G694E [c216] | >100 | n.d.[c] |
| R695A [c217] | >100 | 109 ± 14 |
| G696A [c219] | >100 | 114 ± 14 |
| I699A [c221a] | 8.05 ± 1.5 | 20 ± 17 |
| R702A [c224] | >25 | 40 ± 12 |
| HGF β WT[d] | 0.79 ± .16 | 0 |
| proHGF[e] | 23.5 ± 10.0 | 101 ± 12 |
| C561S[f] | 0.85 ± 0.17 | n.d.[c] |

[a]determined using the HGF β/Met competition binding ELISA.
[b]relative to wildtype 2-chain HGF activity (=100%) in cell migration assay.
[c]not determined.
[d]refers to WT C604 for HGF β and 2-chain WT for full length HGF
[e]refers to R494E for HGF β and 1-chain R424A:R494E for full length HGF
[f]refers to C561S:C604S mutant for HGF β

## FIG._6A

| 2-chain HGF Proteins | 100 ng/ml Protein | | | 25 ng/ml Protein | | |
|---|---|---|---|---|---|---|
| | % activity | SD | n | % activity | SD | n |
| HGF WT | 100.0 | 0.0 | 9 | 100.0 | 13.0 | 12 |
| Q534A | 87.8 | 10.1 | 4 | 109.6 | 13.8 | 4 |
| D578A | 72.5 | 18.0 | 5 | 96.9 | 11.5 | 4 |
| Y673A | 49.1 | 12.0 | 8 | 76.6 | 12.1 | 7 |
| V692A | 84.0 | 8.9 | 4 | 106.2 | 9.7 | 5 |
| R695A | 28.9 | 21.0 | 7 | 38.6 | 30.2 | 7 |
| G696A | 10.6 | 9.8 | 8 | 21.8 | 15.9 | 6 |
| R424A:494E | -16.7 | 3.4 | 3 | -5.5 | 18.8 | 3 |

## FIG._6B

| HGF Proteins | Relative activity | % activity I | % activity II | n |
|---|---|---|---|---|
| No HGF | 1.0 | 0 | | 2 |
| HGF WT | 1.95 | 100 | 100 | 2 |
| R695A | .85 | -16 | 0 | 2 |
| R695A + HGF WT | 1.2 | 21 | 32 | 2 |
| R424A:494E | 1.15 | 16 | 0 | 2 |
| R424A:494E + HGF WT | 1.35 | 37 | 25 | 2 |

# FIG._7

| HGF mutant | $IC_{50}mut/IC_{50}WT \pm SD$ | HGF mutant | $IC_{50}mut/IC_{50}WT \pm SD$ |
|---|---|---|---|
| I499A [c20] | 0.52 | M637A [c163] | 1.38 |
| R514A [c36] | 1.41 ± 0.23 | K641A [c167] | 1.04 |
| N515A [c38] | 1.16 | K649A [c173] | 3.66 |
| Q534A [c57] | 2.04 ± 0.86 | A661N [c184a] | 1.04 ± 0.34 |
| P537A [c60a] | 1.67 | K663A [c186] | 0.73 ± 0.20 |
| R539A [c60c] | 0.94 | G665A [c188] | 0.36 ± 0.03 |
| I550A [c70] | 1.39 | E670A [c192] | 1.77 |
| D552A [c72] | 1.23 | Y673A [c195] | 4.41 ± 1.03 |
| V553A [c73] | 0.99 ± 0.26 | V692A [c214] | 1.74 ± 0.16 |
| E559A [c77] | 1.34 ± 0.07 | P693A [c215] | 1.37 ± 0.46 |
| E575A [c99] | 1.19 ± 0.05 | G694A [c216] | 1.76 ± 0.72 |
| G576A [c100] | 0.78 | R695A [c217] | 1.48 ± 0.52 |
| D578A [c102] | 1.86 ± 0.82 | G696A [c219] | 2.03 ± 1.04 |
| Y619A [c143] | 1.52 ± 0.28 | A698G [c221] | 0.73 ± 0.35 |
| T620A [c144] | 1.89 ± 0.32 | I699A [c221a] | 1.79 ± 0.70 |
| G621A [c145] | 1.08 ± 0.30 | P700A [c222] | 1.30 ± 0.46 |
| L622A [c146] | 1.04 ± 0.22 | N701A [c223] | 1.48 ± 0.59 |
| I623A [c149] | 0.49 ± 0.10 | R702A [c224] | 2.25 |
| N624A [c150] | 0.71 ± 0.18 | proHGF | 4.03 ± 1.05 |
| Y625A [c151] | 0.65 ± 0.26 | wildtype HGF[a] | 1 |

[a]Wildtype 2-chain HGF had an $IC_{50} = 0.83 \pm 0.32$ nM (n=30) in the HGF/Met competition binding ELISA.

Pro-migratory activities of HGF mutants at different concentrations

| Mutant | Pro-migratory activity at 1 nM (% of control) | Pro-migratory activity at 10 nM (% of control) | Pro-migratory activity at 50 nM (% of control) |
|---|---|---|---|
| Y673A | 13.9 ± 8.9 | 9.8 ± 8.3 | 9.1 ± 8.6 |
| V692A | 49.5 ± 17.7 | 20.9 ± 6.9 | 29.8 ± 6.3 |
| G694A | 47.6 ± 19.7 | 23.2 ± 11.4 | 21.2 ± 5.9 |
| R695A | -8.9 ± 5.4 | -4.4 ± 11.6 | 5.3 ± 10.2 |
| G696A | -13.6 ± 13.7 | 4.0 ± 19.8 | 2.8 ± 7.1 |

## FIG._8

**FIG._9**

## REFERENCES CITED IN THE DESCRIPTION

**Patent documents cited in the description**

- US 60476778 B
- US 60532117 B
- US 4816567 A
- US 4657760 A
- US 5206344 A
- US OR5225212 A
- WO 9703692 A
- WO 9640072 A
- WO 9607399 A
- US 5654010 A
- US 6121416 A, Clark
- US 5750373 A
- US 5780279 A
- US 5821047 A
- US 6040136 A
- US 5427908 A
- US 5580717 A

- US 5545807 A
- US 5545806 A
- US 5569825 A
- US 5625126 A
- US 5633425 A
- US 5661016 A
- WO 9308829 A
- WO 9627011 A
- US 4676980 A
- WO 9100360 A
- WO 92200373 A
- EP 03089 A
- WO 9411026 A
- US 4485045 A
- US 4544545 A
- US 5013556 A
- US 3773919 A

**Non-patent literature cited in the description**

- **SAMBROOK et al.** Molecular Cloning: A Laboratory Manual. 1989
- Oligonucleotide Synthesis. 1984
- Animal Cell Culture. 1987
- Methods in Enzymology. Academic Press, Inc,
- Current Protocols in Molecular Biology. 1987
- PCR: The Polymerase Chain Reaction. 1994
- **PERBAL BERNARD V.** A Practical Guide to Molecular Cloning. 1988
- **MORRISON et al.** *Proc. Natl. Acad. Sci. USA,* 1984, vol. 81, 6851-6855
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **VASWANI ; HAMILTON.** *Ann. Allergy, Asthma & Immunol.,* 1998, vol. 1, 105-115
- **HARRIS.** *Biochem. Soc. Transactions,* 1995, vol. 23, 1035-1038
- **HURLE ; GROSS.** *Curr. Op. Biotech.,* 1994, vol. 5, 428-433
- **MARKS et al.** *Bio/Technology,* 1992, vol. 10, 779-783
- **BARBAS et al.** *Proc Nat. Acad. Sci, USA,* 1994, vol. 91, 3809-3813

- **SCHIER et al.** *Gene,* 1995, vol. 169, 147-155
- **YELTON et al.** *J. Immunol.,* 1995, vol. 155, 1994-2004
- **JACKSON et al.** *J. Immunol.,* 1995, vol. 154 (7), 3310-9
- **HAWKINS et al.** *J. Mol. Biol.,* 1992, vol. 226, 889-896
- **AGNEW.** *Chem Intl. Ed. Engl.,* 1994, vol. 33, 183-186
- Cell cycle regulation, oncogenes, and antineoplastic drugs. **MURAKAMI et al.** The Molecular Basis of Cancer. WB Saunders, 1995, 13
- **SIEBENLIST et al.** *Cell,* 1980, vol. 20, 269
- Remington's Pharmaceutical Sciences. 1980
- The use of interspecies scaling in toxicokinetics. **MORDENTI, J. ; CHAPPELL, W. et al.** Toxicokinetics and New Drug Development. Pergamon, 1989, 42-96
- **JOHNSON et al.** *Nat. Med.,* 1996, vol. 2, 795-799
- **YASUDA.** *Biomed. Ther.,* vol. 27, 1221-1223
- **HORA et al.** *Bio/Technology,* 1990, vol. 8, 755-758
- Design and Production of Single Immunization Vaccines Using Polylactide Polyglycolide Microsphere Systems. **CLELAND.** Vaccine Design: The Subunit and Adjuvant Approach. Plenum Press, 1995, 439-462

- Controlled release of bioactive agents from lactide/glycolide polymer. **LEWIS.** Biodegradable Polymers as Drug Delivery Systems. Marcel Dekker, 1990, 1-41
- **FIELDS ; SONG.** *Nature (London,* 1989, vol. 340, 245-246
- **CHIEN et al.** *Proc. Natl. Acad. Sci. USA,* 1991, vol. 88, 9578-9582
- **CHEVRAY ; NATHANS.** *Proc. Natl. Acad. Sci. USA,* 1991, vol. 89, 5789-5793
- **DATE et al.** *FEBS Let.,* 1997, vol. 420, 1-6
- **KOHLER ; MILSTEIN.** *Nature,* 1975, vol. 256, 495
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MUNSON ; POLLARD.** *Anal. Biochem.,* 1980, vol. 107, 220
- **RIECHMANN et al.** *Nature,* 1988, vol. 332, 323-327
- **VERHOEYEN et al.** *Science,* 1988, vol. 239, 1534-1536
- **HOOGENBOOM ; WINTER.** *J. Mol. Biol,* 1991, vol. 227, 381
- **MARKS et al.** *J. Mol. Biol,* 1991, vol. 222, 581
- **COLE et al.** Monoclonal Antibodies and Cancer Therapy. Alan R. Liss, 1985, 77
- **BOERNER et al.** *J. Immunol,* 1991, vol. 147 (1), 86-95
- **MARKS et al.** *Biotechnology,* 1992, vol. 10, 779-783
- **LONBERG et al.** *Nature,* 1994, vol. 368, 856-859
- **MORRISON.** *Nature,* 1994, vol. 368, 812-13
- **FISHWILD et al.** *Nature Biotechnology,* 1996, vol. 14, 845-51
- **NEUBERGER.** *Nature Biotechnology,* 1996, vol. 14, 826
- **LONBERG ; HUSZAR.** *Intern. Rev. Immunol.,* 1995, vol. 13, 65-93
- **MILSTEIN ; CUELLO.** *Nature,* 1983, vol. 305, 537-539
- **TRAUNECKER et al.** *EMBO J.,* 1991, vol. 10, 3655-3659
- **SURESH et al.** *Methods in Enzymology,* 1986, vol. 121, 210
- **BRENNAN et al.** *Science,* 1985, vol. 229, 81
- **SHALABY et al.** *J. Exp. Med,* 1992, vol. 175, 217-225
- **KOSTELNY et al.** *J. Immunol.,* 1992, vol. 148 (5), 1547-1553
- **HOLLINGER et al.** *Proc. Natl. Acad. Sci. USA,* 1993, vol. 90, 6444-6448
- **GRUBER et al.** *J. Immunol.,* 1994, vol. 152, 5368

- **TUTT et al.** *J. Immunol.,* 1991, vol. 147, 60
- **CARON et al.** *J. Exp Med.,* 1992, vol. 176, 1191-1195
- **SHOPES.** *J. Immunol,* 1992, vol. 148, 2918-2922
- **WOLFF et al.** *Cancer Research,* 1993, vol. 53, 2560-2565
- **STEVENSON et al.** *Anti-Cancer Drug Design,* 1989, vol. 3, 219-230
- **VITETTA et al.** *Science,* 1987, vol. 238, 1098
- **EPSTEIN et al.** *Proc. Natl. Acad. Sci. USA,* 1985, vol. 82, 3688
- **HWANG et al.** *Proc. Natl Acad. Sci. USA,* 1980, vol. 77, 4030
- **MARTIN et al.** *J. Biol. Chem,* 1982, vol. 257, 286-288
- **GABIZON et al.** *J. National Cancer Inst.,* 1989, vol. 81 (19), 1484
- **MARASCO et al.** *Proc. Natl. Acad. Sci. USA,* 1993, vol. 90, 7889-7893
- **ANGELONI, D. ; DANILKOVITCH-MIAGKOVA, A. ; MIAGKOV, A. ; LEONARD, E. J. ; LERMAN, M. I.** The soluble sema domain of the RON receptor inhibits MSP-induced receptor activation. *J. Biol. Chem.,* 2004, vol. 279, 3726-3732
- **BIRCHMEIER, C. ; BIRCHMEIER, W. ; GHERARDI, E. ; VANDE WOUDE, G. F.** Met, metastasis, motility and more. *Nature Rev. Mol. Cell Biol.,* 2003, vol. 4, 915-925
- **BOOSE, J. A. ; KUISMANEN, E. ; GERARD, R. ; SAMBROOK, J. ; GETHING, M. J.** The single-chain form of tissue-type plasminogen activator has catalytic activity: Studies with a mutant enzyme that lacks the cleavage site. *Biochemistry,* 1989, vol. 28, 638-643
- **BOTTARO, D. P. ; RUBIN, J. S. ; FALETTO, D. L. ; CHAN, A. M. ; KMIECIK, T. E. ; VANDE WOUDE, G. F. ; AARONSON, S. A.** Identification of the hepatocyte growth factor receptor as the c-met protooncogene product. *Science,* 1991, vol. 251, 802-804
- **BROZE JR., G. J.** Protein Z-dependent regulation of coagulation. *Thromb. Haemost.,* 2001, vol. 86, 8-13
- The CCP4 suite: Programs for protein crystallography. *Acta Crystallogr,* 1994, vol. D50, 760-763
- **CHIRGADZE et al.** *FEBS Letters,* 1998, vol. 430, 126-129
- **COHEN, G. H.** Align - a program to superimpose protein coordinates, accounting for insertions and deletions. *J Appl. Crystallog.,* 1997, vol. 30, 1160-1161
- **COOPER, C. S. ; BLAIR, D. G. ; OSKARSSON, M. K. ; TAINSKY, M. A. ; EADER, L. A. ; VANDE WOUDE, G. F.** Characterization of human transforming genes from chemically transformed, teratocarcinoma, and pancreatic carcinoma cell lines. *Cancer Res.,* 1984, vol. 44, 1-10

- **COOPER, C. S. ; PARK, M. ; BLAIR, D. G. ; TAINSKY, M. A. ; HUEBNER, K. ; CROCE, C. M. ; VANDE WOUDE, G. F.** Molecular cloning of a new transforming gene from a chemically transformed human cell line. *Nature,* 1984, vol. 311, 29-33
- **DANILKOVITCH, A. ; MILLER, M. ; LEONARD, E. J.** Interaction of macrophage-stimulating protein with its receptor. *J. Biol. Chem.,* 1999, vol. 274, 29937-29943
- **DANILKOVITCH-MIAGKOVA, A. ; ZBAR, B.** Dysregulation of Met receptor tyrosine kinase activity in invasive tumors. *J. Clin. Invest.,* 2002, vol. 109, 863-867
- **DATE et al.** *FEBS Lett.,* 1997, vol. 420, 1-6
- **DENNIS, M. S. ; EIGENBROT, C. ; SKELTON, N. J. ; ULTSCH, M. H. ; SANTELL, L. ; DWYER, M. A. ; O'CONNELL, M. P. ; LAZARUS, R. A.** Peptide exosite inhibitors of factor VIIa as anticoagulants. *Nature,* vol. 404, 465-470
- **DERKSEN, P. W. ; KEEHNEN, R. M. J. ; EVERS, L. M. ; VAN OERS, M. H. J. ; SPAARGAREN, M. ; PALS, S. T.** Cell surface proteoglycan syndecan-1 mediates hepatocyte growth factor binding and promotes Met signalling in multiple myeloma. *Blood,* 2002, vol. 99, 1405-1410
- **DI CERA, E. ; GUINTO, E. R. ; VINDIGNI, A. ; DANG, Q. D. ; AYALA, Y. M. ; WUYI, M. ; TULINSKY, A.** The Na+ binding site of thrombin. *J. Biol. Chem.,* 1995, vol. 270, 22089-22092
- **DICKINSON, C. D. ; KELLY, C. R. ; RUF, W.** Identification of surface residues mediating tissue factor binding and catalytic function of the serine protease factor VIIa. *Proc. Natl. Acad. Sci. USA,* 1996, vol. 93, 14379-14384
- **DONATE, L. E. ; GHERARDI, E. ; SRINIVASAN, N. ; SOWDHAMINI, R. ; APARICIO, S. ; BLUNDELL, T. L.** Molecular evolution and domain structure of plasminogen-related growth factors (HGF/SF and HGFI/MSP. *Protein Sci.,* 1994, vol. 3, 2378-2394
- **DRAIN, J. ; BISHOP, J. R. ; HAJDUK, S. L.** Haptoglobin-related protein mediates trypanosome lytic factor binding to Trypanosomes. *J. Biol. Chem.,* 2001, vol. 276, 30254-30260
- **GHERARDI, E. ; YOULES, M. E. ; MIGUEL, R. N. ; BLUNDELL, T. L. ; IAMELE, L. ; GOUGH, J. ; BANDYOPADHYAY, A. ; HARTMANN, G. ; BUTLER, P. J.** Functional map and domain structure of MET, the product of the c-met protooncogene and receptor for hepatocyte growth factor/scatter factor. *Proc. Natl. Acad. Sci. USA,* 2003, vol. 100, 12039-12044
- **HARTMANN, G. ; NALDINI, L. ; WEIDNER, K. M. ; SACHS, M. ; VIGNA, E. ; COMOGLIO, P. M. ; BIRCHMEIER, W.** A functional domain in the heavy chain of scatter factor/hepatocyte growth factor binds the c-Met receptor and induces cell dissociation. *Proc. Natl. Acad. Sci. USA,* 1992, vol. 89, 11574-11578
- **HARTMANN, G. ; PROSPERO, T. ; BRINKMANN, V. ; OZCELIK, C. ; WINTER, G. ; HEPPLE, J. ; BATLEY, S. ; BLADT, F. ; SACHS, M. ; BIRCHMEIER, C. et al.** Engineered mutants of HGF/SF with reduced binding to heparan sulphate proteoglycans, decreased clearance and enhanced activity in vivo. *Curr. Biol.,* 1998, vol. 8, 125-134
- **HEDSTROM, L.** Serine protease mechanism and specificity. *Chem. Rev.,* 2002, vol. 102, 4501-4523
- **HUBER, R. ; BODE, W.** Structural basis of the activation and action of trypsin. *Acc Chem. Res.,* 1978, vol. 11, 114-122
- **KUNKEL, T. A.** *Proc. Natl. Acad. Sci USA,* 1985, vol. 82, 488-492
- **KUROSKY, A. ; BARNETT, D. R. ; LEE, T. H. ; TOUCHSTONE, B. ; HAY, R. E. ; AMOTT, M. S. ; BOWMAN, B. H. ; FITCH, W. M.** Covalent structure of human haptoglobin: a serine protease homolog. *Proc. Natl. Acad. Sci. USA,* 1980, vol. 77, 3388-3392
- **LIJNEN, H. R. ; VAN HOEF, B. ; NELLES, L. ; COLLEN, D.** Plasminogen activation with single-chain urokinase-type plasminogen activator (scu-PA). Studies with active site mutagenized plasminogen (Ser740 → Ala) and plasmin-resistant scu-PA (Lys158 → Glu. *J. Biol. Chem.,* 1990, vol. 265, 5232-5236
- **LIN, C.-Y. ; ANDERS, J. ; JOHNSON, M. ; SANG, Q. A. ; DICKSON, R. B.** Molecular cloning of cDNA for matriptase, a matrix-degrading serine protease with trypsin-like activity. *J. Biol. Chem.,* 1999, vol. 274, 18231-18236
- **LOKKER, N. A. ; MARK, M. R. ; LUIS, E. A. ; BENNETT, G. L. ; ROBBINS, K. A. ; BAKER, J. B. ; GODOWSKI, P. J.** Structure-function analysis of hepatocyte growth factor: identification of variants that lack mitogenic activity yet retain high affinity receptor binding. *EMBO J.,* 1992, vol. 11, 2503-2510
- **LOKKER, N. A. ; PRESTA, L. G. ; GODOWSKI, P. J.** Mutational analysis and molecular modeling of the N-terminal kringle-containing domain of hepatocyte growth factor identifies amino acid side chains important for interaction with the c-Met receptor. *Protein Eng.,* 1994, vol. 7, 895-903
- **MA, P. C. ; MAULIK, G. ; CHRISTENSEN, J. ; SALGIA, R.** c-Met: structure, functions and potential for therapeutic inhibition. *Cancer Metastasis Rev.,* 2003, vol. 22, 309-325
- **MALKOWSKI, M. G. ; MARTIN, P. D. ; GUZIK, J. C. ; EDWARDS, B. F. P.** The co-crystal structure of unliganded bovine α-thrombin and prethrombin-2: movement of the Tyr-Pro-Pro-Trp segment and active site residues upon ligand binding. *Protein Sci.,* 1997, vol. 6, 1438-1448

- **MARK, M. R. ; LOKKER, N. A. ; ZIONCHECK, T. F. ; LUIS, E. A. ; GODOWSKI, P. J.** Expression and characterization of hepatocyte growth factor receptor-IgG fusion proteins. Effects of mutations in the potential proteolytic cleavage site on processing and ligand binding. *J. Biol. Chem.,* 1992, vol. 267, 26166-26171
- **MATSUMOTO, K. ; TAKEHARA, T. ; INOUE, H. ; HAGIYA, M. ; SHIMIZU, S. ; NAKAMURA, T.** *Biochem. Biophys. Res. Commun.,* 1991, vol. 181, 691-699
- **MATSUMOTO, K. ; KATAOKA, H. ; DATE, K. ; NAKAMURA, T.** Cooperative interaction between $\alpha$- and $\beta$-chains of hepatocyte growth factor on c-Met receptor confers ligand-induced receptor tyrosine phosphorylation and multiple biological responses. *J. Biol. Chem.,* 1998, vol. 273, 22913-22920
- **MAULIK, G. ; SHRIKHANDE, A. ; KIJIMA, T. ; MA, P. C. ; MORRISON, P. T. ; SALGIA, R.** Role of the hepatocyte growth factor receptor, c-Met, in oncogenesis and potential for therapeutic inhibition. *Cytokine Growth Factor Rev.,* 2002, vol. 13, 41-59
- **MILLER, M. ; LEONARD, E. J.** Mode of receptor binding and activation by plasminogen-related growth factors. *FEBS Lett.,* 1998, vol. 429, 1-3
- **MIYAZAWA, K. ; SHIMOMURA, T. ; KITAMURA, A. ; KONDO, J. ; MORIMOTO, Y. ; KITAMURA, N.** Molecular cloning and sequence analysis of the cDNA for a human serine protease responsible for activation of hepatocyte growth factor. *J. Biol. Chem.,* 1993, vol. 268, 10024-10028
- **NAKA, D. ; ISHII, T. ; YOSHIYAMA, Y. ; MIYAZAWA, K. ; HARA, H. ; HISHIDA, T. ; KITAMURA, N.** Activation of hepatocyte growth factor by proteolytic conversion of single chain form to a heterodimer. *J. Biol. Chem.,* 1992, vol. 267, 20114-20119
- **NAKAMURA, T. ; NISHIZAWA, T. ; HAGIYA, M. ; SEKI, T. ; SHIMONISHI, M. ; SUGIMURA, A. ; TASHIRO, K. ; SHIMIZU, S.** Molecular cloning and expression of human hepatocyte growth factor. *Nature,* 1989, vol. 342, 440-443
- **NALDINI, L. ; TAMAGNONE, L. ; VIGNA, E. ; SACHS, M. ; HARTMANN, G. ; BIRCHMEIER, W. ; DAIKUHARA, Y. ; TSUBOUCHI, H. ; BLASI, F. ; COMOGLIO, P. M.** Extracellular proteolytic cleavage by urokinase is required for activation of hepatocyte growth factor/scatter factor. *EMBO J.,* 1992, vol. 11, 4825-4833
- **OKIGAKI, M. ; KOMADA, M. ; UEHARA, Y. ; MIYAZAWA, K. ; KITAMURA, N.** Functional characterization of human hepatocyte growth factor mutants obtained by deletion of structural domains. *Biochemistry,* 1992, vol. 31, 9555-9561
- **PARRY, M. A. ; FERNANDEZ-CATALAN, C. ; BERGNER, A. ; HUBER, R. ; HOPFNER, K. P. ; SCHLOTT, B. ; GUHRS, K. H. ; BODE, W.** The ternary microplasmin-staphylokinase-microplasmin complex is a proteinase-cofactor-substrate complex in action. *Nat. Struct. Biol.,* 1998, vol. 5, 917-923
- **PEEK, M. ; MORAN, P. ; MENDOZA, N. ; WICKRAMASINGHE, D. ; KIRCHHOFER, D.** Unusual proteolytic activation of pro-hepatocyte growth factor by plasma kallikrein and coagulation factor XIa. *J. Biol. Chem.,* 2002, vol. 277, 47804-47809
- **PEISACH, E. ; WANG, J. ; DE LOS SANTOS, T. ; REICH, E. ; RINGE, D.** Crystal structure of the proenzyme domain of plasminogen. *Biochemistry,* 1999, vol. 38, 11180-11188
- **PERONA, J. J. ; CRAIK, C. S.** Structural basis of substrate specificity in the serine proteases. *Protein Sci.,* 1995, vol. 4, 337-360
- **RAWLINGS, N. D. ; O'BRIEN, E. ; BARRETT, A. J.** MEROPS: the protease database. *Nucl. Acid Res.,* 2002, vol. 30, 343-346
- **RENATUS, M. ; ENGH, R. A. ; STUBBS, M. T. ; HUBER, R. ; FISCHER, S. ; KOHNERT, U. ; W., B.** Lysine 156 promotes the anomalous proenzyme activity of tPA: X-ray crystal structure of single-chain human tPA. *EMBO J.,* 1997, vol. 16, 4797-4805
- **RICHARDSON, J. L. ; KROGER, B. ; HOEFFKEN, W. ; SADLER, J. E. ; PEREIRA, P. ; HUBER, R. ; BODE, W. ; FUENTES-PRIOR, P.** Crystal structure of the human $\alpha$-thrombin-haemadin complex: an exosite II-binding inhibitor. *EMBO J.,* 2000, vol. 19, 5650-5660
- **SHIMOMURA, T. ; MIYAZAWA, K. ; KOMIYAMA, Y. ; HIRAOKA, H. ; NAKA, D. ; MORIMOTO, Y. ; KITAMURA, N.** Activation of hepatocyte growth factor by two homologous proteases, blood-coagulation factor XIIa and hepatocyte growth factor activator. *Eur J Biochem,* 1995, vol. 229, 257-261
- **STUBBS, M. ; BODE, W.** A player of many parts: The spotlight falls on thrombin's structure. *Thromb. Res.,* 1993, vol. 69, 1-58
- **TAKEUCHI, T. ; SHUMAN, M. A. ; CRAIK, C. S.** Reverse biochemistry: use of macromolecular protease inhibitors to dissect complex biological processes and identify a membrane-type serine protease in epithelial cancer and normal tissue. *Proc Natl Acad Sci USA,* 1999, vol. 96, 11054-11061
- **TRUSOLINO et al.** *FASEB J.,* 1998, vol. 12, 1267-1280
- **TRUSOLINO, L. ; COMOGLIO, P. M.** Scatter-factor and semaphorin receptors: cell signalling for invasive growth. *Nature Rev. Cancer,* 2002, vol. 2, 289-300

- **TSIANG, M. ; JAIN, A. K. ; DUNN, K. E. ; ROJAS, M. E. ; LEUNG, L. L. K. ; GIBBS, C. S.** Functional mapping of the surface residues of human thombin. *J. Biol. Chem.,* 1995, vol. 270, 16854-16863
- **VIJAYALAKSHMI, J. ; PADMANABHAN, K. P. ; MANN, K. G. ; TULINSKY, A.** The isomorphous structures of prethrombin2, hirugen-, and PPACK-thrombin: changes accompanying activation and exosite binding to thrombin. *Protein Sci.,* 1994, vol. 3, 2254-2271

- **WANG, D. ; BODE, W. ; HUBER. R.** Bovine chymotrypsinogen A: x-ray crystal structure analysis and refinement of a new crystal form at 1.8Å resolution. *J. Mol. Biol.,* 1985, vol. 185, 595-624
- **WANG, D. ; JULIAN, F. M. ; BREATHNACH, R. ; GODOWSKI, P. J. ; TAKEHARA, T. ; YOSHIKAWA, W. ; HAGIYA, M. ; LEONARD, E. J.** Macrophage stimulating protein (MSP) binds to its receptor via the MSP β chain. *J. Biol. Chem.,* 1997, vol. 272, 16999-17004